# EUROPEAN PATENT APPLICATION

(11) **EP 4 501 918 A1**
(43) Date of publication of application: **05.02.2025**
(21) Application number: 23775366.0
(22) Date of filing: 24.03.2023
(51) Int. Cl.: C07D 261/08, A61K 31/42, A61K 31/496, A61P 27/02

(54) **3-PHENYLISOXAZOLE DERIVATIVE, AND PHARMACEUTICAL COMPOSITION FOR PREVENTING OR TREATING EYE DISEASE, CONTAINING SAME AS ACTIVE INGREDIENT**

(30) Priority: 25.03.2022 KR 20220037633
(71) Applicant: VASTHERA Co. Ltd., Seoul 03760 (KR)
(72) Inventor: JEONG, Kwang Won, Seoul 06995 (KR); SEO, Seung-Yong, Incheon 22001 (KR); LEE, Kyung Joo, Seoul 04180 (KR); KANG, Sang Won, Seoul 06517 (KR); KANG, Dong Hoon, Goyang-si, Gyeonggi-do 10322 (KR); JANG, Ji Yong, Seoul 07666 (KR); EOM, Ho Hyen, Yongin-si, Gyeonggi-do 17027 (KR); PARK, Keun Hyeong, Gimpo-si, Gyeonggi-do 10125 (KR)
(74) Representative: Engelhard, Markus
(86) International application number: PCT/KR2023/003991
(87) International publication number: WO 2023/182871

(57) **Abstract**

The present invention relates to a 3-phenylisoxazole derivative, a preparation method therefor, and a pharmaceutical composition for preventing or treating eye disease, containing same as an active ingredient. The 3-phenylisoxazole derivative or a pharmaceutically acceptable salt thereof, of the present invention, increases autophagy activity so as to promote the removal of residues of metabolites such as A2E and the like accumulated in the eyeball, and effectively inhibits ocular cell damage or death caused by A2E oxidized by blue light, and thus can be provided as a new concept first-in class therapeutic agent for eye diseases such as dry macular degeneration for which there has been no fundamental treatment thus far.

## Description

### [Technical Field]

This application claims priority based on Korean Patent Application No. 10-2022-0037633 filed on March 25, 2022, and all contents disclosed in the specification and drawings of said application are incorporated herein by reference.

The present invention relates to 3-phenylisoxazole derivatives, a process for preparing the same, and a pharmaceutical composition comprising the same as an active agent for preventing or treating ocular diseases. That is, the present invention relates to novel compounds and their medical uses. The present invention also relates to a method for preventing or treating ocular diseases using the compounds of the present invention.

### [Background Art]

Modern people spend most of their time in front of various digital screens such as computers and smartphones, and the rate of vision deterioration is continuously increasing due to this environmental influence. Since LEDs are frequently used on smart device screens, eye health is worsening due to blue light. Blue light is a blue color light in the 400~500 nm range of visible light, and refers to light emitted from digital devices or smartphones. The reason why digital devices feel bluish overall when turned on at night is due to blue light.

Long-term exposure to blue light stimulates the optic nerve of the eyes, causing eye fatigue and various eye diseases. Blue light has high energy and high penetrability, so if it enters the eyes directly, it focuses on the retina opaquely, reducing clarity, and if chronically exposed, it causes aging and degeneration of the retina. The effects of blue light on the human body are known to include dry eyes, eye fatigue, decreased vision, various eye diseases, accelerated retinal aging, macular degeneration, and insomnia due to suppression of melatonin production (Ganka Gakkai Zasshi. 2001 Oct;105(10):687-95; Archives of Ophthalmology 1992; 110:99-104; Review of Ophthalmology Oct 15 2003; 10(10)).

Age-related macular degeneration has two types: dry and wet. Dry macular degeneration accounts for 90% of age-related macular degeneration patients and progresses by accumulation of debris such as drusen on the retina, while wet macular degeneration accounts for about 10% of age-related macular degeneration patients and progresses by angiogenesis in the macula, causing fluid components in the blood and the blood itself to leak out.

The retinal tissue, especially the retinal pigment epithelium, located beneath the central retina where the rod photoreceptors are densely packed, is the site where metabolic waste products accumulate most actively. As humans reach a certain age threshold, the physiological ability of the body to process these metabolic waste products within the retina declines, and drusen (yellow deposits in the retina) are formed in the retinal pigment epithelium. These drusen are thought to contribute to age-related macular degeneration (AMD) by interfering with the normal physiological/metabolic activity that allows adequate nutrients to reach the photoreceptors and by inducing the death of retinal pigment epithelial cells.

These metabolic waste products are characterized by specific fluorophores. The most important of these is N-retinylidene-N-retinyl-ethanolamine (A2E), a lipofuscin component. A2E is known to be a major cause of retinal diseases caused by phototoxicity, such as when exposed to blue light, photooxidation occurs, causing the production of reactive oxygen species.

Prevention and treatment methods differ depending on the difference between dry macular degeneration and wet macular degeneration. In the case of wet macular degeneration, prevention is done by preventing the main factor, abnormal vascular hemorrhage, and treatment methods mainly include injecting anti-angiogenic growth factor(s) to the eye or applying photodynamic therapy (PDT) using a special laser. In addition, treatments for wet macular degeneration mainly focus on suppressing or improving abnormal blood vessels.

On the other hand, there is no standard treatment for dry macular degeneration, and only hyperlipidemia drugs (statin drugs) are used to reduce the amount of lipids that are the raw material for waste products such as drusen, or powerful antioxidants such as lutein and zeaxanthin in the flavonoid family are prescribed. However, these methods are all indirect treatment methods, and there is still no treatment that directly removes the metabolic products accumulated in retinal cells.

### [Disclosure]

### [Technical Problem]

The object of the present invention is to provide a novel compound useful for preventing or treating eye diseases, especially dry macular degeneration, for which there is still no fundamental treatment drug. In particular, the object is to provide a novel compound that can prevent or treat eye diseases by removing accumulated A2E and directly inhibiting damage or death of retinal cells caused by A2E oxidized by blue light.

Another object of the present invention is to provide a method for manufacturing the compound.

Another object of the present invention is to provide a pharmaceutical composition for preventing or treating eye diseases, especially dry macular degeneration, comprising the compound as an active agent. That is, another object of the present invention is to provide a method for treating or preventing eye diseases, especially dry macular degeneration, which comprises administering a compound according to the present invention to a subject in need of treatment or prevention of eye diseases.

Yet another object of the present invention is to provide a nutraceutical composition that helps improve eye diseases, comprising the compound as an active agent.

Yet another object of the present invention is to provide a nutraceutical or pharmaceutical composition useful for protecting retinal damage caused by blue light. That is, another object of the present invention is to provide a method for protecting the retina from damage caused by blue light, comprising administering a compound according to the present invention to a subject in need of retinal protection.

### [Technical Solution]

To achieve the above objects, the present invention provides a compound represented by the following chemical formula 1 or a pharmaceutically acceptable salt thereof.

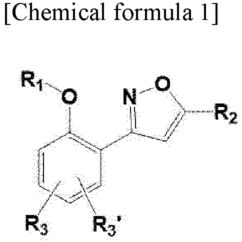

In chemical formula 1,
R₁ is hydrogen, C₁₋₆alkyl (preferably, methyl), or C₃₋₆cycloalkyl, wherein at least one hydrogen of the C₁₋₆alkyl or C₃₋₆cycloalkyl is optionally substituted with at least one substituent independently selected from the group consisting of hydroxy, C₁₋₃alkoxy and halogen (for example, F),
R₂ is hydrogen, -C₁₋₆alkyl, C₃₋₆cycloalkyl, -C₁₋₆alkoxy, -C₁₋₃alkylene-ORa, -C₁₋₃alkylene-NRaRb, -C₁₋₃alkylene-heterocycloalkyl (preferably, heterocycloalkyl is piperazine, piperidine, morpholine, pyrrolidine, 3,8-diazabicyclo[3.2.1]octane, or 2,5-diazabicyclo[2.2.2]octane), or -C₁₋₃alkylene-heteroaryl (preferably, heteroaryl is 4,5,6,7-tetrahydrothieno[3,2-c]pyridine or 5,6,7,8-tetrahydroimidazo[1,2-a]pyrazine), wherein Ra and Rb are each independently hydrogen, C₁₋₆alkyl, C₃₋₆cycloalkyl, allyl, benzyl, -C₁₋₃alkylene-NH-C₁₋₃alkyl, -C₁₋₃alkylene-OH, -C₁₋₃alkylene-COOH, -C₁₋₃alkylene-COO-C₁₋₃alkyl, pyrrolidine, -C₁₋₃alkylene-pyrrolidine, aryl, or heteroaryl (for example, quinazoline or pyridyl), wherein at least one hydrogen of the -C₁₋₆alkyl, C₃₋₆cycloalkyl, C₁₋₆alkoxy, C₁₋₃alkylene or heterocycloalkyl is optionally substituted with at least one substituent independently selected from the group consisting of =O, -C₁₋₃alkyl, -OH, -COOH, -CO-C₁₋₃alkyl, -CONH₂, -SO₂-C₁₋₃alkyl, -NH₂, -NHCH₃, -N(CH₃)₂, -NHCOCH₃, halogen, allyl, phenyl, benzyl, -C₁₋₃alkylene-OH, -C₁₋₃alkylene-COOH, -C₁₋₃alkylene-COOC₁₋₃alkyl, methoxycarbonyl, ethoxycarbonyl, and tert-butoxycarbonyl,
R₃ and R₃' are each independently hydrogen, halogen (preferably, Br, F), C₁₋₆alkyl, C₁₋₆alkoxy, C₃₋₆cycloalkyl, heterocycloalkyl (preferably, morpholine), -CH₂-CR₄ (=CR₅R₆) (for example, allyl), -CN, haloC₁₋₃alkyl (preferably, CF₃), -C₁₋₃alkylene-OR₄, -C(O)R₄, -C₁₋₃alkylene-C(O)R₄, -C₁₋₃alkylene-COOH, or -COOH, wherein R₄, R₅, and R₆ are each independently hydrogen, methyl, or -NH₂.

Under the above background, the inventors of the present invention have endeavored to develop a novel treatment agent for eye diseases, especially dry macular degeneration, for which there is still no fundamental treatment drug. In the meantime, the inventors of the present invention have confirmed that the 3-phenylisoxazole derivative provided by the present invention is excellent in various aspects in suppressing damage or death of retinal cells, and also promotes the removal of waste products such as metabolites such as A2E accumulated in the eye by increasing autophagy activity, so that it can be used as a first-in-class therapeutic agent capable of fundamentally treating eye diseases such as dry macular degeneration, thereby completing the present invention.

In addition, the present invention provides a pharmaceutical composition for preventing or treating eye diseases, particularly dry macular degeneration, comprising a compound represented by the chemical formula 1 or a pharmaceutically acceptable salt thereof as an active agent.

Furthermore, the present invention provides a nutraceutical composition for improving eye diseases, comprising a compound represented by the chemical formula 1 or a pharmaceutically acceptable salt thereof as an active agent.

**In** addition, the present invention provides a method for preventing or treating an eye disease, particularly dry macular degeneration, comprising administering a compound represented by the chemical formula 1 or a pharmaceutically acceptable salt thereof to a subject in need of treatment.

Furthermore, the present invention provides a use of the compound represented by the chemical formula 1 or a pharmaceutically acceptable salt thereof in the manufacture of a medicament for preventing or treating an eye disease.

**In** addition, the present invention provides a pharmaceutical or nutraceutical composition for protecting retinal damage caused by blue light, comprising a compound represented by the above chemical formula 1 or a pharmaceutically acceptable salt thereof as an active agent.

In addition, the present invention provides a method for improving or protecting retinal damage caused by blue light, comprising administering a compound represented by the above chemical formula 1 or a pharmaceutically acceptable salt thereof to a subject in need of improvement or protection.

Furthermore, the present invention provides a use of a compound represented by the above chemical formula 1 or a pharmaceutically acceptable salt thereof in the manufacture of a product for improving retinal damage caused by blue light.

### [Advantageous Effects]

The 3-phenylisoxazole derivative of the present invention, a stereoisomer thereof, or a pharmaceutically acceptable salt thereof increases autophagy activity, thereby promoting the removal of waste products such as metabolites such as A2E accumulated in the eye, and effectively inhibiting damage to or death of eye cells caused by A2E oxidized by blue light, and thus has a useful effect that can be provided as a new concept, first-in-class therapeutic agent for eye diseases for which there is still no fundamental treatment, such as dry macular degeneration.

### [Description of Drawings]

Figure 1 is a photograph showing the change in fluorescence intensity confirmed from the concentration-dependent treatment of compound 17 of the present invention after accumulating fluorescently labeled A2E (A2E-BDP) in human retinal pigment epithelial cells (ARPE-19).
Figure 2 is a graph showing the results of measuring the amount of intracellular A2E by HPLC to confirm the A2E removal effect confirmed from the concentration-dependent treatment of compound 17 of the present invention after accumulating A2E in human retinal pigment epithelial cells (ARPE-19).
Figure 3 is a photograph showing the cell death inhibition effect of compound 17 of the present invention on the death of human retinal pigment epithelial cells (ARPE-19) induced by blue light (BL) and A2E according to the concentration-dependent treatment, and the results of evaluating cell viability.
Figure 4 is a photograph showing the analysis of changes in cytoplasmic or nuclear proteins confirmed by Western immunoblotting to confirm the effect of treatment with compound 17 of the present invention at different concentrations on the activation of NF-κB induced by blue light and A2E.
Figure 5 is a graph showing the mRNA expression levels of inflammation-related genes measured to confirm the effect of treatment with compound 17 of the present invention at different concentrations on the inflammatory response in human retinal pigment epithelial cells (ARPE-19) induced by blue light and A2E.

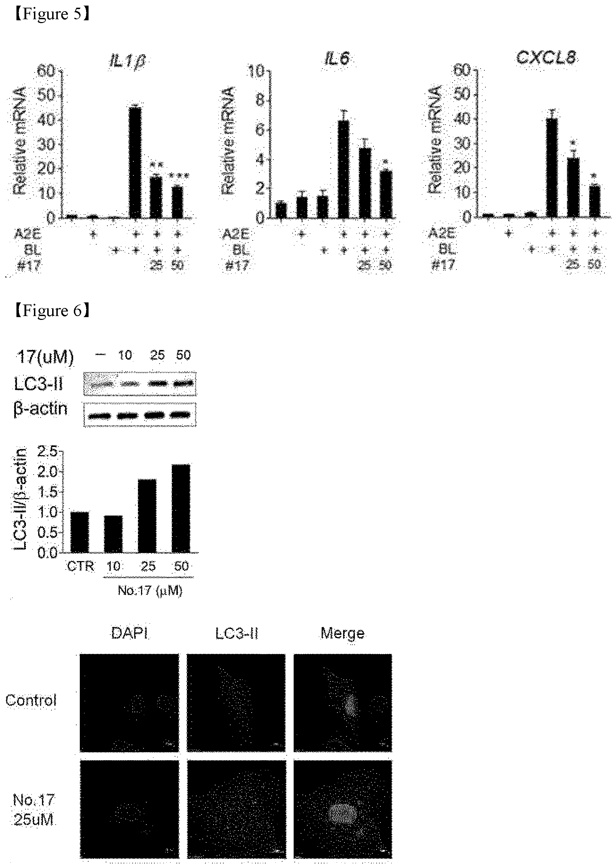

The upper graph of figure 6 is a graph measuring the change in the autophagy marker LC3-II protein by Western immunoblotting to confirm the effect of treatment with compound 17 of the present invention at different concentrations on the change in autophagy activity of human retinal pigment epithelial cells (ARPE-19) induced by blue light and A2E, and the lower photo of figure 6 is a photo observing the puncta formation of GFP-LC3 using a confocal laser scanning microscope (Nikon, Tokyo, Japan).

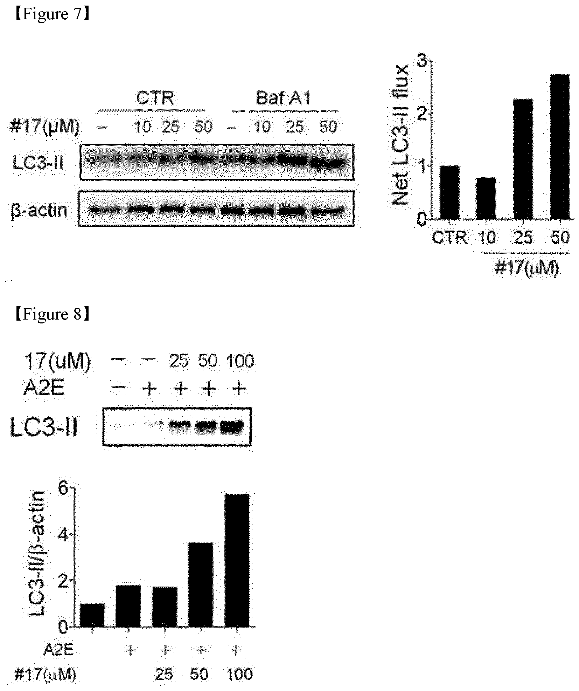

Figure 7 is a photograph of the change in the autophagy marker LC3-II protein analyzed by Western immunoblotting in the presence or absence of bafilomycin A1 to confirm the effect of improving the autophagy flux of human retinal pigment epithelial cells (ARPE-19) according to the treatment with compound 17 of the present invention, and a graph quantitatively showing the autophagy flux by calculating the difference in the amount of LC3-II protein according to the presence or absence of bafilomycin A1.
Figure 8 is a Western immunoblotting photograph and a quantitative graph showing the effect of autophagy activation according to the treatment of compound 17 of the present invention on human retinal pigment epithelial cells (ARPE-19) in which A2E is accumulated.

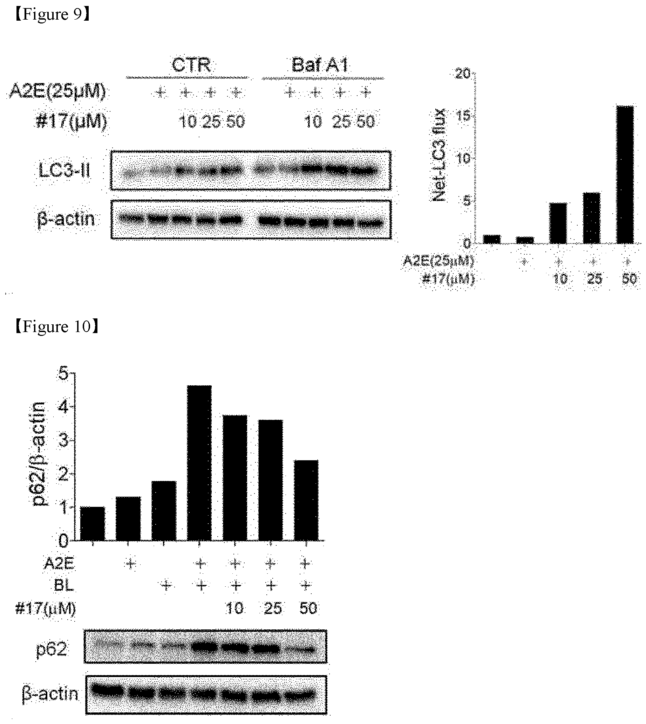

Figure 9 is a Western immunoblotting photograph and a quantitative graph showing the effect of improving autophagy flux according to the treatment of compound 17 of the present invention on human retinal pigment epithelial cells (ARPE-19) in which A2E is accumulated.
Figure 10 is a Western immunoblotting photograph and a quantitative graph showing the effect of treatment of compound 17 of the present invention at different concentrations on p62 protein accumulated in cells due to autophagy inhibition caused by blue light and A2E.

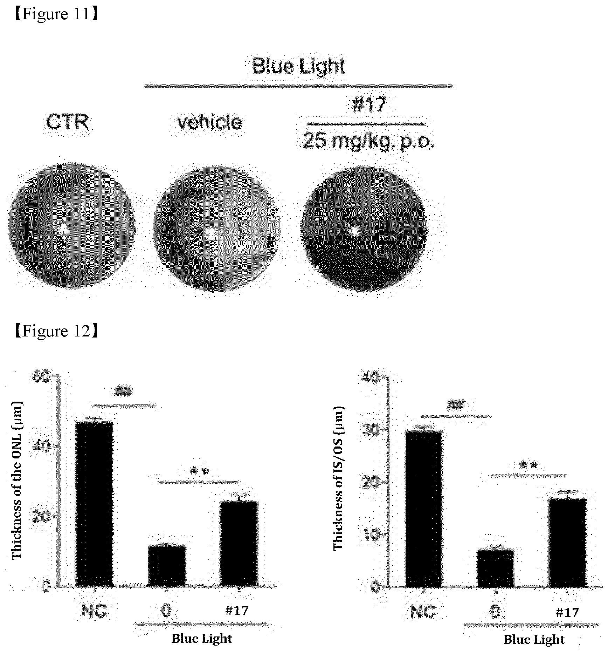

Figure 11 is a photograph of the fundus area showing the retinal damage and improvement effect according to the treatment of compound 17 of the present invention on animals irradiated with blue light, taken with a fundus camera.
Figure 12 shows the results of evaluating the reduction and recovery of the thickness of the outer nuclear layer (ONL) and the inner segments/outer segments layer (IS/OS) according to the treatment with compound 17 of the present invention in animals irradiated with blue light. This graph quantitatively shows the average values of the thickness of the ONL and IS/OS measured using the Axio Vision SE644 (ZEISS) program through hematoxylin and eosin staining.

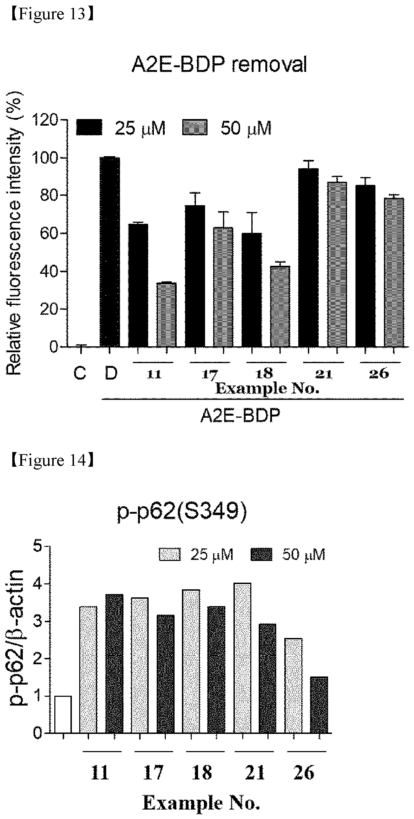

Figure 13 shows the evaluation results of A2E removal of preferred example compounds of the present invention confirmed from concentration-based treatment of test compounds after accumulating A2E-BDP in human retinal pigment epithelial cells (ARPE-19).
Figure 14 shows the evaluation results of autophagy activation of preferred example compounds of the present invention evaluated using concentration changes in p62 protein (p-p62 (S349)) phosphorylated at 349 residue serine.

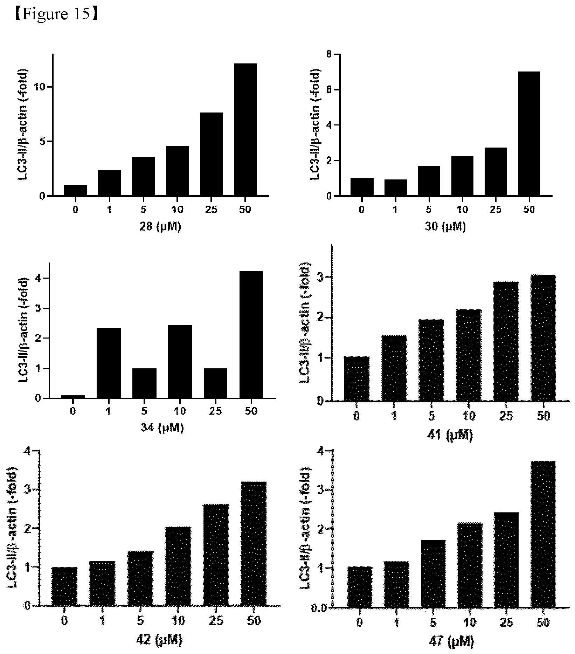

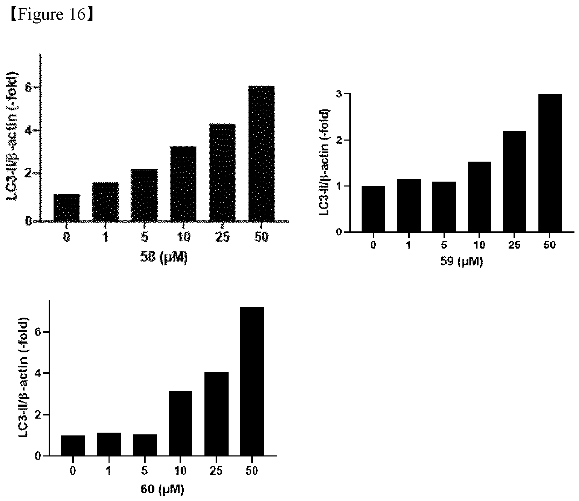

Figures 15 and 16 show the evaluation results of autophagy activation of some example compounds according to the present invention evaluated using concentration changes in LC3-II protein.

### [Mode for Invention]

Hereinafter, the present invention will be described in detail.

The present invention provides a compound represented by the following chemical formula 1 or a pharmaceutically acceptable salt thereof.

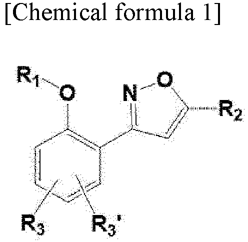

In chemical formula 1,
R₁ is hydrogen, C₁₋₆alkyl (preferably, methyl), or C₃₋₆cycloalkyl, wherein at least one hydrogen of the C₁₋₆alkyl or C₃₋₆cycloalkyl is optionally substituted with at least one substituent independently selected from the group consisting of hydroxy, C₁₋₃alkoxy and halogen (for example, F),
R₂ is hydrogen, -C₁₋₆alkyl, C₃₋₆cycloalkyl, -C₁₋₆alkoxy, -C₁₋₃alkylene-ORa, -C₁₋₃alkylene-NRaRb, -C₁₋₃alkylene-heterocycloalkyl (preferably, heterocycloalkyl is piperazine, piperidine, morpholine, pyrrolidine, 3,8-diazabicyclo[3.2.1]octane, or 2,5-diazabicyclo[2.2.2]octane), or -C₁₋₃alkylene-heteroaryl (preferably, heteroaryl is 4,5,6,7-tetrahydrothieno[3,2-c]pyridine or 5,6,7,8-tetrahydroimidazo[1,2-a]pyrazine), wherein Ra and Rb are each independently hydrogen, C₁₋₆alkyl, C₃₋₆cycloalkyl, allyl, benzyl, -C₁₋₃alkylene-NH-C₁₋₃alkyl, -C₁₋₃alkylene-OH, -C₁₋₃alkylene-COOH, -C₁₋₃alkylene-COO-C₁₋₃alkyl, pyrrolidine, -C₁₋₃alkylene-pyrrolidine, aryl, or heteroaryl (for example, quinazoline or pyridyl), wherein at least one hydrogen of the -C₁₋₆alkyl, C₃₋₆cycloalkyl, C₁₋₆alkoxy, C₁₋₃alkylene or heterocycloalkyl is optionally substituted with at least one substituent independently selected from the group consisting of =O, -C₁₋₃alkyl, -OH, -COOH, -CO-C₁₋₃alkyl, -CONH₂, -SO₂-C₁₋₃alkyl, -NH₂, -NHCH₃, -N(CH₃)₂, -NHCOCH₃, halogen, allyl, phenyl, benzyl, -C₁₋₃alkylene-OH, -C₁₋₃alkylene-COOH, -C₁₋₃alkylene-COOC₁₋₃alkyl, methoxycarbonyl, ethoxycarbonyl, or tert-butoxycarbonyl,
R₃ and R₃' are each independently hydrogen, halogen (preferably, Br, F), C₁₋₆alkyl, C₁₋₆alkoxy, C₃₋₆cycloalkyl, heterocycloalkyl (preferably, morpholine), -CH₂-CR₄ (=CR₅R₆) (for example, allyl), -CN, haloC₁₋₃alkyl (preferably, CF₃), -C₁₋₃alkylene-OR₄, -C(O)R₄, -C₁₋₃alkylene-C(O)R₄, -C₁₋₃alkylene-COOH, or -COOH, wherein R₄, R₅, and R₆ are each independently hydrogen, methyl, or -NH₂.

In one embodiment of the present invention, the present invention provides a compound or a pharmaceutically acceptable salt thereof, wherein the compound of the chemical formula 1 has a structure of the following chemical formula 1a: 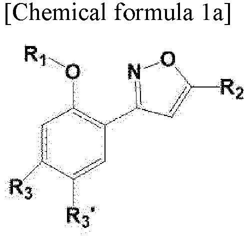 in the chemical formula 1a,
R₁ is hydrogen, C₁₋₆alkyl (preferably, methyl), or C₃₋₆cycloalkyl, wherein at least one hydrogen of the C₁₋₆alkyl or C₃₋₆cycloalkyl is optionally substituted with at least one substituent independently selected from the group consisting of hydroxy, C₁₋₃alkoxy and halogen (for example, F),
R₂ is hydrogen, -C₁₋₆alkyl, C₃₋₆cycloalkyl, -C₁₋₆alkoxy, -C₁₋₃alkylene-ORa, -C₁₋₃alkylene-NRaRb, -C₁₋₃alkylene-heterocycloalkyl (preferably, heterocycloalkyl is piperazine, piperidine, morpholine, pyrrolidine, 3,8-diazabicyclo[3.2.1]octane, or 2,5-diazabicyclo[2.2.2]octane), or -C₁₋₃alkylene-heteroaryl (preferably, heteroaryl is 4,5,6,7-tetrahydrothieno[3,2-c]pyridine or 5,6,7,8-tetrahydroimidazo[1,2-a]pyrazine), wherein Ra and Rb are each independently hydrogen, C₁₋₆alkyl, C₃₋₆cycloalkyl, allyl, benzyl, -C₁₋₃alkylene-NH-C₁₋₃alkyl, -C₁₋₃alkylene-OH, -C₁₋₃alkylene-COOH, -C₁₋₃alkylene-COO-C₁₋₃alkyl, pyrrolidine, -C₁₋₃alkylene-pyrrolidine, aryl, or heteroaryl (for example, quinazoline or pyridyl), wherein at least one hydrogen of the -C₁₋₆alkyl, C₃₋₆cycloalkyl, C₁₋₆alkoxy, C₁₋₃alkylene or heterocycloalkyl is optionally substituted with at least one substituent independently selected from the group consisting of =O, -C₁₋₃alkyl, -OH, -COOH, -CO-C₁₋₃alkyl, -CONH₂, -SO₂-C₁₋₃alkyl, -NH₂, -NHCH₃, -N(CH₃)₂, -NHCOCH₃, halogen, allyl, phenyl, benzyl, -C₁₋₃alkylene-OH, -C₁₋₃alkylene-COOH, -C₁₋₃alkylene-COOC₁₋₃alkyl, methoxycarbonyl, ethoxycarbonyl, and tert-butoxycarbonyl,
R₃ and R₃' are each independently hydrogen, halogen (preferably, Br, F), C₁₋₆alkyl, C₁₋₆alkoxy, C₃₋₆cycloalkyl, heterocycloalkyl (preferably, morpholine), -CH₂-CR₄ (=CR₅R₆) (for example, allyl), -CN, haloC₁₋₃alkyl (preferably, CF₃), -C₁₋₃alkylene-OR₄, -C(O)R₄, -C₁₋₃alkylene-C(O)R₄, -C₁₋₃alkylene-COOH, or -COOH, wherein R₄, R₅, and R₆ are each independently hydrogen, methyl, or -NH₂.

In a preferred embodiment of the present invention, the present invention provides a compound represented by the chemical formula 1a or a pharmaceutically acceptable salt thereof, wherein
R₁ is hydrogen or C₁₋₆alkyl (preferably, methyl), wherein at least one hydrogen of the C₁₋₆alkyl is optionally substituted with at least one substituent independently selected from halogen (for example, F),
R₂ is -C₁₋₆alkyl, -C₁₋₃alkylene-ORa, -C₁₋₃alkylene-NRaRb, -C₁₋₃alkylene-piperazine, - C₁₋₃alkylene-piperidine, -C₁₋₃alkylene-morpholine, -C₁₋₃alkylene-4,5,6,7-tetrahydrothieno[3,2-c]pyridine, -C₁₋₃alkylene-5,6,7,8-tetrahydroimidazo[1,2-a]pyrazine, -C₁₋₃alkylene-pyrrolidine, -C₁₋₃alkylene-3,8-diazabicyclo[3.2.1]octane, or -C₁₋₃alkylene-2,5-diazabicyclo[2.2.2]octane, wherein Ra and Rb are each independently hydrogen, C₁₋₆alkyl, C₃₋₆cycloalkyl, allyl, benzyl, C₁₋₃alkylene-OH, -C₁₋₃alkylene-COOH, -C₁₋₃alkylene-COO-C₁₋₃alkyl, aryl, or heteroaryl (for example, quinazoline or pyridyl), wherein at least one hydrogen of the -C₁₋₆alkyl, C₃₋₆cycloalkyl, C₁₋₆alkoxy, C₁₋₃alkylene or heterocycloalkyl is optionally substituted with at least one substituent independently selected from the group consisting of =O, -C₁₋₃alkyl, -OH, -COOH, -CO-C₁₋₃alkyl, -CONH₂, -SO₂-C₁₋₃alkyl, -NH₂, -NHCH₃, - N(CH₃)₂, -NHCOCH₃, halogen, allyl, phenyl, benzyl, -C₁₋₃alkylene-OH, -C₁₋₃alkylene-COOH, -C₁₋₃alkylene-COOC₁₋₃alkyl, methoxycarbonyl, ethoxycarbonyl, and tert-butoxycarbonyl,
R₃ and R₃' are each independently hydrogen, halogen (preferably, Br, F), C₁₋₆alkyl, C₁₋₆alkoxy, heterocycloalkyl (preferably, morpholine), -CH₂-CR₄(=CR₅R₆) (for example, allyl), - CN, haloC₁₋₃alkyl (preferably, CF₃), -C₁₋₃alkylene-OR₄, -C(O)R₄, -C₁₋₃alkylene-C(O)R₄, -C₁₋₃alkylene-COOH, or -COOH, wherein R₄, R₅, and R₆ are each independently hydrogen, methyl, or -NH₂.

In a more preferred embodiment of the present invention, the present invention provides a compound represented by the chemical formula 1a or a pharmaceutically acceptable salt thereof, wherein
R₁ is hydrogen, methyl, or -CF₃,
R₂ is -C₁₋₆alkyl, -C₁₋₃alkylene-ORa, -C₁₋₃alkylene-piperazine, -C₁₋₃alkylene-piperidine, -C₁₋₃alkylene-morpholine, -C₁₋₃alkylene-4,5,6,7-tetrahydrothieno[3,2-c]pyridine, or -C₁₋₃alkylene-5,6,7,8-tetrahydroimidazo[1,2-a]pyrazine, wherein Ra and Rb are each independently hydrogen, C₁₋₆alkyl, allyl, benzyl, -C₁₋₃alkylene-COO-C₁₋₃alkyl, phenyl, quinazoline, or pyridyl, wherein at least one hydrogen of the -C₁₋₆alkyl, C₁₋₃alkylene or heterocycloalkyl is optionally substituted with at least one substituent independently selected from the group consisting of =O, -C₁₋₃alkyl, -OH, -COOH, -CO-C₁₋₃alkyl, -CONH₂, -SO₂-C₁₋₃alkyl, halogen, allyl, phenyl, benzyl, -C₁₋₃alkylene-OH, -C₁₋₃alkylene-COOH, -C₁₋₃alkylene-COOC₁₋₃alkyl, methoxycarbonyl, ethoxycarbonyl, and tert-butoxycarbonyl,
R₃ and R₃' are each independently hydrogen, halogen, C₁₋₆alkyl, C₁₋₆alkoxy, heterocycloalkyl (preferably, morpholine), -CH₂-CR₄(=CR₅R₆) (for example, allyl), -CN, - haloC₁₋₃alkyl (preferably, CF₃), -C₁₋₃alkylene-OR₄, -C(O)R₄, -C₁₋₃alkylene-C(O)R₄, -C₁₋₃alkylene-COOH, or -COOH, wherein R₄, R₅, and R₆ are each independently hydrogen or methyl.

In one specific embodiment of the present invention, a preferred example of the compound represented by the chemical formula 1 is
(1) 3-(5-bromo-2-methoxyphenyl)-5-propylisoxazole;
(2) 4-bromo-2-(5-propylisoxazole-3-yl)phenol;
(3) 3-(2-methoxy-5-propylphenyl)-5-propylisoxazole;
(4) 3-(5-allyl-2-methoxyphenyl)-5-propylisoxazole;
(5) 4-allyl-2-(5-propylisoxazole-3-yl)phenol;
(6) 4-propyl-2-(5-propylisoxazole-3-yl)phenol;
(7) (3-(5-bromo-2-methoxyphenyl)isoxazole-5-yl)methanol;
(8) 4-bromo-2-(5-(hydroxymethyl)isoxazole-3-yl)phenol;
(9) 5-((allyloxy)methyl)-3-(5-bromo-2-methoxyphenyl)isoxazole;
(10) (3-(2-methoxy-5-propylphenyl)isoxazole-5-yl)methanol;
(11) 5-((benzyloxy)methyl)-3-(5-bromo-2-methoxyphenyl)isoxazole;
(12) ethyl 2-((3-(5-bromo-2-methoxyphenyl)isoxazole-5-yl)methoxy)acetate;
(13) 3-(5-bromo-2-methoxyphenyl)-5-(methoxymethyl)isoxazole;
(14) 2-(5-(hydroxymethyl)isoxazole-3-yl)-4-propylphenol;
(15) (3-(5-allyl-2-methoxyphenyl)isoxazole-5-yl)methanol;
(16) 4-allyl-2-(5-(hydroxymethyl)isoxazole-3-yl)phenol;
(17) 3-(5-allyl-2-methoxyphenyl)-5-((allyloxy)methyl)isoxazole;
(18) 3-(5-allyl-2-methoxyphenyl)-5-((benzyloxy)methyl)isoxazole;
(19) ethyl 2-((3-(5-allyl-2-methoxyphenyl)isoxazole-5-yl)methoxy)acetate;
(20) 3-(2-methoxy-5-propylphenyl)-5-(methoxymethyl)isoxazole;
(21) tert-butyl 4-((3-(5-bromo-2-methoxyphenyl)isoxazole-5-yl)methyl)piperazine-1-carboxylate;
(22) 3-(5-allyl-2-methoxyphenyl)-5-((4-allylpiperazine-1-yl)methyl)isoxazole;
(23) 3-(5-allyl-2-methoxyphenyl)-5-((4-benzylpiperazine-1-yl)methyl)isoxazole;
(24) ethyl 2-(4-((3-(5-allyl-2-methoxyphenyl)isoxazole-5-yl)methyl)piperazine-1-yl)acetate;
(25) tert-butyl 4-((3-(5-allyl-2-methoxyphenyl)isoxazole-5-yl)methyl)piperazine-1-carboxylate;
(26) tert-butyl 4-((3-(2-methoxy-5-propylphenyl)isoxazole-5-yl)methyl)piperazine-1-carboxylate;
(27) 4-allyl-2-(5-((allyloxy)methyl)isoxazole-3-yl)phenol;
(28) 3-(5-bromo-2-methoxyphenyl)-5-(piperazine-1-ylmethyl)isoxazole;
(29) 3-(5-allyl-methoxyphenyl)-5-(piperazine-1-ylmethyl)isoxazole;
(30) 3-(2-methoxy-5-propylphenyl)-5-(piperazine-1-ylmethyl)isoxazole;
(31) (3-(2-methoxy-5-(trifluoromethyl)phenyl)isoxazole-5-yl)methanol;
(32) 2-(3-(2-methoxy-5-(trifluoromethyl)phenyl)isoxazole-5-yl)propan-2-ol;
(33) 2-(3-(2-methoxy-5-(trifluoromethyl)phenyl)isoxazole-5-yl)ethan-1-ol;
(34) 3-(2-methoxy-5-(trifluoromethyl)phenyl)-5-(piperazine-1-ylmethyl)isoxazole;
(35) (3-(2-methoxy-4-(trifluoromethyl)phenyl)isoxazole-5-yl)methanol;
(36) 2-((3-(2-methoxy-5-(trifluoromethyl)phenyl)isoxazole-5-yl)methoxy)acetic acid;
(37) 4-((3-(2-methoxy-5-(trifluoromethyl)phenyl)isoxazole-5-yl)methyl)-1-methylpiperazine-2-on;
(38) (3-(2-methoxy-5-morpholinophenyl)isoxazole-5-yl)methanol;
(39) 2-(3-(5-allyl-2-methoxyphenyl)isoxazole-5-yl)propan-2-ol;
(40) 2-(3-(2-methoxy-5-propylphenyl)isoxazole-5-yl)propan-2-ol;
(41) 3-(5-allyl-2-methoxyphenyl)-5-((4-methylpiperazine-1-yl)methyl)isoxazole;
(42) 3-(2-methoxy-5-propylphenyl)-5-((4-methylpiperazine-1-yl)methyl)isoxazole;
(43) (3-(5-allyl-2-(trifluoromethoxy)phenyl)isooxazole-5-yl)methanol;
(44) (3-(4-allyl-2-methoxyphenyl)isoxazole-5-yl)methanol;
(45) (3-(5-isopropyl-2-methoxyphenyl)isoxazole-5-yl)methanol;
(46) tert-butyl 4-((3-(5-isopropyl-2-methoxyphenyl)isoxazole-5-yl)methyl)piperazine-1-carboxylate;
(47) 3-(5-isopropyl-2-methoxyphenyl)-5-(piperazine-1-ylmethyl)isoxazole;
(48) tert-butyl 4-((3-(4-allyl-2-methoxyphenyl)isoxazole-5-yl)methyl)piperazine-1-carboxylate;
(49) 3-(4-allyl-2-methoxyphenyl)-5-(piperazine-1-ylmethyl)isoxazole;
(50) tert-butyl 4-((3-(5-allyl-2-(trifluoromethoxy)phenyl)isoxazole-5-yl)methyl)piperazine-1-carboxylate;
(51) 3-(5-allyl-2-(trifluoromethoxy)phenyl)-5-(piperazine-1-ylmethyl)isoxazole;
(52) 3-(5-allyl-2-(trifluoromethoxy)phenyl)-5-((4-methylpiperazine-1-yl)methyl)isoxazole;
(53) (2S,6R)-4-((3-(2-methoxy-5-propylphenyl)isoxazole-5-yl)methyl)-2,6-dimethylmorpholine;
(54) 3-(2-methoxy-5-propylphenyl)-5-((4-methoxypiperidine-1-yl)methyl)isoxazole;
(55) 1-((3-(2-methoxy-5-propylphenyl)isoxazole-5-yl)methyl)piperidine-4-carboxylic acid; (56) 5-((6,7-dihydrothieno[3,2-c]pyridin-5(4H)-yl)methyl)-3-(2-methoxy-5-propylphenyl)isoxazole;
(57) 5-((5,6-dihydroimidazo[1,2-a]pyrazin-7(8H)-yl)methyl)-3-(2-methoxy-5-propylphenyl)isoxazole;
(58) 3-(4-fluoro-2-methoxy-5-propylphenyl)-5-(piperazine-1-ylmethyl)isoxazole;
(59) 3-(4-fluoro-2-methoxy-5-propylphenyl)-5-((4-methylpiperazine-1-yl)methyl)isoxazole;
(60) 5-((4-methylpiperazine-1-yl)methyl)-3-(5-propyl-2-(trifluoromethoxy)phenyl)isoxazole;
(61) 2-(4-methoxy-3-(5-((4-methylpiperazine-1-yl)methyl)isoxazole-3-yl)phenyl)ethan-1-ol;
(62) 3-(2-methoxy-5-(2-methoxyethyl)phenyl)-5-((4-methylpiperazine-1-yl)methyl)isoxazole;
(63) 1-(4-((3-(5-isopropyl-2-methoxyphenyl)isoxazole-5-yl)methyl)piperazine-1-yl)ethan-1-on;
(64) 3-(5-isopropyl-2-methoxyphenyl)-5-((4-(methylsulfonyl)piperazine-1-yl)methyl)isoxazole;
(65) 3-(5-allyl-2-methoxyphenyl)-5-((quinazolin-4-yloxy)methyl)isoxazole;
(66) tert-butyl 4-((3-(2-methoxy-5-(2-methylallyl)phenyl)isoxazole-5-yl)methyl)piperazine-1-carboxylate;
(67) 3-(2-methoxy-5-(2-methylallyl)phenyl)-5-(piperazine-1-ylmethyl)isoxazole;
(68) (3-(2-methoxy-5-(2-methylallyl)phenyl)isoxazole-5-yl)methanol;
(69) 3-(2-methoxy-5-(3-methylbut-2-en-1-yl)phenyl)-5-((4-methylpiperazine-1-yl)methyl)isoxazole, or a pharmaceutically acceptable salt thereof.

Among the above compounds (1) to (69), in particular,
(11) 5-((benzyloxy)methyl)-3-(5-bromo-2-methoxyphenyl)isoxazole;
(15) (3-(5-allyl-2-methoxyphenyl)isoxazole-5-yl)methanol;
(17) 3-(5-allyl-2-methoxyphenyl)-5-((allyloxy)methyl)isoxazole;
(18) 3-(5-allyl-2-methoxyphenyl)-5-((benzyloxy)methyl)isoxazole;
(21) tert-butyl 4-((3-(5-bromo-2-methoxyphenyl)isoxazole-5-yl)methyl)piperazine-1-carboxylate;
(25) tert-butyl 4-((3-(5-allyl-2-methoxyphenyl)isoxazole-5-yl)methyl)piperazine-1-carboxylate;
(26) tert-butyl 4-((3-(2-methoxy-5-propylphenyl)isoxazole-5-yl)methyl)piperazine-1-carboxylate;
(28) 3-(5-bromo-2-methoxyphenyl)-5-(piperazine-1-ylmethyl)isoxazole;
(29) 3-(5-allyl-methoxyphenyl)-5-(piperazine-1-ylmethyl)isoxazole;
(30) 3-(2-methoxy-5-propylphenyl)-5-(piperazine-1-ylmethyl)isoxazole;
(34) 3-(2-methoxy-5-(trifluoromethyl)phenyl)-5-(piperazine-1-ylmethyl)isoxazole;
(41) 3-(5-allyl-2-methoxyphenyl)-5-((4-methylpiperazine-1-yl)methyl)isoxazole;
(42) 3-(2-methoxy-5-propylphenyl)-5-((4-methylpiperazine-1-yl)methyl)isoxazole;
(43) (3-(5-allyl-2-(trifluoromethoxy)phenyl)isooxazole-5-yl)methanol;
(44) (3-(4-allyl-2-methoxyphenyl)isoxazole-5-yl)methanol;
(47) 3-(5-isopropyl-2-methoxyphenyl)-5-(piperazine-1-ylmethyl)isoxazole;
(48) tert-butyl 4-((3-(4-allyl-2-methoxyphenyl)isoxazole-5-yl)methyl)piperazine-1-carboxylate;
(50) tert-butyl 4-((3-(5-allyl-2-(trifluoromethoxy)phenyl)isoxazole-5-yl)methyl)piperazine-1-carboxylate;
(51) 3-(5-allyl-2-(trifluoromethoxy)phenyl)-5-(piperazine-1-ylmethyl)isoxazole;
(52) 3-(5-allyl-2-(trifluoromethoxy)phenyl)-5-((4-methylpiperazine-1-yl)methyl)isoxazole;
(58) 3-(4-fluoro-2-methoxy-5-propylphenyl)-5-(piperazine-1-ylmethyl)isoxazole;
(59) 3-(4-fluoro-2-methoxy-5-propylphenyl)-5-((4-methylpiperazine-1-yl)methyl)isoxazole;
(60) 5-((4-methylpiperazine-1-yl)methyl)-3-(5-propyl-2-(trifluoromethoxy)phenyl)isoxazole;
(66) tert-butyl 4-((3-(2-methoxy-5-(2-methylallyl)phenyl)isoxazole-5-yl)methyl)piperazine-1-carboxylate;
(67) 3-(2-methoxy-5-(2-methylallyl)phenyl)-5-(piperazine-1-ylmethyl)isoxazole;
(68) (3-(2-methoxy-5-(2-methylallyl)phenyl)isoxazole-5-yl)methanol; or
a pharmaceutically acceptable salt thereof is more preferable for various purposes of the present invention.

Particularly, among the above compounds,
(28) 3-(5-bromo-2-methoxyphenyl)-5-(piperazine-1-ylmethyl)isoxazole;
(30) 3-(2-methoxy-5-propylphenyl)-5-(piperazine-1-ylmethyl)isoxazole;
(34) 3-(2-methoxy-5-(trifluoromethyl)phenyl)-5-(piperazine-1-ylmethyl)isoxazole;
(41) 3-(5-allyl-2-methoxyphenyl)-5-((4-methylpiperazine-1-yl)methyl)isoxazole;
(42) 3-(2-methoxy-5-propylphenyl)-5-((4-methylpiperazine-1-yl)methyl)isoxazole;
(47) 3-(5-isopropyl-2-methoxyphenyl)-5-(piperazine-1-ylmethyl)isoxazole;
(58) 3-(4-fluoro-2-methoxy-5-propylphenyl)-5-(piperazine-1-ylmethyl)isoxazole;
(59) 3-(4-fluoro-2-methoxy-5-propylphenyl)-5-((4-methylpiperazine-1-yl)methyl)isoxazole;
(60) 5-((4-methylpiperazine-1-yl)methyl)-3-(5-propyl-2-(trifluoromethoxy)phenyl)isoxazole; or
a pharmaceutically acceptable salt thereof is much more preferable for various purposes of the present invention.

The compound represented by the above chemical formula 1 of the present invention can be used in the form of a pharmaceutically acceptable salt, and as the salt, an acid addition salt formed by a pharmaceutically acceptable free acid is useful. The acid addition salt is obtained from inorganic acids such as hydrochloric acid, nitric acid, phosphoric acid, sulfuric acid, hydrobromic acid, hydroiodic acid, nitrous acid, phosphorous acid, and the like; non-toxic organic acids such as aliphatic mono- and dicarboxylates, phenyl-substituted alkanoates, hydroxy alkanoates and alkanedioates, aromatic acids, aliphatic and aromatic sulfonic acids; and organic acids such as acetic acid, benzoic acid, citric acid, lactic acid, maleic acid, gluconic acid, methanesulfonic acid, 4-toluenesulfonic acid, tartaric acid, and fumaric acid. The types of these pharmaceutically non-toxic salts include sulfates, pyrosulfates, bisulfates, sulfites, bisulfites, nitrates, phosphates, monohydrogen phosphates, dihydrogen phosphates, metaphosphates, pyrophosphate chlorides, bromides, iodides, fluorides, acetates, propionates, decanoates, caprylates, acrylates, formates, isobutyrates, caprates, heptanoates, propiolates, oxalates, malonates, succinates, suberates, sebacates, fumarates, maleates, butyn-1,4-dioates, hexane-1,6-dioates, benzoates, chlorobenzoates, methylbenzoates, dinitrobenzoates, hydroxybenzoates, methoxybenzoates, phthalates, terephthalates, benzenesulfonates, toluenesulfonates, chlorobenzenesulfonates, xylenesulfonates, phenylacetates, phenylpropionates, phenylbutyrates, citrates, lactates, β-hydroxybutyrates, glycolates, malates, tartrates, methanesulfonates, propanesulfonates, naphthalene-1-sulfonates, naphthalene-2-sulfonates, mandelates, etc. The acid addition salt according to the present invention can be prepared by a conventional method, for example, by dissolving the derivative of chemical formula 1 in an organic solvent such as methanol, ethanol, acetone, methylene chloride, acetonitrile, etc., adding an organic acid or inorganic acid, filtering and drying the resulting precipitate, or by distilling the solvent and an excess acid under reduced pressure, drying, and crystallizing the resultant in the organic solvent.

In one embodiment of the present invention, the pharmaceutically acceptable salt according to the present invention is a hydrochloride (for example, dihydrochloride).

In addition, a pharmaceutically acceptable metal salt can be prepared using a base. An alkali metal or alkaline earth metal salt is obtained, for example, by dissolving a compound in an excess alkali metal hydroxide or alkaline earth metal hydroxide solution, filtering the undissolved compound salt, and evaporating and drying the filtrate. At this time, it is pharmaceutically suitable to prepare a sodium, potassium or calcium salt as the metal salt. In addition, the corresponding salt is obtained by reacting an alkali metal or alkaline earth metal salt with a suitable anion salt (for example, silver nitrate).

As used herein, the terms "substituent", "radical", "group", "moiety", and "fragment" may be used interchangeably.

If a substituent is described as "optionally substituted", the substituent may be (1) unsubstituted or (2) substituted with one or more of the defined substituents. If the substitutable position is unsubstituted, the default substituent is hydrogen.

As used herein, the term "alkyl" means a saturated straight chain or branched noncyclic hydrocarbon, unless the context clearly dictates otherwise, having from 1 to 10 carbon atoms. "Lower alkyl" means alkyl having from 1 to 4 carbon atoms. Representative saturated straight chain alkyls include -methyl, -ethyl, -n-propyl, -n-butyl, -n-pentyl, -n-hexyl, -n-heptyl, -n-octyl, -n-nonyl and -n-decyl, while saturated branched alkyls include -isopropyl, - sec-butyl, -isobutyl, -tert-butyl, -isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl and the like. In a preferred embodiment of the present invention, the alkyl is methyl, ethyl, or isopropyl.

As used herein, the term "alkoxy" means -O-(alkyl), including -OCH₃, -OCH₂CH₃, - O(CH₂)₂CH₃, -OC(CH₃)₂H, -OC(CH₃)₃, and the like, wherein the alkyl is as defined above.

As used herein, if the term "C₁₋₆", "C1-6", or "C1-C6" is used, it means the number of carbon atoms is from 1 to 6. For example, C₁₋₆alkyl means an alkyl which carbon number is any integer of from 1 to 6.

As used herein, the terms "halogen" and "halo" mean fluorine, chlorine, bromine or iodine. In a preferred embodiment of the present invention, the halogen is bromine or fluorine.

As used herein, the term "haloalkyl", "haloalkoxy", "haloalkenyl", or "haloalkynyl" means an alkyl, alkoxy, alkenyl, or alkynyl group, wherein one or more hydrogen atoms are substituted with halogen atoms. For example, the haloalkyl includes -CF₃, -CHF₂, -CH₂F, - CBr₃, -CHBr₂, -CH₂Br, -CC1₃, -CHC1₂, -CH₂CI, -CI₃, -CHI₂, -CH₂I, -CH₂-CF₃, -CH₂-CHF₂, - CH₂-CH₂F, -CH₂-CBr₃, -CH₂-CHBr₂, -CH₂-CH₂Br, -CH₂-CC1₃, -CH₂-CHC1₂, -CH₂-CH₂CI, - CH₂-CI₃, -CH₂-CHI₂, -CH₂-CH₂I, and the like, wherein alkyl, alkoxy and halogen are as described above. In a preferred embodiment of the present invention, haloalkyl is -CF₃. Here, alkyl and halogen are as defined above.

As used herein, the term "cycloalkyl" means a monocyclic or polycyclic saturated ring having carbon and hydrogen atoms and having no carbon-carbon multiple bonds. Examples of monocyclic rings include, but are not limited to, (C₃-C₇)cycloalkyl groups, including cyclopropyl, cyclobutyl, cyclopentyl, cyclohexyl, and cycloheptyl. Examples of polycyclic rings include, but are not limited to, fused bicyclic rings such as octahydropentalene and decahydronaphthalene; spiro rings such as spiro[3.3]heptane, spiro[3.4 ]octane, spiro[3.5]nonane, spiro[4.4]nonane, spiro[4.5]decane, and spiro[5.5]undecane; and bridged bicycle rings such as bicyclo[2.1.1]hexane, bicyclo[2.2. 1]heptane, and bicyclo[2.2.2]octane. A cycloalkyl group can be unsubstituted or optionally substituted. In an embodiment of the present invention, cycloalkyl is monocyclic ring.

The term "heterocycle" or "heterocycloalkyl" means a 4- to 7-membered monocyclic, or 7- to 12-membered bicyclic, saturated heterocyclic ring which contains from 1 to 4 heteroatoms independently selected from nitrogen, oxygen and sulfur, and wherein the nitrogen and sulfur heteroatoms can be optionally oxidized, and the nitrogen heteroatom can be optionally quaternized. Heterocycles include heteroaryls as defined above. Representative heterocycles include oxiran, oxetan, tetrahydrofuran, tetrahydropyran, 1,4-dioxane, aziridine, azetidine, pyrrolidine, piperidine, piperazine, pyrrolidinone, hydantoine, valerolactam, thiirane, thietane, tetrahydrothiophene, tetrahydrothiopyran, morpholine, tetrahydropyridine, and tetrahydropyrimidine. Heterocycles include a bicyclic ring in which part of the heterocycle is fused to a benzene or cyclopenta-1,3-diene ring. The heterocycle can be attached via any heteroatom or carbon atom. In addition, heterocycles include fused bicyclic rings, spiro rings and bridged bicyclic rings in which one or more carbon atoms of the aforementioned polycyclic rings are replaced with nitrogen, oxygen or sulfur atoms. For example, when the heteroatom is nitrogen, these include, but are limited to, fused heterobicyclic rings such as octahydrocyclopenta[c]pyrrole, octahydropyrrolo[3,4-c]pyrrole, decahydroisoquinoline, and decahydro-2,6-naphthyridine; spiro rings such as 2-azaspiro[3.3]heptane, 2,6-diazaspiro[3.3]heptane, 2-azaspiro[3.4]octane, 2,6-diazaspiro[3.4]octane, 2-azaspiro[3.5]nonane, 2,7-diazaspiro[3.5]nonane, 2-azaspiro[4.4]nonane, 2,7-diazaspiro[4.4]nonane, 8-azaspiro[4.5]decane, 2,8-diazaspiro[4.5]decane, 3-azaspiro[5.5]undecane, and 3,9-diazaspiro[5.5]undecane; and bridged heterobicyclic rings such as 2-azabicyclo[2.1.1]hexane, 2-azabicyclo[2.2.1]heptane, 2,5-diazabicyclo[2.2.1]heptane, 2-azabicyclo[2.2.2]octane, 2,5-diazabicyclo[2.2.2]octane, and 3,8-diazabicyclo[3.2.1]octane.

As used herein, the term "aryl" means a carbocyclic aromatic group containing from 5 to 10 atoms. Representative examples include, but are not limited to, phenyl (benzene), tolyl, xylyl, naphthyl, tetrahydronaphthyl, anthracenyl, fluorenyl, indenyl, and azulenyl. A carbocyclic aromatic group can be unsubstituted or optionally substituted.

As used herein, the term "heteroaryl" means an aromatic heterocycle ring of 5- to 10 members and having at least one heteroatom selected from nitrogen, oxygen and sulfur, and containing at least 1 carbon atom, including both mono- and bicyclic ring systems. Representative heteroaryls are furan, 4H-pyran, pyrrole, imidazole, pyrazole, triazole, tetrazole, pyridine, pyrimidine, pyridazine, pyrazine, triazine, thiophene, ozaxole, isoxazole, thiazole, isothiazole, oxadiazole, benzofuran, benzothiophene, quinoline, dihydroquinoline, isoquinoline, dihydroisoquinoline, indole, benzoxazole, benzimidazole, benzothiazole, cinnoline, phthalazine, quinazoline, 1H-azepine, thiadiazole, tetrahydroisoquinoline, tetrahydropyrazolopyrazine, etc. Unless otherwise specified in the above heteroaryl, dihydro and tetrahydro forms such as 2,3-dihydro-1H-benzo[d]imidazole, 4,5,6,7-tetrahydrothieno[3,2-c]pyridine, and 5,6,7,8-tetrahydroimidazo[1,2-a]pyrazine, in which hydrogen atoms are added to the double bond, are also included in the definition.

As used herein, the phrase "compound(s) of this/the invention" includes any compound(s) of Chemical Formula 1, as well as clathrates, hydrates, solvates, or polymorphs thereof. And, even if the term "compound(s) of the invention" does not mention its pharmaceutically acceptable sat, the term includes salts thereof. In one embodiment, the compounds of this disclosure include stereo-chemically pure compounds, e.g., those substantially free (e.g., greater than 85% ee, greater than 90% ee, greater than 95% ee, greater than 97% ee, or greater than 99% ee) of other stereoisomers. That is, if the compounds of Chemical Formula 1 according to the present disclosure or salts thereof are tautomeric isomers and/or stereoisomers (e.g., geometrical isomers and conformational isomers), such isolated isomers and their mixtures also are included in the scope of this disclosure. If the compounds of the present disclosure or salts thereof have an asymmetric carbon in their structures, their active optical isomers and their racemic mixtures also are included in the scope of this disclosure.

As used herein, the term "polymorph" refers to solid crystalline forms of a compound of this disclosure or complex thereof. Different polymorphs of the same compound can exhibit different physical, chemical and/or spectroscopic properties. Different physical properties include, but are not limited to stability (e.g., to heat or light), compressibility and density (important in formulation and product manufacturing), and dissolution rates (which can affect bioavailability). Differences in stability can result from changes in chemical reactivity (e.g., differential oxidation, such that a dosage form discolors more rapidly when comprised of one polymorph than when comprised of another polymorph) or mechanical characteristics (e.g., tablets crumble on storage as a kinetically favored polymorph converts to thermodynamically more stable polymorph) or both (e.g., tablets of one polymorph are more susceptible to breakdown at high humidity). Different physical properties of polymorphs can affect their processing. For example, one polymorph might be more likely to form solvates or might be more difficult to filter or wash free of impurities than another due to, for example, the shape or size distribution of particles of it.

As used herein, the term "solvate" means a compound or its salt according to this disclosure that further includes a stoichiometric or non-stoichiometric amount of a solvent bound by non-covalent intermolecular forces. Preferred solvents are volatile, non-toxic, and acceptable for administration to humans in trace amounts.

As used herein, the term "hydrate" means a compound or its salt according to this disclosure that further includes a stoichiometric or non-stoichiometric amount of water bound by non-covalent intermolecular forces.

As used herein, the term "clathrate" means a compound or its salt in the form of a crystal lattice that contains spaces (e.g., channels) that have a guest molecule (e.g., a solvent or water) trapped within.

As used herein, the term "purified" means that when isolated, the isolate is greater than 90% pure, in one embodiment greater than 95% pure, in another embodiment greater than 99% pure and in another embodiment greater than 99.9% pure.

In addition, the present invention provides a pharmaceutical composition for preventing or treating eye diseases, particularly dry macular degeneration, comprising a compound represented by the chemical formula 1 or a pharmaceutically acceptable salt thereof as an active agent.

In one embodiment of the present invention, the compound represented by the chemical formula 1 according to the present invention increases autophagy activity and promotes autophagy flux, for example, increases the removal effect for unnecessary components accumulated in the eye, so the compound represented by the chemical formula 1 of the present invention or a pharmaceutically acceptable salt thereof can be used for preventing or treating eye diseases, for example, eye diseases caused by unnecessary components such as metabolites in cells of the eye, and can be particularly useful for preventing or treating dry macular degeneration.

In the present invention, "treatment" means all acts in which the symptoms of an eye disease are improved or beneficially changed by administering a composition according to the present invention.

In the present invention, "prevention" means all acts in which the onset of an eye disease is inhibited or delayed by administering a composition according to the present invention.

In the present invention, "eye disease" may include any disease caused by unnecessary components accumulating in the eye, for example, and may specifically mean a retinal disease caused by A2E and blue light. Specifically, the eye disease is dry macular degeneration. In addition, A2E and blue light may worsen glaucoma, retinitis pigmentosa, Stargardt disease, choroideremia, gyrate-atrophy, dry eye syndrome, eye strain, etc., and the compound of the present invention or a pharmaceutically acceptable salt thereof may be useful for the treatment or prevention of these conditions.

The exact etiology of age-related macular degeneration, especially dry macular degeneration, is known to be the accumulation of excessive pigment substances in retinal pigment epithelial cells due to aging, which appears in the early stage of macular degeneration, and A2E, which is produced by the synthesis of all-trans-retinal and ethanolamine, is a substance that is typically accumulated in retinal epithelial cells, which can cause damage to retinal pigment epithelial cells when it generates singlet oxygen by blue light and the carbon-carbon double bond site is oxidized.

Prevention or treatment of the above eye diseases, especially dry macular degeneration, can be achieved by increasing autophagy activity in ocular cells and promoting autophagy flux. It can also be achieved by suppressing cell death caused by A2E and blue light.

In one embodiment of the present invention, the prevention or treatment of the eye disease may be achieved by recovering the ONL (outer nuclear layer) and/or IS/OS (inner segments/outer segments layer) damaged by exposure of N-retinylidene-N-retinyl-ethanolamine (A2E) accumulated in the eye cells to blue light.

The compound represented by chemical formula 1 according to the present invention or a pharmaceutically acceptable salt thereof increases autophagy activity.

Autophagy is a destructive mechanism that naturally decomposes unnecessary or non-functional cellular components, and components such as unnecessary metabolic products for various cells are isolated from other components in the cell and surrounded by a double membrane, which is called autophagosome formation. The autophagosome then fuses with lysosomes and decomposes the contents. For example, in the case of dry macular degeneration, metabolites such as A2E are the cause. A2E is a metabolite derived from retinol, and A2E is created when trans-retinal meets phosphatidylethanolamine. When A2E is exposed to blue light, it forms an A2E-epoxide structure, which creates reactive oxygen species in the process, causing irreversible damage or cell death of retinal cells. In young or healthy people, unnecessary metabolites such as A2E are removed from cells through autophagy activity. However, in the case of the elderly, where autophagy activity is reduced or damaged, A2E is not smoothly removed from retinal cells, which eventually causes retinal cell damage and death, and dry macular degeneration.

In one embodiment of the present invention, it was surprisingly confirmed that the 3-phenylisoxazole derivative represented by the chemical formula 1 according to the present invention increases autophagy activity, that is, promotes autophagy flux, thereby removing unnecessary metabolites, such as A2E, from retinal cells. Therefore, the compound represented by the chemical formula 1 of the present invention or a pharmaceutically acceptable salt thereof can be used for the prevention or treatment of eye diseases, for example, eye diseases caused by unnecessary components, such as metabolites, in ocular cells, and can be particularly useful for the prevention or treatment of dry macular degeneration. The compound of the present invention can be provided as a new concept of first-in-class therapeutic agent for dry macular degeneration, for which only indirect treatments existed in the past.

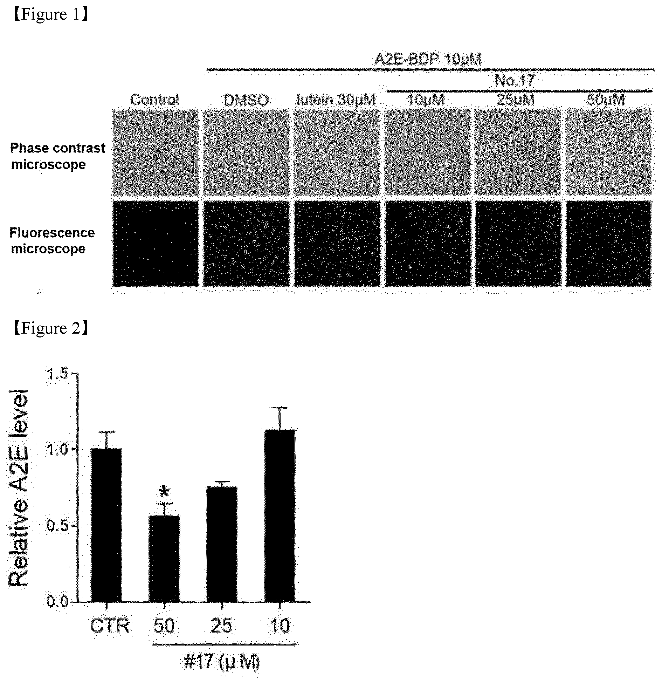

In one specific embodiment of the present invention, when A2E-fluorescent labeling substance (A2E-BDP) was accumulated in retinal pigment epithelial cells and then the compound of the present invention was treated, it was confirmed that the A2E-BDP signal was significantly reduced, confirming that the compound of the present invention removes A2E-BDP (see Table 1 and Figure 1). In addition, when A2E was accumulated in retinal pigment epithelial cells and then the compound of the present invention was treated, it was confirmed that the amount of accumulated A2E was decreased in a concentration-dependent manner, confirming that the compound of the present invention directly removes A2E (see Figure 2).

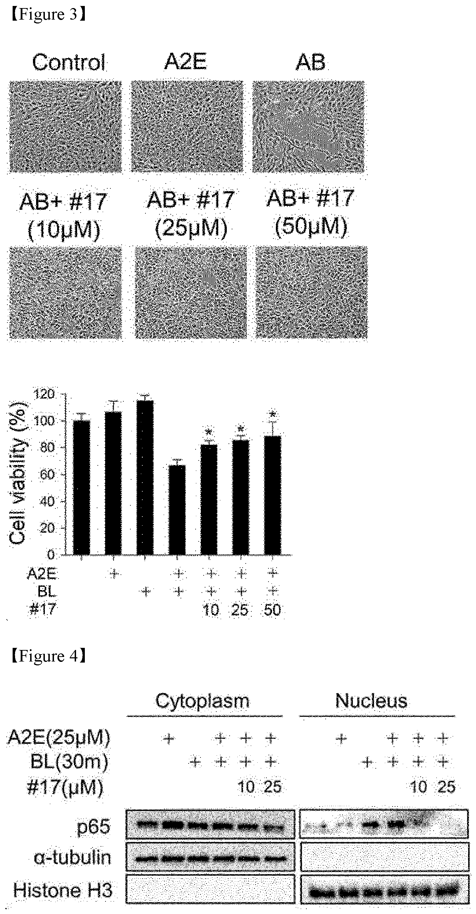

In another specific embodiment of the present invention, death of retinal pigment epithelial cells was induced with A2E and blue light, and it was confirmed that cell death was inhibited in a concentration-dependent manner when the compound of the present invention was treated (see Figure 3).

In another specific embodiment of the present invention related to the activation of NF-κB, when retinal pigment epithelial cells induced by A2E and blue light were treated with the compound of the present invention, it was confirmed that the movement of p65 induced by A2E and blue light into the nucleus was inhibited and the activation of NF-κB was inhibited (see Figure 4).

In another specific embodiment of the present invention related to the increase in the expression of inflammatory genes induced by A2E and blue light in retinal pigment epithelial cells, when retinal pigment epithelial cells were treated with the compound of the present invention, it was confirmed that the expression of inflammatory genes (IL-1β, IL-6, and CXCL8) was inhibited in a concentration-dependent manner (see Figure 5).

In another specific embodiment of the present invention, when retinal pigment epithelial cells were treated with the compound of the present invention, it was confirmed that the autophagy marker LC3-II was increased in a concentration-dependent manner (see Figure 6).

In another specific embodiment of the present invention, when retinal pigment epithelial cells were treated with the compound of the present invention under conditions with or without saturation concentration of bafilomycin A1, it was confirmed that autophagy flux increased in a concentration-dependent manner (see Figure 7).

In another specific embodiment of the present invention, when retinal pigment epithelial cells in which A2E was accumulated were treated with the compound of the present, it was confirmed that LC3-II, an autophagy marker, increased in a concentration-dependent manner (see Figure 8).

In another specific embodiment of the present invention, when retinal pigment epithelial cells in which A2E was accumulated were treated with the compound of the present invention with under conditions with or without saturation concentration of bafilomycin A1, it was confirmed that autophagy flux increased in a concentration-dependent manner (see Figure 9).

In another specific embodiment of the present invention, when retinal pigment epithelial cells were treated with the compound of the present invention with respect to autophagy of retinal pigment epithelial cells inhibited by A2E and blue light, it was confirmed that the p62 protein concentration was decreased in a concentration-dependent manner, thereby restoring autophagy activity in human retinal pigment epithelial cells that had been reduced by A2E and blue light (see Figure 10).

In another specific embodiment of the present invention, when blue light was irradiated to an experimental animal to damage retinal pigment epithelial cells, and then the compound of the present invention was treated, it was confirmed that retinal damage was improved (see Figure 11) and ONL and IS/OS thickness were also restored (see Figure 12).

Therefore, the compound of chemical formula 1 of the present invention is useful for preventing or treating eye diseases, for example, eye diseases caused by unnecessary components accumulated in the eye, and is particularly useful for preventing or treating eye diseases caused by A2E and blue light.

The pharmaceutical composition of the present invention can be used as a single agent, and can be manufactured and used as a combinational preparation by additionally comprising a pharmaceutical composition known to have an approved prevention or treatment effect for ophthalmic disease. It can be formulated into a pharmaceutical unit dosage form by adding a pharmaceutically acceptable carrier, excipient, or diluent.

**In** the present invention, "pharmaceutically acceptable" means not significantly stimulating a living organism and not inhibiting the biological activity and properties of the administered active agent.

**In** the present invention, the pharmaceutical composition comprising the pharmaceutically acceptable carrier can have any one dosage form selected from the group consisting of tablets, pills, powders, granules, capsules, suspensions, oral solutions, emulsions, syrups, sterilized aqueous solutions, non-aqueous solvent preparations, suspensions, emulsions, lyophilized preparations, and suppositories.

The pharmaceutical composition can be in various oral or parenteral dosage forms. When formulating, it can be prepared using diluents or excipients such as fillers, bulking agents, binders, wetting agents, disintegrants, and surfactants that are commonly used.

Solid preparations for oral administration include tablets, pills, powders, granules, capsules, etc., and these solid preparations can be prepared by mixing one or more compounds with at least one excipient, such as starch, calcium carbonate, sucrose or lactose, gelatin, etc. In addition to simple excipients, lubricants such as magnesium stearate and talc can also be used. Liquid preparations for oral administration include suspensions, oral solutions, emulsions, syrups, etc., and in addition to commonly used simple diluents such as water and liquid paraffin, various excipients such as wetting agents, sweeteners, flavoring agents, and preservatives can be contained.

Preparations for parenteral administration can include sterile aqueous solutions, non-aqueous solvent preparations, suspensions, emulsions, lyophilized preparations, and suppositories. Non-aqueous solvents and suspending agents may include propylene glycol, polyethylene glycol, vegetable oils such as olive oil, and injectable esters such as ethyl oleate. Suppository bases may include, but are not limited to, witepsol, macrogol, Tween 61, cacao butter, laurin butter, and glycerogelatin. Specifically, eye drops for intraocular administration, such as eye drops, may be included.

In the composition of the present invention, the compound of formula 1 may be comprised in a pharmaceutically effective amount. The term "pharmaceutically effective amount" means an amount sufficient to treat a disease at a reasonable benefit/risk ratio applicable to medical treatment and not causing side effects, and the effective dosage level may be determined based on factors including the patient's health condition, the type and severity of the disease, the activity of the drug, the sensitivity to the drug, the method of administration, the time of administration, the route of administration and the excretion rate, the treatment period, the combination or concurrent use of drugs, and other factors well known in the medical field. The composition of the present invention may be administered as an individual therapeutic agent or in combination with other therapeutic agents, may be administered sequentially or simultaneously with conventional therapeutic agents, and may be administered singly or in multiple doses. It is important to administer an amount that can obtain the maximum effect with the minimum amount without side effects by considering all of the above factors, and this can be easily determined by those skilled in the art.

For example, the compound of the present invention or its pharmaceutically acceptable salt can be administered by any suitable route in the form of a pharmaceutical composition adapted to such a route, and in a dose effective for the treatment intended. An effective dosage is typically in the range of about 0.001 to about 100 mg per kg body weight per day, preferably about 0.01 to about 50 mg/kg/day, in single or divided doses. Depending on age, species and disease or condition being treated, dosage levels below the lower limit of this range may be suitable. In other cases, still larger doses may be used without harmful side effects. Larger doses may also be divided into several smaller doses, for administration throughout the day.

Furthermore, the present invention provides a nutraceutical composition for preventing or improving eye diseases comprising a compound represented by the chemical formula 1 or a pharmaceutically acceptable salt thereof as an active agent.

In the present invention, "improvement" means all acts of reducing parameters related to a condition treated by ingestion of the composition, for example, the degree of symptoms.

The health food means a food manufactured and processed in the form of tablets, capsules, powders, granules, liquids, and pills using raw materials or ingredients having useful functionality for the human body. The nutraceutical composition of the present invention can be manufactured by a method commonly used in the art, and can be manufactured by adding raw materials and ingredients commonly added in the art during the manufacturing process. In addition, the formulation of the nutraceutical composition can be manufactured without limitation as long as it is a formulation recognized as a nutraceutical composition.

In addition, the present invention provides a method for preventing or treating an eye disease, which comprises administering a compound represented by the chemical formula 1 or a pharmaceutically acceptable salt thereof to a subject in need of treatment.

In the present invention, the term "subject" includes both mammals and non-mammals that have developed or are likely to develop the eye disease, and for example, all animals including monkeys, cows, horses, sheep, pigs, chickens, turkeys, quails, cats, dogs, mice, rats, rabbits or guinea pigs, including humans, and is preferably humans.

In the present invention, the term "administration" means providing a predetermined substance to a patient by any appropriate method, and the route of administration of the composition of the present invention may be administered through any general route as long as it can reach the target tissue. For example, intravenous administration, oral administration, topical administration, etc. are included, but are not limited thereto.

In addition, the present invention provides a pharmaceutical or nutraceutical composition for protecting retinal damage caused by blue light, comprising a compound represented by the above chemical formula 1 or a pharmaceutically acceptable salt thereof as an active agent.

In addition, the present invention provides a method for improving or protecting retinal damage caused by blue light, comprising administering a compound represented by the above chemical formula 1 or a pharmaceutically acceptable salt thereof to a subject in need of improvement or protection.

Furthermore, the present invention provides a use of the compound represented by the above chemical formula 1 or a pharmaceutically acceptable salt thereof in the manufacture of a product for improving retinal damage caused by blue light.

Hereinafter, the present invention will be described in detail by examples and experimental examples.

However, the following examples and experimental examples are merely illustrative of the present invention, and the content of the present invention is not limited to the following examples and experimental examples.

### <Example 1> Preparation of 3-(5-bromo-2-methoxyphenyl)-5-propylisoxazole

The target compound was prepared by performing steps 1 and 2 of Example 17 below and performing step 3 using pentyne instead of propargyl alcohol in step 3.

### <Example 2> Preparation of 4-bromo-2-(5-propylisoxazole-3-yl)phenol

The target compound was prepared as in Example 1 above, except that 5-bromo-2-hydroxybenzaldehyde was used instead of 5-bromo-2-methoxybenzaldehyde as a starting material.

### <Example 3> Preparation of 3-(2-methoxy-5-propylphenyl)-5-propylisoxazole

Steps 1 and 2 of Example 17 were performed, step 3 was performed using pentyne instead of propargyl alcohol in step 3, and step 4 was performed. The obtained compound was reacted using H₂ and Pd/C in methanol, and the obtained product was purified to prepare the target compound.

### <Example 4> Preparation of 3-(5-allyl-2-methoxyphenyl)-5-propylisoxazole

Steps 1 and 2 of Example 17 were performed, and step 3 was performed using pentene instead of propargyl alcohol. Then, step 4 was performed to prepare the target compound.

### <Example 5> Preparation of 4-allyl-2-(5-propylisoxazole-3-yl)phenol

The target compound was prepared as in Example 4 above, except that 5-bromo-2-hydroxybenzaldehyde was used instead of 5-bromo-2-methoxybenzaldehyde as a starting material.

### <Example 6> Preparation of 4-propyl-2-(5-propylisoxazole-3-yl)phenol

The target compound was prepared as in Example 3 above, except that 5-bromo-2-hydroxybenzaldehyde was used instead of 5-bromo-2-methoxybenzaldehyde as the starting material.

### <Example 7> Preparation of (3-(5-bromo-2-methoxyphenyl)isoxazole-5-yl)methanol

The target compound was prepared by performing step 3 of Example 17 below.

### <Example 8> Preparation of 4-bromo-2-(5-(hydroxymethyl)isoxazole-3-yl)phenol

The target compound was prepared as in Example 7 above, except that 5-bromo-2-hydroxybenzaldehyde was used instead of 5-bromo-2-methoxybenzaldehyde as a starting material.

### <Example 9> Preparation of 5-((allyloxy)methyl)-3-(5-bromo-2-methoxyphenyl)isoxazole

The target compound was prepared by performing steps 1, 2, 3, and 5 as in Example 17 below, excluding step 4.

### <Example 10> Preparation of (3-(2-methoxy-5-propylphenyl)isoxazole-5-yl)methanol

After performing step 4 of Example 17 below, the obtained compound was reacted using H₂ and Pd/C in methanol, and the obtained product was purified to prepare the target compound.

### <Example 11> Preparation of 5-((benzyloxy)methyl)-3-(5-bromo-2-methoxyphenyl)isoxazole

After performing step 3 of Example 17 below, the obtained compound was reacted with NaH and benzyl bromide in DMF, and the obtained product was purified to prepare the target compound.

### <Example 12> Preparation of ethyl 2-((3-(5-bromo-2-methoxyphenyl)isoxazole-5-yl)methoxy)acetate

The target compound was prepared as in Example 11 above, except using ethyl 2-bromoacetate instead of benzyl bromide.

### <Example 13> Preparation of 3-(5-bromo-2-methoxyphenyl)-5-(methoxymethyl)isoxazole

The target compound was prepared as in Example 11 above, except using bromomethane instead of benzyl bromide.

### <Example 14> Preparation of 2-(5-(hydroxymethyl)isoxazole-3-yl)-4-propylphenol

The target compound was prepared as in Example 10 above, except that 5-bromo-2-hydroxybenzaldehyde was used instead of 5-bromo-2-methoxybenzaldehyde as a starting material.

### <Example 15> Preparation of (3-(5-allyl-2-methoxyphenyl)isoxazole-5-yl)methanol

The target compound was prepared by performing step 4 of Example 17 below.

### <Example 16> Preparation of 4-allyl-2-(5-(hydroxymethyl)isoxazole-3-yl)phenol

The target compound was prepared as in Example 15 above, except that 5-bromo-2-hydroxybenzaldehyde was used instead of 5-bromo-2-methoxybenzaldehyde as a starting material.

### <Example 17> Preparation of 3-(5-allyl-2-methoxyphenyl)-5-((allyloxy)methyl)isoxazole

### Step 1: Preparation of (E)-5-bromo-2-methoxybenzaldehyde oxime

Hydroxylamine hydrochloride (320 mg, 4.60 mmol) was added to a solution of 5-bromo-2-methoxybenzaldehyde (1 g, 4.65 mmol) in 0.5M NaOH H₂O (23 mL). The mixture was stirred at room temperature for 4 hours. The reaction mixture was diluted with dichloromethane. And the organic layer was washed with brine and water. The combined organic layers were dried over Na₂SO₄, concentrated under reduced pressure and purified by flash column chromatography on silica gel (EtOAc/hexane = 1:20) to obtain the target compound (960 mg, 90%).

¹H NMR (600 MHz, CDCl₃) δ 8.39 (s, 1H), 7.78 (d, J=2.5 Hz, 1H), 7.43 (dd, J=8.8, 2.5 Hz, 1H), 3.84 (s, 3H). ¹³C NMR (150 MHz, CDCl₃) δ 156.7, 145.7, 133.8, 129.8, 122.6, 113.3, 113.0, 56.0.

### Step 2: Preparation of (Z)-5-bromo-N-hydroxy-2-methoxybenzimidoyl chloride

N-chlorosuccinimide (551 mg, 4.13 mmol) was added to a solution of (E)-5-bromo-2-methoxybenzaldehyde oxime (950 mg, 4.13 mmol) in DMF (4 mL). The mixture was stirred at room temperature for 1 hour. The reaction mixture was diluted with dichloromethane. The organic layer was then washed with brine and water. The combined organic layers were dried over Na₂SO₄ and concentrated under reduced pressure.

### Step 3: Preparation of (3-(5-bromo-2-methoxyphenyl)isoxazole-5-yl)methanol

To the mixture generated in step 2 above were added propargyl alcohol (0.26 mL, 4.54 mmol), CuSO₄.5H₂O (31 mg, 0.12 mmol), sodium ascorbate (82 mg, 0.41 mmol), t-BuOH/H₂O (1/1, 20 mL), and KHCO₃ (1.24 g, 12.4 mmol). The mixture was stirred at room temperature overnight. The reaction mixture was diluted with dichloromethane. And the organic layer was washed with brine and water. The combined organic layers were dried over Na₂SO₄, concentrated under reduced pressure, and purified by flash column chromatography on silica gel (EtOAc/hexane = 1:3) to obtain the target compound (630 mg, 54%).

¹H NMR (600 MHz, CDCl₃) δ 7.96 (d, J=2.5 Hz, 1H), 7.47 (dd, J=8.8, 2.5 Hz, 1H), 6.85 (d, J=8.8 Hz, 1H), 6.72 (s, 1H), 4.80 (s, 2H), 3.85 (s, 3H), 2.61 (s, 2H). ¹³C NMR (150 MHz, CDCl₃) δ 171.1, 159.1, 156.4, 133.9, 132.0, 119.7, 113.3, 113.3, 103.5, 56.6, 56.0.

### Step 4: Preparation of (3-(5-allyl-2-methoxyphenyl)isoxazole-5-yl)methanol.

To a solution of (3-(5-bromo-2-methoxyphenyl)isoxazole-5-yl)methanol (500 mg, 1.76 mmol) and Pd(PPh₃)₄ (203 mg, 0.176 mmol) in DMF (6 mL) was added allyltributyl tin (Sn) (0.65 mL, 2.11 mmol) in a shielded tube. After that, nitrogen gas was bubbled into the solvent for 10 minutes to remove air. The mixture was heated to 90 °C and stirred for 2 hours. The reaction mixture was quenched with NH₄Cl solution and diluted with ethyl acetate. And the organic layer was washed with brine and water. The combined organic layers were dried over Na₂SO₄, concentrated under reduced pressure and purified by flash column chromatography on silica gel (EtOAc/hexane = 1 : 4) to obtain the target compound (350 mg, 81%).

¹H NMR (600 MHz, CDCl₃) δ 7.70 (d, J=2.3 Hz, 1H), 7.23 (dd, J=8.5, 2.3 Hz, 1H), 6.94 (d, J=8.5 Hz, 1H), 6.76 (s, 1H), 5.96 (ddt, J=16.9, 10.0, 6.7 Hz, 1H), 5.14-4.98 (m, 2H), 4.82 (d, J=4.8 Hz, 2H), 3.88 (s, 3H), 3.37 (d, J=6.7 Hz, 2H). ¹³C NMR (150 MHz, CDCl₃) δ 170.4, 160.3, 155.8, 137.5, 132.7, 131.4, 129.7, 117.8, 116.1, 111.7, 103.7, 56.8, 55.8.

### Step 5: Preparation of 3-(5-allyl-2-methoxyphenyl)-5-((allyloxy)methyl)isoxazole

To a solution of (3-(5-allyl-2-methoxyphenyl)isoxazole-5-yl)methanol (340 mg, 1.39 mmol) in DMF (4 mL) was added allyl bromide (0.16 mL, 01.86 mmol). Then, NaH (57 mg, 1.43 mmol) was added at 0 °C. The resulting mixture was warmed to room temperature and stirred for 2 hours. The reaction mixture was quenched with NH₄Cl solution and diluted with ethyl acetate. The combined organic layers were dried over Na₂SO₄, concentrated under reduced pressure and purified by flash column chromatography on silica gel (EtOAc/hexane = 1:10) to obtain the target compound (370 mg, 93%).

### <Example 18> Preparation of 3-(5-allyl-2-methoxyphenyl)-5-((benzyloxy)methyl)isoxazole

The target compound was prepared as in Example 17 above, except that benzyl bromide was used instead of allyl bromide in step 5.

### <Example 19> Preparation of ethyl 2-((3-(5-allyl-2-methoxyphenyl)isoxazole-5-yl)methoxy)acetate

The target compound was prepared as in Example 17 above, except that ethyl 2-bromoacetate was used instead of allyl bromide in step 5.

### <Example 20> Preparation of 3-(2-methoxy-5-propylphenyl)-5-(methoxymethyl)isoxazole

The procedures were performed as in Example 17 above, except using bromomethane instead of allyl bromide in step 5, and the obtained compound was reacted with H₂ and Pd/C in methanol, and the obtained product was purified to prepare the target compound.

### <Example 21> Preparation of tert-butyl 4-((3-(5-bromo-2-methoxyphenyl)isoxazole-5-yl)methyl)piperazine-1-carboxylate

The target compound was prepared by performing step 3 of Example 17 above, except using tert-butyl 4-(pro-2-pyn-1-yl)piperazine-1-carboxylate instead of propargyl alcohol in step 3.

### <Example 22> Preparation of 3-(5-allyl-2-methoxyphenyl)-5-((4-allylpiperazine-1-yl)methyl)isoxazole

The compound prepared in Example 21 above was reacted with Pd(PPh₃)₄ and allyltributyl tin (Sn) under toluene. The obtained compound was reacted with CH₂Cl₂ under TFA to remove the Boc group, and then reacted with NaH and allyl bromide under THF to prepare the target compound.

### <Example 23> Preparation of 3-(5-allyl-2-methoxyphenyl)-5-((4-benzylpiperazine-1-yl)methyl)isoxazole

The target compound was prepared as in Example 22 above, except using benzyl bromide instead of allyl bromide.

### <Example 24> Preparation of ethyl 2-(4-((3-(5-allyl-2-methoxyphenyl)isoxazole-5-yl)methyl)piperazine-1-yl)acetate

The target compound was prepared as in Example 22 above, except using ethyl bromoacetate instead of allyl bromide.

### <Example 25> Preparation of tert-butyl 4-((3-(5-allyl-2-methoxyphenyl)isoxazole-5-yl)methyl)piperazine-1-carboxylate

The compound prepared in Example 21 above was reacted with Pd(PPh₃)₄ and allyltributyl tin (Sn) in toluene to prepare the target compound.

### <Example 26> Preparation of tert-butyl 4-((3-(2-methoxy-5-propylphenyl)isoxazole-5-yl)methyl)piperazine-1-carboxylate

The compound prepared in Example 25 was reacted by adding H₂ and Pd/C to methanol, and the obtained product was purified to prepare the target compound.

### <Example 27> Preparation of 4-allyl-2-(5-((allyloxy)methyl)isoxazole-3-yl)phenol

The compound prepared in Example 17 (15 mg) was dissolved in 2 mL of dichloromethane. Then, boron tribromide (0.15 mL, 1 M dichloromethane solution) was added at 0°C and stirred under a nitrogen atmosphere. After completion of the reaction, the mixture was diluted with dichloromethane and the organic layer was washed with brine and water. The combined organic layers were dried over Na₂SO₄, concentrated under reduced pressure, and purified by flash column chromatography on silica gel (EtOAc/hexane = 1/3) to obtain the target compound (13 mg, 90%).

### <Example 28> Preparation of 3-(5-bromo-2-methoxyphenyl)-5-(piperazine-1-ylmethyl)isoxazole hydrochloride salt

To the compound prepared in Example 21 (53 mg) was added 1 mL of hydrochloric acid solution dissolved in dioxane, and stirred for 4 hours. The reactant was concentrated under reduced pressure, and the resultant was purified by flash column chromatography on silica gel (dichloromethane/methanol=10/1) to obtain the target compound (30 mg, 66%).

### <Example 29> Preparation of 3-(5-allyl-methoxyphenyl)-5-(piperazine-1-ylmethyl)isoxazole· hydrochloride salt

To the compound prepared in Example 25 (25 mg) above, 0.6 mL of hydrochloric acid solution dissolved in dioxane was added, and the mixture was stirred for 4 hours. The reactant was concentrated under reduced pressure, and the resultant was purified by flash column chromatography on silica gel (dichloromethane/methanol=10/1) to obtain the target compound (14 mg, 77%).

### <Example 30> Preparation of 3-(2-methoxy-5-propylphenyl)-5-(piperazine-1-ylmethyl)isoxazole· hydrochloride salt

To the compound prepared in Example 26 (49 mg) above, 1 mL of hydrochloric acid solution dissolved in dioxane was added, and the mixture was stirred for 4 hours. The reactant was concentrated under reduced pressure, and the resultant was purified by flash column chromatography on silica gel (dichloromethane/methanol=10/1) to obtain the target compound (26 mg, 53%).

### <Example 31> Preparation of (3-(2-methoxy-5-(trifluoromethyl)phenyl)isoxazole-5-yl)methanol

The target compound was prepared by performing up to step 3 of Example 17 above, except using 2-methoxy-5-trifluoromethyl benzaldehyde as a starting material.

### <Example 32> Preparation of 2-(3-(2-methoxy-5-(trifluoromethyl)phenyl)isoxazole-5-yl)propan-2-ol

The target compound was prepared by the same method as in Example 31 above, except that 2,2-dimethylpropargyl alcohol was used in step 3.

### <Example 33> Preparation of 2-(3-(2-methoxy-5-(trifluoromethyl)phenyl)isoxazole-5-yl)ethan-1-ol

The target compound was prepared by the same method as in Example 31 above, except that but-3-yn-1-ol was used in step 3.

### <Example 34> Preparation of 3-(2-methoxy-5-(trifluoromethyl)phenyl)-5-(piperazine-1-ylmethyl)isoxazole

An intermediate was synthesized as in the method of Example 31 above, except that t-butyl-4-(pro-2-pyn-1-yl)-piperazine-1-carboxylate was used instead of propargyl alcohol in step 3. Thereafter, the Boc group was removed using a hydrochloric acid/dioxane solution to prepare the target compound.

### <Example 35> Preparation of (3-(2-methoxy-4-(trifluoromethyl)phenyl)isoxazole-5-yl)methanol

The target compound was synthesized as in the method of Example 31 above, except using 2-methoxy-4-trifluoromethyl benzaldehyde as a starting material.

### <Example 36> Preparation of 2-((3-(2-methoxy-5-(trifluoromethyl)phenyl)isoxazole-5-yl)methoxy)acetic acid

The compound prepared in Example 31 was reacted with t-butylbromo acetate/NaH under DMF, and then the t-butyl group was removed using TFA (trifluoro acetic acid) under DCM to prepare the target compound.

### <Example 37> Preparation of 4-((3-(2-methoxy-5-(trifluoromethyl)phenyl)isoxazole-5-yl)methyl)-1-methylpiperazine-2-on

The target compound was synthesized as in the method of Example 31 above, except using 1-methyl-4-(prop-2-yn-1-yl)piperazine-2-one instead of propargyl alcohol in step 3.

### <Example 38> Preparation of (3-(2-methoxy-5-morpholinophenyl)isoxazole-5-yl)methanol

Step 4 of Example 17 was performed using the compound prepared in Example 7 above, except morpholine was reacted in the presence of RuPhos-Pd-G3/cesium carbonate to prepare the target compound.

### <Example 39> Preparation of 2-(3-(5-allyl-2-methoxyphenyl)isoxazole-5-yl)propan-2-ol

The process was performed up to step 4 of Example 17 above, except 2-methylbut-3-yn-2-ol was used in step 3 to prepare the target compound.

### <Example 40> Preparation of 2-(3-(2-methoxy-5-propylphenyl)isoxazole-5-yl)propan-2-ol

The target compound was prepared by reducing the compound of Example 39 above using H₂ and Pd/C under methanol.

### <Example 41> Preparation of 3-(5-allyl-2-methoxyphenyl)-5-((4-methylpiperazine-1-yl)methyl)isoxazole

The target compound was prepared by performing up to step 4 of Example 17 above, except using 1-methyl-4-(prop-2-yn-1-yl)piperazine in step 3.

### <Example 42> Preparation of 3-(2-methoxy-5-propylphenyl)-5-((4-methylpiperazine-1-yl)methyl)isoxazole

The target compound was prepared by reducing the compound of Example 41 above using H₂ and Pd/C in methanol.

### <Example 43> Preparation of (3-(5-allyl-2-(trifluoromethoxy)phenyl)isooxazole-5-yl)methanol

The target compound was prepared as in Example 15 above, except using 5-bromo-2-(trifluoromethoxy)benzaldehyde as a starting material.

### <Example 44> Preparation of (3-(4-allyl-2-methoxyphenyl)isoxazole-5-yl)methanol

The target compound was prepared as in Example 15 above, except using 4-bromo-2-methoxybenzaldehyde as a starting material.

### <Example 45> Preparation of (3-(5-isopropyl-2-methoxyphenyl)isoxazole-5-yl)methanol

The target compound was prepared by performing the steps up to step 3 of Example 17 above, except using 5-isopropyl-2-methoxybenzaldehyde instead of 5-bromo-2-methoxybenzaldehyde as a starting material.

### <Example 46> Preparation of tert-butyl 4-((3-(5-isopropyl-2-methoxyphenyl)isoxazole-5-yl)methyl)piperazine-1-carboxylate

The target compound was prepared by performing the procedure up to step 3 of Example 17 above, except using 5-isopropyl-2-methoxybenzaldehyde as a starting material and using tert-butyl 4-(pro-2-pyn-1-yl)piperazine-1-carboxylate instead of propargyl alcohol in step 3.

### <Example 47> Preparation of 3-(5-isopropyl-2-methoxyphenyl)-5-(piperazine-1-ylmethyl)isoxazole

After performing as in Example 46 above, the Boc group was removed using hydrochloric acid/dioxane solution to prepare the target compound.

### <Example 48> Preparation of tert-butyl 4-((3-(4-allyl-2-methoxyphenyl)isoxazole-5-yl)methyl)piperazine-1-carboxylate

The target compound was prepared by performing up to step 3 of Example 17 above, except using 4-bromo-2-methoxybenzaldehyde as a starting material and using tert-butyl 4-(pro-2-pyn-1-yl)piperazine-1-carboxylate instead of propargyl alcohol in step 3.

### <Example 49> Preparation of 3-(4-allyl-2-methoxyphenyl)-5-(piperazine-1-ylmethyl)isoxazole

After performing as in Example 48 above, the Boc group was removed using hydrochloric acid/dioxane solution to prepare the target compound.

### <Example 50> Preparation of tert-butyl 4-((3-(5-allyl-2-(trifluoromethoxy)phenyl)isoxazole-5-yl)methyl)piperazine-1-carboxylate

The target compound was prepared by performing up to step 4 of Example 17 above, except using 5-bromo-2-(trifluoromethoxy)benzaldehyde as a starting material and using tert-butyl 4-(pro-2-pyn-1-yl)piperazine-1-carboxylate instead of propargyl alcohol in step 3.

### <Example 51> Preparation of 3-(5-allyl-2-(trifluoromethoxy)phenyl)-5-(piperazine-1-ylmethyl)isoxazole

After performing as in Example 50 above, the Boc group was removed using hydrochloric acid/dioxane solution to prepare the target compound.

### <Example 52> Preparation of 3-(5-allyl-2-(trifluoromethoxy)phenyl)-5-((4-methylpiperazine-1-yl)methyl)isoxazole

After performing up to step 4 of Example 17 above, the target compound was prepared in the form of a salt using hydrochloric acid/dioxane solution, except using 5-bromo-2-(trifluoromethoxy)benzaldehyde as a starting material and using 1-methyl-4-(prop-2-yn-1-yl)piperazine instead of propargyl alcohol in step 3.

### <Example 53> Preparation of (2S,6R)-4-((3-(2-methoxy-5-propylphenyl)isoxazole-5-yl)methyl)-2,6-dimethylmorpholine

### Step 1: (3-(2-methoxy-5-propylphenyl)isoxazole-5-yl)methanol

Up to step 3 of Example 17 above was performed to obtain the target compound as a yellow solid, except using 2-methoxy-5-propylbenzaldehyde as the starting material.
LC-MS, m/z [M+1]=248.1

### Step 2: 3-(2-methoxy-5-propylphenyl)isoxazole-5-carbaldehyde

The compound of step 1 (2.3 g, 9.3 mmol) was dissolved in DCM (30 mL) and Dess-Martin periodinane (4.1 g, 8.1 mmol) was added. The reaction mixture was stirred at room temperature for 4 hours, and then the reaction mixture was filtered. The filtered cake was washed with DCM (20 mL). The filtrate was concentrated to obtain the desired compound (1.7 g, yield 74%) as a yellow solid.
LC-MS, m/z [M+1]=246.1

### Step 3: (2S,6R)-4-((3-(2-methoxy-5-propylphenyl)isoxazole-5-yl)methyl)-2,6-dimethylmorpholine

The compound of step 2 (100 mg, 0.41 mmol) was dissolved in DCM, and acetic acid (0.05 mL) was added to a solution of (2S,6R)-2,6-dimethylmorpholine (56 mg, 0.49 mmol). The reactant was stirred at room temperature for 30 minutes, and NaBHAc₃ (174 mg, 0.82 mmol) was added to the reaction mixture. The reaction mixture was stirred at room temperature for 2 hours, and the reaction mixture was quenched with water and extracted with ethyl acetate. The combined organic phases were washed with brine, dried over Na₂SO₄, filtered and concentrated under reduced pressure. The residue was purified by prep-HPLC to obtain the compound in the form of a free base. This was treated with 2N HCl to obtain the title compound (94.8 mg) in the form of an HCl salt as a yellow solid.

### <Example 54> Preparation of 3-(2-methoxy-5-propylphenyl)-5-((4-methoxypiperidine-1-yl)methyl)isoxazole

The target compound was prepared by the same method as in Example 53 above, except using 4-methoxypiperidine instead of dimethylmorpholine in step 3.

### <Example 55> Preparation of 1-((3-(2-methoxy-5-propylphenyl)isoxazole-5-yl)methyl)piperidine-4-carboxylic acid

The target compound was prepared by the same method as in Example 53 above, except using piperidine-4-carboxylic acid instead of dimethylmorpholine in step 3.

### <Example 56> Preparation of 5-((6,7-dihydrothieno[3,2-c]pyridin-5(4H)-yl)methyl)-3-(2-methoxy-5-propylphenyl)isoxazole

The target compound was prepared by the same method as in Example 53, except that 4,5,6,7-tetrahydrothieno[3,2-c]pyridine was used instead of dimethylmorpholine in step 3.

### <Example 57> Preparation of 5-((5,6-dihydroimidazo[1,2-a]pyrazin-7(8H)-yl)methyl)-3-(2-methoxy-5-propylphenyl)isoxazole

The target compound was prepared by the same method as in Example 53, except that 5,6,7,8-tetrahydroimidazo[1,2-a]pyrazine was used instead of dimethylmorpholine in step 3.

### <Example 58> Preparation of 3-(4-fluoro-2-methoxy-5-propylphenyl)-5-(piperazine-1-ylmethyl)isoxazole

The procedure up to step 3 of Example 17 above was performed, except that 2-methoxy-4-fluoro-5-propylbenzaldehyde was used as a starting material, and t-butyl-4-(pro-2-pyn-1-yl)-piperazine-1-carboxylate was used instead of propargyl alcohol in step 3 to synthesize an intermediate, and then the Boc group was removed using hydrochloric acid/dioxane solution to prepare the target compound.

### <Example 59> Preparation of 3-(4-fluoro-2-methoxy-5-propylphenyl)-5-((4-methylpiperazine-1-yl)methyl)isoxazole

The target compound was prepared similarly to Example 58 above, except using 4-methylpiperazine instead of t-butyl-4-(pro-2-pyn-1-yl)-piperazine-1-carboxylate in step 3.

### <Example 60> Preparation of 5-((4-methylpiperazine-1-yl)methyl)-3-(5-propyl-2-(trifluoromethoxy)phenyl)isoxazole

The target compound was prepared by performing up to step 3 of Example 17 above, except using 2-trifluoromethoxy-5-propylbenzaldehyde as a starting material and using 1-methyl-4-(prop-2-yn-1-yl)piperazine in step 3.

### <Example 61> Preparation of 2-(4-methoxy-3-(5-((4-methylpiperazine-1-yl)methyl)isoxazole-3-yl)phenyl)ethan-1-ol

The procedure up to step 3 of Example 17 above was performed, except using 3-formyl-4-methoxyphenethyl acetate as a starting material and using 1-methyl-4-(prop-2-yn-1-yl)piperazine in Step 3 to obtain an intermediate (4-methoxy-3-(5-((4-methylpiperazin-1-yl)methyl)isoxazol-3-yl)phenethyl acetate) in which the hydroxyl group is protected by an acetyl group. The intermediate is hydrolyzed with lithium hydroxide solution to obtain the target compound.

### <Example 62> Preparation of 3-(2-methoxy-5-(2-methoxyethyl)phenyl)-5-((4-methylpiperazine-1-yl)methyl)isoxazole

The target compound was obtained by performing up to step 3 of Example 17 above, except using 2-methoxy-5-(2-methoxyethyl)benzaldehyde as a starting material and using 1-methyl-4-(prop-2-yn-1-yl)piperazine in step 3.

### <Example 63> Preparation of 1-(4-((3-(5-isopropyl-2-methoxyphenyl)isoxazole-5-yl)methyl)piperazine-1-yl)ethan-1-on

The compound prepared in Example 29 above was reacted with triethylamine and acetyl chloride in THF to prepare the target compound.

### <Example 64> Preparation of 3-(5-isopropyl-2-methoxyphenyl)-5-((4-(methylsulfonyl)piperazine-1-yl)methyl)isoxazole

The compound prepared in Example 29 above was reacted with triethylamine and methanesulfonyl chloride in THF to prepare the target compound.

### <Example 65> Preparation of 3-(5-allyl-2-methoxyphenyl)-5-((quinazoline-4-yloxy)methyl)isoxazole

The compound prepared in Example 15 above was added to 4-chloroquinazoline under DMF, and then NaH was added at 0 °C and reacted at room temperature to prepare the target compound.

### <Example 66> Preparation of tert-butyl 4-((3-(2-methoxy-5-(2-methylallyl)phenyl)isoxazole-5-yl)methyl)piperazine-1-carboxylate

The target compound was prepared by performing up to step 4 of Example 17 above, except using 1-methyl-4-(prop-2-yn-1-yl)piperazine instead of propargyl alcohol in step 3 and using tributyl (2-methylallyl)stannane instead of allyltributyl tin (Sn) in step 4.

### <Example 67> Preparation of 3-(2-methoxy-5-(2-methylallyl)phenyl)-5-(piperazine-1-ylmethyl)isoxazole

After performing as in Example 66 above, the Boc group was removed using hydrochloric acid/dioxane solution to prepare the target compound.

### <Example 68> Preparation of (3-(2-methoxy-5-(2-methylallyl)phenyl)isoxazole-5-yl)methanol

The target compound was prepared as in Example 15 above, except that tributyl (2-methylallyl)stannane was used instead of allyltributyl tin (Sn) in step 4.

### <Example 69> Preparation of 3-(2-methoxy-5-(3-methylbut-2-en-1-yl)phenyl)-5-((4-methylpiperazine-1-yl)methyl)isoxazole

After performing up to step 4 of the above Example 17, the target compound was synthesized in salt form using hydrochloric acid/dioxane solution, except that 1-methyl-4-(prop-2-en-1-yl)piperazine was used instead of propargyl alcohol in step 3, and tributyl (3-methylbut-2-en-1-yl)stannane was used instead of allyltributyl tin (Sn) in step 4, to prepare the target compound.

The NMR and Mass measurement results of the compounds prepared in the above Example 1-69 are summarized and presented in the table below.

**[Table 1]**

| Exam ple No. | NMR and Mass |
|---|---|
| 1 | ¹H NMR (400 MHz, CDCl₃) d 7.97 (d, 1H, *J*=2.4 Hz), 7.44 (dd, 1H, *J*=8.8 and 2.9 Hz), 8.83 (d, 1H, *J*=8.8 Hz), 6.43 (s, 1H), 3.84 (s, 3H), 2.75 (t, 2H, *J*=7.8 Hz), 1.79 (m, 2H), 1.00 (t, 3H, *J*=7.8 Hz); ¹³C NMR (100 MHz, CDCl₃) d 173.1, 158.7, 156.2, 133.3, 131.8, 120.2, 113.1, 112.9, 102.0, 55.8, 28.6, 20.9, 13.6; LRMS (ESI) m/z 318.0 (M+Na⁺). |
| 2 | ¹H NMR (400 MHz, CDCl₃) d 9.63 (s, 1H), 7.58 (d, 1H, *J=2.4* Hz), 7.41 (dd, 1H, *J*=2.4 and 8.7 Hz), 6.97 (d, 1H, *J*=8.8 Hz), 6.37 (s, 3H), 2.82 (t, 2H, *J*=7.8 Hz), 1.8 (m, 2H), 1.05 (t, 3H, *J*=7.3 Hz); ¹³C NMR (100 MHz, CDCl₃) d 173.6, 161.3, 155.7, 133.9, 130.1, 119.3, 115.3, 111.3, 98.4, 28.4, 20.8, 13.6; LRMS (ESI) m/z 281.0 (M+H⁺). |
| 3 | ¹H NMR (600 MHz, CDCl₃) d 7.67 (d, 1H, *J*=1.6 Hz), 7.17 (dd. 1H, *J*=1.6, 5.6 Hz), 6.88 (d, 1H, *J*=5.6 Hz), 6.46 (s, 1H), 3.84 (s, 3H), 2.74 (m, 2H), 2.54 (t, 2H, *J*=5.2 Hz), 1.76 (m, 2H), 1.62 (m, 2H), 1.02 (t, *J*=7.2 Hz, 3H), 0.93 (t, *J*=7.2 Hz, 3H); ¹³C NMR (150 MHz, CDCl₃) δ 172.7, 160.1, 155.3, 135.1, 130.8, 129.3, 118.0, 111.4, 102.3, 55.7, 37.0, 28.8, 24.7, 21.0, 13.8, 13.8. |
| 4 | ¹H NMR (400 MHz, CDCl₃) d 7.70 (d, 1H, *J*=2.6 Hz), 7.22 (dd, 1H, *J*=2.4 and 8.2 Hz), 6.93 (d, 1H, *J*=8.8 Hz), 6.47 (s, 1H), 6.01 (m, 1H), 5.10 (m, 2H), 3.87 (s, 3H), 3.37 (d. 2H, *J*=6.3 Hz), 2.78 (t, 2H, *J*=7.3 Hz), 1.82(m, 2H), 1.04 (t, 3H, *J=7.3* Hz); ¹³C NMR (100 MHz, CDCl₃) d 172.7, 159.9, 155.6, 137.4, 132.4, 130.8, 129.5, 118.2, 115.7, 111.5, 102.2, 55.6, 39.2,.28.7, 20.9, 13.7.; LRMS (ESI) m/z 296.0 (M+K⁺). |
| 5 | ¹H NMR (400 MHz, CDCl₃) d 9.47 (br s, 1H), 7.27 (d, 1H, *J*=1.9 Hz), 7.16 (dd, 1H, *J*=8.3 and 2.4 Hz), 7.01 (d, 1H, *J*=8.3 Hz), 6.4 (s, 1H), 6.01 (m, 1H), 5.10 (m, 2H), 3.37 (d, 2H, *J*=6.8 Hz) 2.81 (t, 2H, *J*=7.3 Hz) 1.84 (m, 2H), 1.05 (t, 3H, *J*=7.3 Hz); ¹³C NMR (100 MHz, CDCl₃) d 173.0, 162.4, 154.9, 137.4, 131.7, 131.7, 130.9, 127.5, 117.3, 115.7, 113.2, 98.4, 39.1, 28.3, 20.8, 13.5; LRMS (ESI) m/z 244.1 (M+H+) and 266.1 (M+Na⁺). |
| 6 | ¹H NMR (600 MHz, CDCl₃) δ 9.42 (s, 1H), 7.25 (d, *J*=1.8 Hz, 1H), 7.12 (dd, *J*=8.4, 1.8 Hz, 1H), 6.98 (d, *J*=8.4 Hz, 1H), 6.39 (s, 1H), 2.78-2.75 (m, 2H), 2.54 (t, *J*=1.8 Hz, 2H), 1.81-1.74 (m, 2H), 1.65-1.59 (m, 2H), 1.02 (t, *J*=7.2 Hz, 3H), 0.93 (t, *J=7.2* Hz, 3H); ¹³C NMR (150 MHz, CDCl₃) δ 173.1, 162.6, 154.6, 133.7, 131.6, 127.5, 117.2, 113.1, 98.6. 37.1, 28.5, 24.8, 20.9, 13.7, 13.6. |
| 7 | ¹H NMR (400 MHz, CDCl₃) δ 7.98 (d, 1H, *J*=2.4 Hz), 7.49 (dd, 1H, *J*=9.3, 2.9 Hz), 6.86 (d, 1H, *J*=9.3 Hz), 6.71 (s, 1H), 4.80 (d, 2H, *J*=5.4 Hz), 3.85 (s, 3H), 2.20 (br s, 1H); ¹³C NMR (100 MHz, CDCl₃) d 170.7, 158.9, 156.2, 133.7, 131.9, 119.6, 113.1, 103.3, 56.6, 55.8, 29.6; LRMS (ESI) m/z 305.9 (M+Na⁺). |
| 8 | ¹H NMR (600 MHz, CDCl₃) δ 9.46 (s, 1H), 7.57 (d, *J*=2.4 Hz, 1H), 7.40 (dd, *J*=8.4, 2.4 Hz, 1H), 6.96 (d, *J*=8.4 Hz, 1H), 6.64 (s, 1H), 4.85 (s, 2H); ¹³C NMR (150 MHz, CDCl₃) δ 171.3, 161.5, 155.7, 134.4, 130.29, 119.5, 114.9, 111.5, 99.6, 56.5. |
| 9 | ¹H NMR (600 MHz, CDCl₃) δ 8.00 (d, *J*=3 Hz, 1H), 7.45 (dd, *J=9,* 2.4 Hz, 1H), 6.84 (d, *J*=9 Hz, 1H), 6.74 (s, 1H), 5.95-5.88 (m, 1H), 5.32 (ddd, *J*=17.2, 3.1, 1.5 Hz, 1H), 5.23 (ddd, *J*=10.4, 2.6, 1.2 Hz, 1H), 4.61 (*d*, *J*=0.6 Hz, 2H), 4.10-4.08 (m, 2H), 3.84 (s, 3H). ¹³C NMR (150 MHz, CDCl₃) δ 168.9, 158.9, 156.3, 133.8, 133.7, 131.8, 119.7, 118.2, 113.2, 113.1, 104.5, 71.9, 62.7, 55.9. HRMS (EI): mass calculated for C₁₄H₁₄BrNO₃ [M⁺], 323.0157; found, 323.0157. |
| 10 | ¹H NMR (600 MHz, CDCl₃) δ 7.67 (d, *J*=2.4 Hz, 1H), 7.20 (dd, *J*=8.4, 2.4 Hz, 1H), 6.90 (d, *J*=8.4 Hz, 1H), 6.74 (s, 1H), 4.80 (d, *J*=6.0, 2H), 3.85 (s, 3H), 2.56 (t, *J*=7.5 Hz, 2H), 1.65-1.56 (m, 2H), 0.91 (t, *J*=7.2 Hz, 3H); ¹³C NMR (150 MHz, CDCl₃) δ 170.2, 160.3, 155.4, 135.2, 131.2, 129.3, 117.4, 111.4, 103.6, 56.7, 55.7, 37.0, 24.7, 13.8. |
| 11 | ¹H NMR (600 MHz, CDCl3) δ 8.05 (d, *J*=2.4 Hz, 1H), 7.50-7.49 (m, 1H), 7.39-7.38 (m, 5H), 6.86 (dd, *J*=9, 1.2 Hz, 1H), 6.79 (s, 1H), 4.67 (s, 2H), 4.65 (s, 2H), 3.87 (d, *J*=1.8 Hz, 3H). ¹³C NMR (150 MHz, CDCl₃) δ 168.9, 158 9, 156.3, 137.2, 133.8, 131.9, 128.6, 128.1, 128.0, 119.7, 113.3, 113.2, 104.6, 72.9. 62.8, 55.9. HRMS (EI): mass calculated for C₁₆H₁₆BrNO₃ [M⁺], 373.0314; found, 373.0316. |
| 12 | ¹H NMR (600 MHz, CDCl₃) δ 8.01- 8.00 (m, 1H), 7.49-7.47 (m, 1H), 6.86 (d, *J*=9 Hz, 1H), 6.80 (s, 1H), 4.78 (s, 2H), 4.23 (q, *J*=7.2 Hz, 2H), 4.18 (s, 2H), 3.86 (s, 3H), 1.29 (t, *J*=7.2 Hz, 3H). ¹³C NMR (150 MHz, CDCl₃) δ 169.8, 167.9, 159.0, 156.4, 133.9, 132.0, 119.6, 113.3, 113.3, 105.2, 67.8, 63.9, 61.2, 55.9, 14.3. HRMS (EI): mass calculated for C₁₅H₁₆BrNO₅ [M⁺], 369.0212; found, 369.0212. |
| 13 | ¹H NMR (600 MHz, CDCl₃) δ 8.00 (d, *J*=2.4 Hz, 1H), 7.46 (dd, *J*=9.0, 2.4 Hz, 1H), 6.85 (d, *J*=9.0 Hz, 1H), 6.73 (s, 1H), 4.56 (d, *J*=3.0, 2H), 3.85 (s, 3H), 3.44 (s, 3H); ¹³C NMR (150 MHz, CDCl₃) δ 168.7, 158.9, 156.3, 133.7, 131.9, 119.7, 113.2, 113.2, 104.4, 65.3, 58.9, 55.9. |
| 14 | ¹H NMR (600 MHz, CDCl₃) δ 9.27 (s, 1H), 7.23 (d, *J*=1.8 Hz, 1H), 7.13 (dd, *J*=8.4, 1.8 Hz, 1H), 6.97 (d, *J*=8.4 Hz, 1H), 6.63 (s, 1H), 4.81 (s, 2H), 2.53 (t, *J*=7.2, 2H), 2.50 (bs, 1H), 1.63-1.57 (m, 2H), 0.92 (t, *J*=7.2, 3H); ¹³C NMR (150 MHz, CDCl₃) δ 170.8, 162.6, 154.4, 134.1, 131.9, 127.6, 117.2, 112.3, 99.7, 56.5, 37.1, 24.7, 13.7. |
| 15 | ¹H NMR (400 MHz, CDCl₃) d 9.32 (s, 1H), 7.27 (d, 1H, *J*=1.9 Hz), 7.18 (dd, 1H, *J*=8.8 and 2.4 Hz), 7.01 (d, 1H, *J*=8.3 Hz), 6.66 (s, 1H), 6.00 (m, 1H), 5.10 (m, 2H), 4.84 (s, 2H), 3.36 (d, 2H, *J*=6.8 Hz), 2.37 (br s, 1H); ¹³C NMR (100 MHz, CDCl₃) d 170.7, 162.5, 154.8, 137.3, 132.1, 131.2, 127.7, 117.4, 115.9, 112.8, 99.6, 56.5, 39.1; LRMS (ESI) m/z 254.0 (M+Na+). |
| 16 | ¹H NMR (400 MHz, CDCl₃) d 9.32 (s, 1H), 7.27 (d, 1H, *J*=1.9 Hz), 7.18 (dd, 1H, *J*=8.8 and 2.4 Hz), 7.01 (d, 1H, *J*=8.3 Hz), 6.66 (s, 1H), 6.00 (m, 1H), 5.10 (m, 2H), 4.84 (s, 2H), 3.36 (d, 2H, *J*=6.8 Hz), 2.37 (br s, 1H); ¹³C NMR (100 MHz, CDCl₃) d 170.7, 162.5, 154.8, 137.3, 132.1, 131.2, 127.7, 117.4, 115.9, 112.8, 99.6, 56.5, 39.1; LRMS (ESI) m/z 254.0 (M+Na+). |
| 17 | ¹H NMR (600 MHz, CDCl₃) δ 7.72 (d, *J*=2.3 Hz, 1H), 7.22 (dd, *J*=8.5, 2.3 Hz, 1H), 6.93 (d, *J*=8.5 Hz, 1H), 6.77 (s, 1H), 5.99-5.91 (m, 2H), 5.36-5.24 (m, 2H), 5.10-5.05 (m, 2H), 4.64 (d, *J*=0.6 Hz, 2H), 4.12-4.10 (m, 2H), 3.87 (s, 3H), 3.37 (d, *J*=6.7 Hz, 2H). ¹³C NMR (150 MHz, CDCl₃) δ 168.5, 160.2, 155.8, 137.5,133.9, 132.6, 131.3, 129.6, 118.2, 117.8, 116.0, 111.7, 104.7, 71.9, 62.8, 55.8, 39.3. HRMS (EI): mass calculated for C₁₇H₁₉NO₃ [M⁺] , 285.1365; found, 285.1368. |
| 18 | ¹H NMR (600 MHz, CDCl₃) δ 7.74 (d, *J*=2.3 Hz, 1H), 7.40-7.36 (m, 4H), 7.34-7.30 (m, 1H), 7.24 (dd, *J*=8.5, 2.3 Hz, 1H), 5.01-5.94 (ddt, *J*=16.8, 10.0, 6.7 Hz, 1H), 5.12-5.06 (m, 2H), 4.68 (d, *J*=0.5 Hz, 2H), 4.65 (s, 2H), 3.88 (s, 3H), 3.38 (d, *J*=6.7 Hz, 2H). ¹³C NMR (150 MHz, CDCl₃) δ 168.5, 160.2, 155.8, 137.5, 137.4, 132.6, 131.4, 129.7, 128.7, 128.1, 117.8, 116.0, 111.7, 104.9, 72.9, 62.9, 55.8, 39.3. HRMS (EI): mass calculated for C₂₁H₂₁NO₃ [M⁺], 335.1521; found, 335.1522. |
| 19 | ¹H NMR (600 MHz, CDCl₃) δ 7.71 (d, *J=2.2* Hz, 1H), 7.22 (dd, J=8.5, 2.3 Hz, 1H), 6.93 (d, *J*=8.5 Hz, 1H), 6.82 (s, 1H), 5.98-5.92 (m, 1H), 5.09-5.05 (m, 2H), 4.78 (d, *J*=0.5 Hz, 2H), 4.23 (q, *J=7.1* Hz, 2H), 4.19 (s, 2H), 3.87 (s, 3H), 3.36 (d, *J*=6.7 Hz, 2H), 1.29 (t, *J*=7.1 Hz, 3H). ¹³C NMR (150 MHz, CDCl₃) δ 169.8, 167.4, 160.3, 155.8, 137.5, 132.6, 131.4, 129.6, 117.6, 116.0, 111.6, 105.4, 67.8, 63.9, 61.2, 55.8, 39.3, 14.3. HRMS (El): mass calculated for C₁₈H₂₁NO₅ [M⁺], 331.1420; found, 331.1420. |
| 20 | ¹H NMR (600 MHz, CDCl₃) δ 7.69 (d, *J*=2.4 Hz, 1H), 7.18 (dd, *J*=8.4, 2.4 Hz, 1H), 6.88 (d, *J*=8.4 Hz, 1H), 6.76 (s, 1H), 4.56 (m, 2H), 3.83 (s, 3H), 3.43 (s, 2H), 2.54 (t, *J*=9.0 Hz, 2H), 1.64-1.58 (m, 2H), 0.90 (t, *J*=7.2 Hz, 3H). ¹³C NMR (150 MHz, CDCl₃) δ 188.2, 160.2, 155.4, 135.1, 131.1, 129.3, 117.4, 111.4, 104.7, 65.3, 58.8, 55.6, 37.0, 24.7, 13.8. |
| 21 | ¹H NMR (600 MHz, CDCl₃) δ 7.98 (s, 1H), 7.43-7.45 (m, 1H), 6.83 (d, *J*=8.4 Hz, 1H), 6.65 (s, 1H), 3.84 (s, 3H), 3.71 (s, 2H), 3.43 (s, 4H), 2.47 (s, 4H), 1.41 (s, 9H). ¹³C NMR (150 MHz, CDCl₃) δ 168.5, 158.8, 156.3, 154.7, 133.7, 131.9, 119.7, 113.2, 113.2, 105.0, 79.8, 55.9, 53.3, 52.6, 29.7, 28.4. HRMS (El): mass calculated for C₂₀H₂₆BrN₃O₄ [M⁺], 451.1107; found, 451.1106. |
| 22 | ¹H NMR (600 MHz, CDCl₃) δ 7.71 (d, *J=*2.2 Hz, 1H), 7.21 (dd, *J*=8.5, 2.3 Hz, 1H), 6.92 (d, *J*=8.5 Hz, 1H), 6.68 (s, 1H), 5.99-5.92 (m, 1H), 5.89-5.82 (m, 1H), 5.20-5.14 (m, 2H), 5.10-5.05 (m, 2H), 3.87 (s, 3H), 3.74 (s, 2H), 3.36 (d, *J*=6.7 Hz, 2H), 3.01 (d, *J*=6.6 Hz, 2H), 2.62-2.52 (m, 8H). ¹³C NMR (150 MHz, CDCl₃) δ 168.4, 160.1, 155.8, 137.5, 134.9, 132.6, 131.2, 129.7, 118.4, 118.0, 116.0, 111.7, 105.1, 61.8, 55.8, 53.5, 53.0, 52.9, 39.3. HRMS (EI): mass calculated for C₂₁H₂₇N₃O₂ [M⁺], 353.2103; found, 353.2100. |
| 23 | ¹H NMR (600 MHz, CDCl3) δ 7.72 (d, *J*=2.3 Hz, 1H), 7.31-7.30 (m, 4H), 7.25-7.21 (m, 2H), 6.92 (d, *J*=8.5 Hz, 1H), 6.68 (s, 1H), 5.99-5.92 (m, 1H), 5.10-5.05 (m, 2H), 3.87 (s, 3H), 3.74 (s, 2H), 3.52 (s, 2H), 3.37 (d, *J*=6.7 Hz, 2H), 2.60-2.52 (m, 8H). ¹³C NMR (150 MHz, CDCl₃) δ 168.5, 160.1, 155.8, 137.5, 132.6, 131.2, 129.7, 129.3, 128.7, 128.4, 127.2, 127.1, 118.0, 116.0, 111.7, 105.1, 63.1, 55.8, 53.5, 53.0, 39.3. HRMS (EI): mass calculated for C₂₅H₂₉N₃O₂ [M⁺], 403.2260; found, 403.2256. |
| 24 | ¹H NMR (600 MHz, CDCl3) δ 7.70 (d, *J*=2.3 Hz, 1H), 7.21 (dd, *J*=8.5, 2.3 Hz, 1H), 6.92 (d, *J*=8.5 Hz, 1H), 6.67 (s, 1H), 5.98-5.92 (m, 1H), 5.09-5.04 (m, 2H), 4.17 (q, *J*=7.1 Hz, 2H), 3.86 (s, 3H), 3.74 (s, 2H), 3.36 (d, J=6.7 Hz, 2H), 3.20 (s, 2H), 2.64 (s, 7H), 1.26 (t, *J*=7.1 Hz, 3H). ¹³C NMR (150 MHz, CDCl₃) δ 170.3, 168.3, 160.1, 155.8, 137.5, 132.6, 131.2, 129.7, 117.9, 116.0, 111.6, 105.1, 60.8, 59.5, 55.8, 53.5, 53.0, 52.7, 39.3, 14.4. HRMS (EI): mass calculated for C₂₂H₂₉N₃O₄ [M⁺], 399.2158; found, 399.2160. |
| 25 | ¹H NMR (600 MHz, CDCl₃) δ 7.71 (d, *J*=1.8 Hz, 1H), 7.20 (dd, *J*=8.4, 2.4 Hz, 1H), 8.91 (d, *J*=8.4 Hz, 1H), 6.67 (s, 1H), 5.92-5.96 (m, 1H), 5.03-5.08 (m, 2H), 3.85 (s, 3H), 3.73 (s, 2H), 3.45 (t, *J*=4.2 Hz, 4H), 3.35 (d, *J*=6.6 Hz, 2H), 2.49 (s, 4H), 1.43 (s, 9H). ¹³C NMR (150 MHz, CDCl₃) δ 168.1, 160.1, 155.7, 154.7, 137.4, 132.6, 131.3, 129.6, 117.8, 115.9, 111.6, 105.1, 79.8, 55.7, 53.5, 52.7, 39.3, 28.5. HRMS (EI): mass calculated for C₂₃H₃₁N₃O₄ [M⁺], 413.2315; found, 413.2318. |
| 26 | ¹H NMR (600 MHz, CDCl₃) δ 7.68 (d, *J*=2.4 Hz, 1H), 7.18 (dd, *J*=8.4, 2.4 Hz, 1H), 6.88 (d, *J*=8.4 Hz, 1H), 6.67 (s, 1H), 3.85 (s, 3H), 3.73 (s, 2H), 3.44 (t, *J*=4.2 Hz, 4H), 2.54 (t, *J*=7.2 Hz, 2H), 2.48 (bs, 4H), 1.64-1.59 (m, 2H), 1.42 (s, 9H), 0.90 (t, *J*=7.2 Hz, 3H); ¹³C NMR (150 MHz, CDCl₃) δ 168.0, 160.2, 155.3, 154.7, 135.2, 131.1, 129.3, 117.5, 111.4, 105.1, 79.7, 55.7, 53.4, 52.6, 37.0, 28.4, 24.7, 13.8. |
| 27 | ¹H NMR (600 MHz, CDCl₃) δ 9.33 (s, 1H), 7.29 (d, *J=*2.2 Hz, 1H), 7.17 (m, 1H), 7.02 (d, *J*=8.4 Hz, 1H), 6.69 (s, 1H), 5.95 (m, 2H), 5.36 (dq, *J*=17.2, 1.6 Hz, 1H), 5.29 (dq, J=10.4, 1.3 Hz, 1H), 5.09 (m, 2H), 4.68 (d, *J*=0.8 Hz, 2H), 4.13 (dt, *J*=5.7, 1.4 Hz, 2H), 3.37 (d, *J*=6.6 Hz, 2H). ¹³C NMR (150 MHz, CDCl₃)δ 169.2, 162.6, 155.1, 137.6, 133.7, 132.2, 131.3, 127.9, 118.6, 117.7, 116.1, 113.0, 100.7, 72.2, 62.8, 39.4.; HRMS (EI+) calcd for C₁₆H₁₇NO₃ (M⁺) 271.1208, found 271.1206. |
| 28 | ¹H NMR (600 MHz, CDCl₃) δ 8.01 (d, *J*=2.6 Hz, 1H), 7.48 (dd, *J=*8.8, 2.6 Hz, 1H), 6.86 (d, *J*=8.8 Hz, 1H), 6.66 (d, *J*=0.7 Hz, 1H), 3.87 (s, 3H), 3.72 (d, *J*=0.7 Hz, 2H), 2.91 (t, *J*=4.9 Hz, 4H), 2.52 (s, 4H); ¹³C NMR (150 MHz, CDCl₃) δ 169.0, 158.9, 156.4, 133.7, 132.1, 120.0, 113.3, 113.3, 104.9, 56.0, 54.4, 54.1, 46,1.; HRMS (El+) calcd for C₁₅H₁₈BrN₃O₂ (M⁺) 351.0582, found 351.0577. |
| 29 | ¹H NMR (600 MHz, CDCl3) δ 7.71 (d, *J*=2.3 Hz, 1H), 7.22 (dd, *J*=8.4, 2.3 Hz, 1H), 6.92 (d, *J*=8.5 Hz, 1H), 6.68 (s, 1H), 5.95 (ddt, *J*=16.9, 10.0, 6.7 Hz, 1H), 5.07 (m, 2H), 3.87 (s, 3H), 3.73 (s, 2H), 3.36 (d, *J*=6.7 Hz, 2H), 2.95 (t, *J*=4.9 Hz, 4H), 2.56 (s, 4H); ¹³C NMR (150 MHz, CDCl₃) δ 168.4, 160.1, 155.8, 137.5, 132.6, 131.3, 129.7, 117.9, 116.0, 111.7, 105.1, 55.8, 54.0, 53.9, 45.9, 39.3.; HRMS (EI+) calcd for C₁₃H₂₃N₃O₂ (M⁺) 313.1790, found 313.1789. |
| 30 | ¹H NMR (600 MHz, Chloroform-*d*) δ 7.64 (d, *J*=2.3 Hz, 1H), 7.13 (dd, *J*=8.4, 2.3 Hz, 1H), 6.84 (d, *J*=8.4 Hz, 1H), 6.62 (d, *J*=0.7 Hz, 1H), 3.80 (s, 3H), 3.65 (d, *J*=0.7 Hz, 2H), 2.85 (t, *J*=4.9 Hz, 4H), 2.49 (m, 2H), 2.46 (s, 4H), 1.57 (m, 2H), 0.86 (t, *J*=7.3 Hz, 3H); ¹³C NMR (150 MHz, CDCl₃) δ 168.4, 160.2, 155.4, 135.2, 131.1, 129.4, 117.7, 111.5, 105.1, 55.8, 54.3, 54.1, 46.1, 37.1, 24.8, 13.9.; HRMS (El+) calcd for C₁₈H₂₅N₃O₂ (M⁺) 315.1947, found 315.1944. |
| 31 | ¹H NMR (400 MHz, CDCl₃) δ 2.04 (br t, *J*=6.32 Hz, 1 H) 3.97 (s, 3 H) 4.86 (d, *J*=6.13 Hz, 2 H) 6.78 (s, 1 H) 7.09 (d, *J*=8.63 Hz, 1 H) 7.69 (dd, *J*=8.63, 1.88 Hz, 1 H) 8.18 (d, *J*=1.88 Hz, 1 H). LC-MS, m/z [M+1]=274.3, |
| 32 | ¹H NMR (400 MHz, CDCl₃) δ 1.70 (s, 6 H) 2.18 (br s, 1 H) 3.97 (s, 3 H) 6.66 (s, 1 H) 7.08 (d, *J*=8.80 Hz, 1 H) 7.68 (dd, *J*=8.68, 1.83 Hz, 1 H) 8.16 (d, *J*=1.96 Hz, 1 H). LC-MS, m/z [M+1]=302.2 |
| 33 | ¹H NMR (400 MHz, CDCl₃) δ 3.11 (t, *J*=6.25 Hz, 2 H) 3.97 (s, 3 H) 4.03 (t, *J*=6.25 Hz, 2 H) 6.63 (s, 1 H) 7.08 (d, *J*=8.63 Hz, 1 H) 7.67 (dd, *J*=8.76, 2.00 Hz, 1 H) 8.16 (d, *J*=2.13 Hz, 1 H). LC-MS, m/z [M+1]=288.1 |
| 34 | ¹H NMR (400 MHz, MeOD) δ 3.33-3.41 (m, 4 H) 3.45-3.55 (m, 4 H) 4.02 (s, 3 H) 4.49 (s, 2 H) 7.20 (s, 1 H) 7.36 (d, *J*=8.68 Hz, 1 H) 7.80 (dd, *J*=8.74, 1.65 Hz, 1 H) 8.10 (d, *J*=1.83 Hz, 1 H). LC-MS, m/z [M+1]=342.0 |
| 35 | ¹H NMR (400 MHz, DMSO-d6, ppm) δ 7.95 (d, *J*=7.9 Hz, 1 H), 7.48 (s, 1 H), 7.43 (d, *J*=8.0 Hz, 1 H), 6.81 (s, 1 H), 5.71 (t, *J*=6.0 Hz, 1 H), 4.63 (dd, *J*=6.0, 0.5 Hz, 2 H), 3.97 (s, 3 H). LC-MS, m/z [M+1]=274 |
| 36 | ¹H NMR (400 MHz, DMSO-*d₆*, ppm) δ 12.82 (s, 1 H), 8.02 (d, *J*=2.2 Hz, 1 H), 7.88 (dd, *J*=8.8, 1.9 Hz, 1 H), 7.42 (d, *J*=8.8 Hz, 1 H), 8.98 (s, 1 H), 4.76 (s, 2 H), 4.15 (s, 2 H), 3.97 (s, 3 H). LC-MS, m/z [M+1]=332 |
| 37 | ¹H NMR (400 MHz, DMSO-*d*₆, ppm) δ 8.03 (d, *J*=2.2 Hz, 1 H), 7.87 (dd, *J*=8.8, 1.9 Hz, 1 H), 7.41 (d, *J*=8.8 Hz, 1 H), 6.90 (s, 1 H), 3.97 (s, 3 H), 3.85 (s, 2 H), 3.32-3.24 (m, 2 H), 3.08 (s, 2 H), 2.82 (s, 3 H), 2.79-2.71 (m, 2 H). LC-MS, m/z [M+1]=370 |
| 38 | ¹H NMR (400 MHz, DMSO-*d₆*, ppm) δ 7.28 (d, *J*=1.4 Hz, 1 H), 7.10 (d, *J*=1.5 Hz, 2H), 6.73 (s, 1 H), 5.67 (t, *J*=6.0 Hz, 1 H), 4.60 (d, *J*=5.5 Hz, 2 H), 3.80 (s, 3 H), 3.74 (t, *J*=4.6 Hz, 4 H), 3.03 (t, *J*=4.7 Hz, 4 H). LC-MS, m/z [M+1]=291 |
| 39 | ¹H NMR (400 MHz, DMSO-*d*₆): δ 7.54 (d, *J*=2.0 Hz, 1H), 7.29-7.27 (m, 1H), 7.11 (d, *J*=8.8 Hz, 1H), 6.63 (s, 1H), 5.96-5.94 (m, 1H), 5.63 (s, 1H), 5.10-5.04 (m, 2H), 3.84 (s, 3H), 3.35 (d, *J*=6.4 Hz, 2H), 1.50 (s, 6H). LC-MS, m/z [M+1]=274 |
| 40 | ¹H NMR (400 MHz, DMSO-*d*₆): δ 7.53 (d, *J*=2.0 Hz, 1H), 7.28 (dd, *J*=8.4, 2.0 Hz, 1H), 7.09 (d, *J*=8.4 Hz, 1H), 6.63 (s, 1H), 5.63 (s, 1H), 3.84 (s, 3H), 2.56-2.52 (m, 2H), 1.60-1.55 (m, 2H), 1.50 (s, 6H), 0.89 (t, *J*=7.2 Hz, 3H). LC-MS, m/z [M+1]=276.2 |
| 41 | ¹H NMR (400 MHz, DMSO-*d*₆): δ 7.59 (d, *J=2.8* Hz, 1H), 7.31 (dd, *J*=11.6, 3.2 Hz, 1H), 7.15 (d, *J*=11.2 Hz, 1H), 6.76 (s, 1H), 6.03-5.94 (m, 1H), 5.14-5.06 (m, 2H), 3.87 (s, 3H), 3.73 (s, 2H), 3.39 (s, 2H), 2.48-2.35 (m, 8H), 2.17 (s, 3H). LC-MS, m/z [M+1]=328.1. |
| 42 | ¹H NMR (400 MHz, DMSO-*d₆*): δ 7.56 (d, *J*=2.4 Hz, 1H), 7.28 (dd, *J*=8.4, 2.4 Hz, 1H), 7.09 (d, *J*=8.4 Hz, 1H), 6.73 (s, 1H), 3.84 (s, 3H), 3.70 (s, 2H), 2.56-2.54 (m, 2H), 2.50-2.32 (m, 8H), 2.14 (s, 3H), 1.60-1.55 (m, 2H), 0.89 (t, *J*=7.2 Hz, 3H). LC-MS, m/z [M+1]=330.1 |
| 43 | ¹H NMR (500 MHz, CDCl₃) δ 7.81-7.77 (m, 1H), 7.30 (d, J=1.9 Hz, 2H), 6.65 (s, 1H), 6.00-5.91 (m, 1H), 5.15-5.09 (m, 2H), 4.85 (dd, J=6.5, 0.6 Hz, 2H), 3.44 (d, J=6.7 Hz, 2H). LC-MS, m/z [M+1]=300.1 |
| 44 | ¹H NMR (500 MHz, CDCl₃) δ 7.80 (d, J=7.8 Hz, 1H), 6.91-8.86 (m, 1H), 6.82 (d, J=1.1 Hz, 1H), 6.74 (s, 1H), 5.98 (ddt, J=16.9, 10.1, 6.7 Hz, 1H), 5.17-5.10 (m, 2H), 4.82 (dd, J=6.5, 0.5 Hz, 2H), 3.89 (d, J=3.8 Hz, 3H), 3.43 (d, J=6.7 Hz, 2H). LC-MS, m/z [M+1]=246.1. |
| 45 | ¹H NMR (500 MHz, CDCl₃) δ 7.74 (d, J=2.4 Hz, 1H), 7.28 (dd, J=8.6, 2.4 Hz, 1H), 6.94 (d, J=8.5 Hz, 1H), 6.77 (s, 1H), 4.82 (s, 2H), 3.88 (s, 3H), 2.92 (dt, J=13.8, 6.9 Hz, 1H), 1.27-1.24 (m, 6H). LC-MS, m/z [M+1]=248.1. |
| 46 | ¹H NMR (500 MHz, CDCl₃) δ 7.76 (d, J=2.3 Hz, 1H), 7.27 (d, J=2.3 Hz, 1H), 6.93 (d, J=8.5 Hz, 1H), 6.69 (s, 1H), 3.87 (s, 3H), 3.76 (s, 2H), 3.47 (s, 4H), 2.91 (dt, J=14.0, 7.0 Hz, 1H), 2.52-2.50 (m, 4H), 1.44 (d, J=10.3 Hz, 9H), 1.27-1.23 (m, 6H). |
| 47 | ¹H NMR (500 MHz, DMSO) δ 7.62 (d, J=2.0 Hz, 1H), 7.38 (dd, J=8.6, 2.0 Hz, 1H), 7.18 (s, 1H), 7.14 (d, J=8.6 Hz, 1H), 4.52 (s, 2H), 3.86 (s, 3H), 3.44 (s, 4H), 3.38 (s, 4H), 2.91 (dt, J=13.8, 6.9 Hz, 1H), 1.21 (d, J=6.9 Hz, 6H).). LC-MS, m/z [M+1]=316.2. |
| 48 | ¹H NMR (500 MHz, CDCl₃) δ 7.81 (d, J=7.8 Hz, 1H), 6.87 (d, J=7.8 Hz, 1H), 6.82 (s, 1H), 6.67 (s, 1H), 5.98 (ddd, J=16.9, 6.7, 3.5 Hz, 1H), 5.18-5.10 (m, 2H), 3.89 (s, 3H), 3.75 (s, 2H), 3.47 (s, 4H), 3.32 (d, J=2.4 Hz, 2H), 2.51 (s, 4H), 1.45 (s, 9H). LC-MS, m/z [M+1]=414.3. |
| 49 | LC-MS, m/z [M+1]=314.2. |
| 50 | ¹H NMR (500 MHz, CDCl₃) δ 7.79 (s, 1H), 7.30 (s, 2H), 6.58 (s, 1H), 6.00-5.92 (m, 1H), 5.12 (ddd, J=4.2, 3.2, 1.6 Hz, 2H), 3.77 (s, 2H), 3.50-3.45 (m, 4H), 3.44 (d, J=6.7 Hz, 2H), 2.53-2.48 (m, 4H), 1.45 (s, 9H). LC-MS, m/z [M+1]=468.2 |
| 51 | ¹H NMR (500 MHz, DMSO) δ 7.72 (d, J=1.5 Hz, 1H), 7.55-7.47 (m, 2H), 6.99 (s, 1H), 6.04-5.95 (m, 1H), 5.13 (t, J=13.6 Hz, 2H), 4.25 (s, 2H), 3.48 (d, J=6.8 Hz, 2H), 3.24 (s, 4H), 3.01 (s, 4H). LC-MS, m/z [M+1]=368.2. |
| 52 | ¹H NMR (500 MHz, MeOD) δ 7.72 (dd, *J*=2.2, 0.5 Hz, 1H), 7.45 (dd, *J*=8.5, 2.2 Hz, 1H), 7.42-7.39 (m, 1H), 6.88 (s, 1H), 6.00 (d, *J*=6.7 Hz, 1H), 5.12 (dd, *J*=13.6, 4.0 Hz, 2H), 4.23 (s, 2H), 3.66 (s, 2H), 3.31 (d, *J*=1.6 Hz, 4H), 3.30 (d, *J*=1.6 Hz, 4H), 2.94 (s, 3H). LC-MS, m/z [M+1]=382.2 |
| 53 | ¹H NMR (400 MHz, DMSO-*d*₆): δ 12.07 (s, 1H), 7.60 (d, *J*=2.0 Hz, 1H), 7.33 (dd, *J*=8.8 Hz, 2.0 Hz, 1H), 7.27 (s, 1H), 7.13 (d, *J*=8.8 Hz, 1H), 4.61 (s, 2H), 3.95-3.83 (m, 5H), 3.35 (d, *J*=11.2 Hz, 2H), 2.69-2.54 (m, 4H), 1.61-1.56 (m, 2H), 1.13 (d, *J*=6.4 Hz, 6H), 0.89 (t, *J*=7.2 Hz, 3H). LC-MS, m/z [M+1]=345.1 |
| 54 | ¹H NMR (400 MHz, DMSO-*d*₆): δ 11.67 (s, 1H), 7.59 (d, *J*=2.4 Hz, 1H), 7.09 (dd, *J*=8.4 Hz, 2.0 Hz, 1H), 7.29 (s, 1H), 7.13 (d, *J*=8.4 Hz, 1H), 4.61 (s, 2H), 3.86 (s, 3H), 3.55 (s, 2H), 3.44-3.24 (m, 4H), 3.07-3.05 (m, 2H), 2.56 (t, *J*=7.6 Hz, 2H), 2.15-2.12 (m, 1H), 2.07-2.00 (m, 2H), 1.76-1.73 (m, 1H), 1.63-1.54 (m , 2H), 0.89 (t, *J*=7.2 Hz, 3H). LC-MS, m/z [M+1]=345.1 |
| 55 | ¹H NMR (400 MHz, DMSO-*d*₆): δ 11.29 (s, 1H), 7.60 (d, *J*=1.6Hz, 1H), 7.09 (dd, *J*=8.4 Hz, 2.4Hz, 1H), 7.25 (s, 1H), 7.13 (d, *J*=8.8 Hz, 1H), 4.67 (s, 2H), 3.86 (s, 3H), 3.47-3.33 (m, 2H), 3.03-3.01 (m, 2H), 2.56 (t, *J*=7.6 Hz, 2H), 2.08-2.05 (m, 2H), 1.93-1.83 (m, 2H), 1.63-1.54 (m , 2H), 0.89 (t, *J*=7.2 Hz, 3H). LC-MS, m/z [M+1]=359.1 |
| 56 | ¹H NMR (400 MHz, DMSO-*d*₆): δ 11.66 (s, 1H), 7.61 (d, *J*=2.4 Hz, 1H), 7.47 (d, *J*=5.2 Hz, 1H), 7.33 (dd, *J*=8.8 Hz, 2.4 Hz, 1H), 7.27 (s, 1H), 7.14 (d, *J*=8.0 Hz, 1H), 6.94 (d, *J*=5.2 Hz, 1H), 4.78 (s, 2H), 4.27 (s, 2H), 3.86 (s, 3H), 3.49-3.42 (m, 2H), 3.16 (s, 2H), 2.56 (t, *J*=7.6 Hz, 2H), 1.61-1.54 (m , 2H), 0.89 (t, *J*=7.6 Hz, 3H). LC-MS, m/z [M+1]=369.1 |
| 57 | ¹H NMR (400 MHz, DMSO-*d*₆): δ 7.65 (dd, *J*=8.8 Hz, 2.4 Hz, 2H), 7.57 (d, *J*=2.4 Hz, 1H), 7.31 (dd, *J*=8.8 Hz, 2.0 Hz, 1H), 7.11 (d, *J*=8.4 Hz, 1H), 6.90 (s, 1H), 4.14 (s, 4H), 4.08 (s, 2H), 3.85 (s, 3H), 3.13 (t, *J*=5.2 Hz, 2H), 2.55 (t, *J*=7.2 Hz, 2H), 1.61-1.55 (m, 2H), 0.89 (t, *J*=7.2 Hz, 2H). LC-MS, m/z [M+1]=353.1 |
| 58 | ¹H NMR (400 MHz, DMSO-*d*₆): δ 9.09 (s, 2H), 7.65 (d, *J*=8.8 Hz, 1H), 7.08 (d, *J*=12.4 Hz, 1H), 6.91 (s, 1H), 3.87 (s, 3H), 3.17 (s, 4H), 2.86 (s, 2H), 2.57 (t, *J*=7.2Hz, 2H), 1.59-1.54 (m , 2H), 0.90 (t, *J=7.2* Hz, 3H). LC-MS, m/z [M+1]=334.1 |
| 59 | ¹H NMR (400 MHz, DMSO-*d*₆): δ 10.61 (s, 1H), 7.65 (d, *J*=8.8 Hz, 1H), 7.08 (d, *J*=12.4 Hz, 1H), 6.91 (s, 1H), 3.91 (s, 2H), 3.87 (s, 3H), 3.39 (d, *J*=12.0 Hz, 2H), 3.07-3.04 (m, 4H), 2.74 (s , 3H), 2.67-2.61 (m, 2H), 2.57 (t, *J*=7.2Hz, 2H), 1.59-1.54 (m , 2H), 0.90 (t, *J*=7.2 Hz, 3H). LC-MS, m/z [M+1]=348.1 |
| 60 | ¹H NMR (400 MHz, DMSO-*d*₆): δ 11.06 (s, 1H), 7.71 (s, 1H), 7.48 (s, 1H), 6.94 (s, 1H), 4.13 (s, 2H), 3.45 (d, *J*=12.0 Hz, 3H), 3.15 (s, 4H), 2.82-2.75 (m, 5H), 2.66 (t, *J*=7.6 Hz, 2H), 1.66-1.58 (m , 2H), 0.91 (t, *J*=7.2 Hz, 3H). LC-MS, m/z [M+1]=384.1 |
| 61 | ¹H NMR (400 MHz, DMSO-*d*₆) δ 10.90 (s, 1H), 7.60 (d, *J*=2.0 Hz, 1H), 7.33 (dd, *J*=8.4, 2.0 Hz, 1H), 7.11 (d, *J*=8.8 Hz, 1H), 6.96 (s, 1H), 4.14 (s, 2H), 3.84 (s, 3H), 3.58 (t, *J*=6.8 Hz, 2H), 3.45 (d, *J*=11.2 Hz, 1H), 3.19 (s, 4H), 2.84-2.69 (m, 7H). LC-MS, m/z [M+1]=332 |
| 62 | ¹H NMR (400 MHz, DMSO-*d*₆): δ 10.91 (s, 1H), 7.61 (, *J*=2.4 Hz, 1H), 7.34 (dd, *J*=8.4, 2.8 Hz, 1H), 7.11 (d, *J*=8.8 Hz, 1H), 6.96 (s, 1H), 4.13 (s, 2H), 3.84 (s, 3H), 3.53-3.40 (m, 4H), 3.23-3.05 (m, 7H), 2.81-2.66 (m, 7H). LC-MS, m/z [M+1]=346 |
| 63 | ¹H NMR (500 MHz, MeOD) δ 7.63 (d, J=2.3 Hz, 1H), 7.33 (dd, J=8.6, 2.3 Hz, 1H), 7.06 (d, J=8.6 Hz, 1H), 6.81 (s, 1H), 3.88 (s, 3H), 3.81 (s, 2H), 3.63-3.56 (m, 4H), 2.93-2.86 (m, 1H), 2.60-2.53 (m, 4H), 2.08 (s, 3H), 1.26 (s, 3H), 1.25 (s, 3H). LC-MS, m/z [M+1]=358.2 |
| 64 | ¹H NMR (500 MHz, MeOD) δ 7.64 (d, J=2.3 Hz, 1H), 7.32 (dd, J=8.6, 2.2 Hz, 1H), 7.04 (d, J=8.6 Hz, 1H), 6.80 (s, 1H), 3.89 (s, 3H), 3.83 (s, 2H), 3.28-3.25 (m, 4H), 2.85 (s, 4H), 2.68 (d, J=4.5 Hz, 4H), 1.27 (s, 3H), 1.25 (s, 3H). LC-MS, m/z [M+1]=394.2 |
| 65 | ¹H NMR (500 MHz, CDCl₃) δ 8.85 (s, 1H), 8.22 (ddd, J=8.2, 1.5, 0.6 Hz, 1H), 7.99-7.96 (m, 1H), 7.87 (ddd, J=8.5, 7.0, 1.5 Hz, 1H), 7.73 (d, J=2.3 Hz, 1H), 7.64-7.56 (m, 1H), 7.24 (dd, J=8.5, 2.3 Hz, 1H), 6.94 (d, J=8.9 Hz, 2H), 5.96 (dd, J=16.9, 10.1 Hz, 1H), 5.77 (d, J=0.5 Hz, 2H), 5.11-5.07 (m, 1H), 5.07-5.05 (m, 1H), 3.88 (d, J=1.5 Hz, 3H), 3.38 (d, J=6.7 Hz, 2H). LC-MS, m/z [M+1]=374.2 |
| 66 | ¹H NMR (500 MHz, CDCl3) δ 7.72 (d, J = 2.2 Hz, 1H), 7.22 (dd, J = 8.5, 2.3 Hz, 1H), 6.93 (d, J = 8.5 Hz, 1H), 6.70 (s, 1H), 4.80 (s, 1H), 4.74(s, 1H), 3.88 (s, 3H), 3.75 (s, 2H), 3.46 (s, 4H), 3.30 (s, 2H), 2.51 (s, 4H), 1.67 (s, 3H), 1.45 (s, 9H), 0.92 (t, J = 7.3 Hz, 1H). LC-MS, m/z [M+1] = 428.3 |
| 67 | ¹H NMR (500 MHz, MeOD) δ 7.75 (d, J=2.2 Hz, 1H), 7.42 (dd, J=8.5, 2.3 Hz, 1H), 7.14 (d, J=3.3 Hz, 1H), 7.11 (d, J=8.6 Hz, 1H), 4.46 (s, 2H), 3.92 (d, J=2.5 Hz, 3H), 3.66 (s, 2H), 3.49-3.46 (m, 4H), 3.34 (s, 4H), 3.06 (s, 2H), 1.56 (s, 3H). ). LC-MS, m/z [M+1]=328.2 |
| 68 | ¹H NMR (500 MHz, CDCl₃) δ 7.70 (d, J=2.0 Hz, 1H), 7.24 (dd, J=8.5, 2.0 Hz, 1H), 6.94 (d, J=8.5 Hz, 1H), 6.77 (s, 1H), 4.83 (d, J=6.5 Hz, 2H), 4.81 (s, 1H), 4.75 (s, 1H), 3.88 (s, 3H), 3.30 (s, 2H), 1.68 (s, 3H). LC-MS, m/z [M+1]=260.1 |
| 69 | ¹H NMR (500 MHz, DMSO) δ 7.87 (d, J=2.6 Hz, 1H), 7.68 (dd, J=8.9, 2.5 Hz, 1H), 7.20 (d, J=9.0 Hz, 1H), 6.99 (d, J=12.6 Hz, 1H), 3.94 (d, J=9.5 Hz, 1H), 3.88 (d, J=3.3 Hz, 3H), 3.85 (d, J=3.3 Hz, 2H), 3.46 (s, 2H), 3.17 (s, 8H), 2.83-2.70 (m, 9H). LC-MS, m/z [M+1]=356.2 |

The chemical structures of the compounds prepared in Example 1-69 above are summarized in the table below.

**[Table 2]**

| Example No. | Chemical Structure | Example No. | Chemical Structure |
|---|---|---|---|
| 1 | 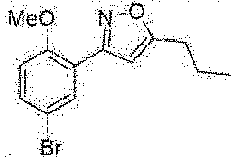 | 2 | 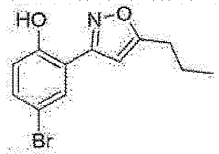 |
| 3 | 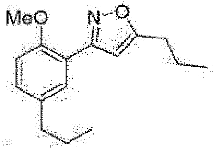 | 4 | 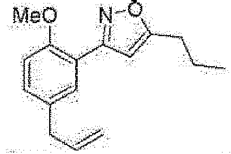 |
| 5 | 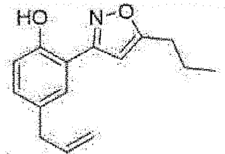 | 6 | 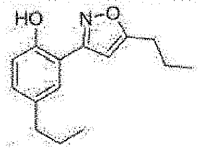 |
| 7 | 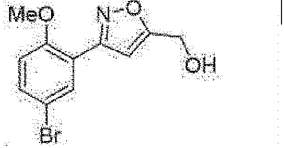 | 8 | 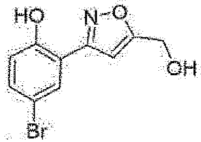 |
| 9 | 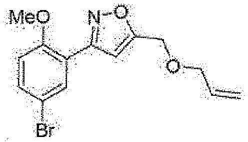 | 10 | 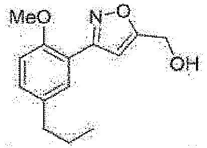 |
| 11 | 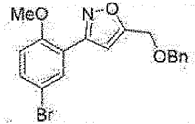 | 12 | 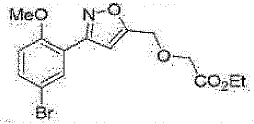 |
| 13 | 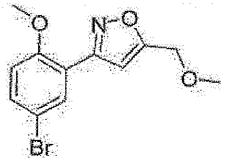 | 14 | 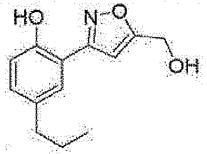 |
| 15 | 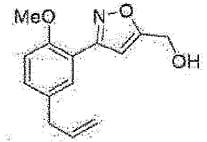 | 16 | 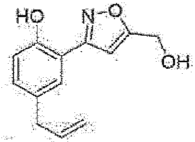 |
| 17 | 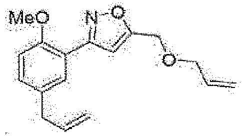 | 18 | 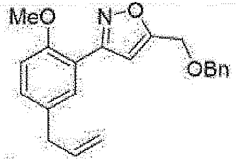 |
| 19 | 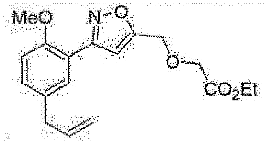 | 20 | 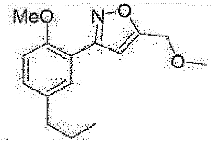 |
| 21 | 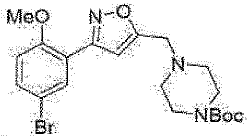 | 22 | 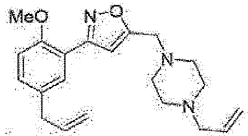 |
| 23 | 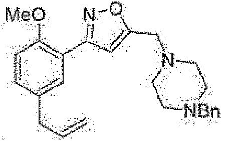 | 24 | 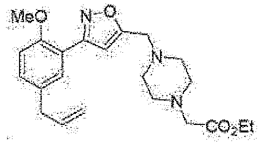 |
| 25 | 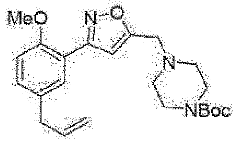 | 26 | 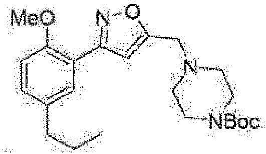 |
| 27 | 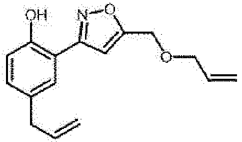 | 28 | 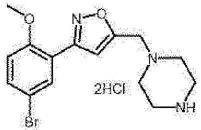 |
| 29 | 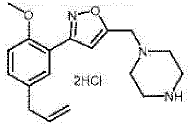 | 30 | 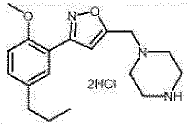 |
| 31 | 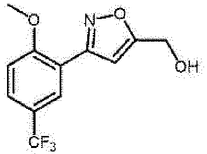 | 32 | 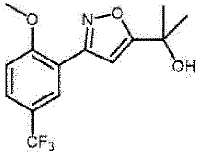 |
| 33 | 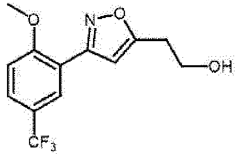 | 34 | 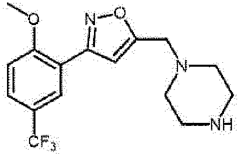 |
| 35 | 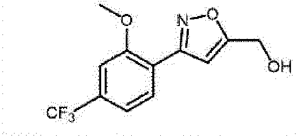 | 36 | 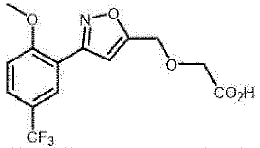 |
| 37 | 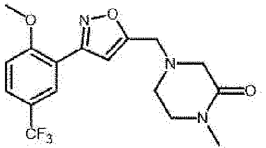 | 38 | 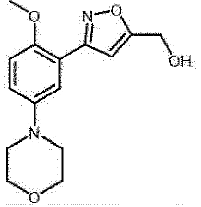 |
| 39 | 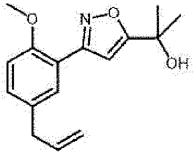 | 40 | 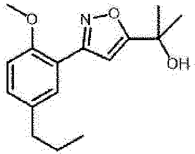 |
| 41 | 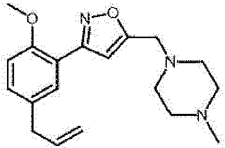 | 42 | 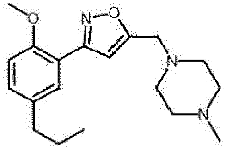 |
| 43 | 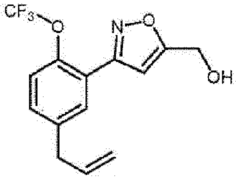 | 44 | 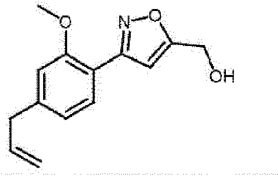 |
| 45 | 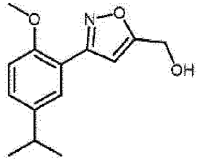 | 46 | 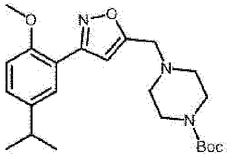 |
| 47 | 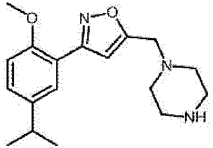 | 48 | 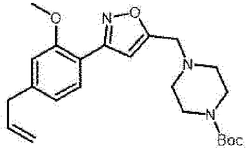 |
| 49 | 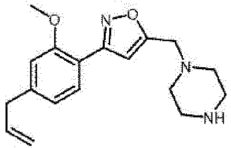 | 50 | 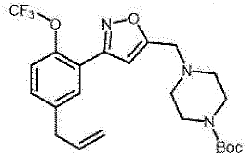 |
| 51 | 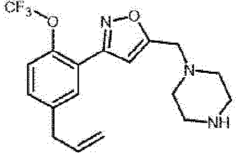 | 52 | 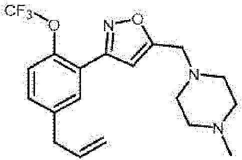 |
| 53 | 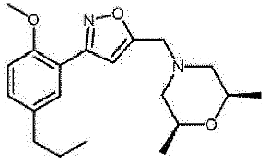 | 54 | 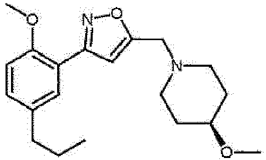 |
| 55 | 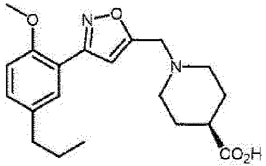 | 56 | 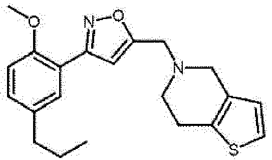 |
| 57 | 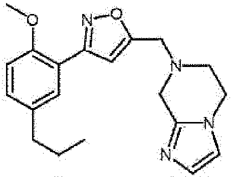 | 58 | 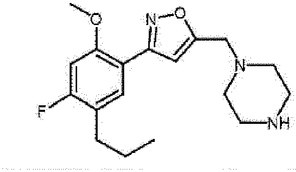 |
| 59 | 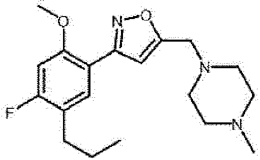 | 60 | 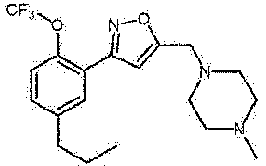 |
| 61 | 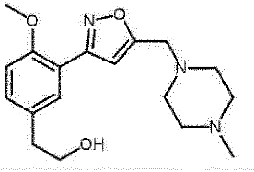 | 62 | 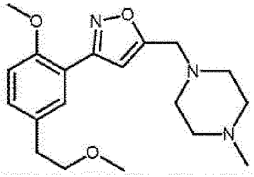 |
| 63 | 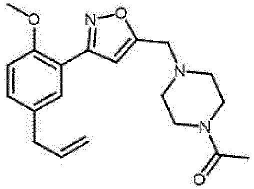 | 64 | 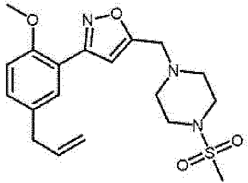 |
| 65 | 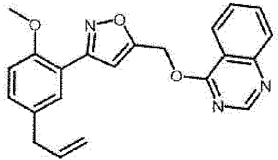 | 66 | 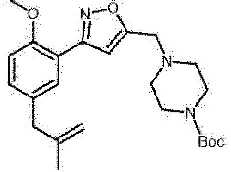 |
| 67 | 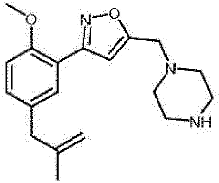 | 68 | 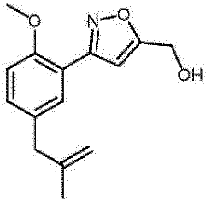 |
| 69 | 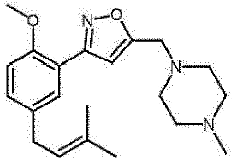 | | |

### <Experimental Example 1> Screening of compounds having the ability to remove A2E-fluorescent labeling substance (A2E-BDP) present in human retinal pigment epithelial cells

### <1-1> Experimental method

The human retinal pigment epithelial cell line (ARPE-19) used in the experiment was provided by ATCC (American Type Culture Collection). The ARPE-19 cell line was cultured in DMEM medium containing 10% FBS. After culture, the cells were dispensed into 96-well plates at a concentration of 4×10³ cells/well and cultured for 24 hours. The ARPE-19 cell line was treated with A2E-fluorescent labeling substance (A2E-BDP) at a concentration of 10 µM for 24 hours to induce the accumulation of A2E-BDP in the ARPE-19 cell line.

In order to evaluate the intracellular A2E removal ability of the compound of the present invention, an easily detectable A2E-fluorescent labeling substance (A2E-BDP) was used. The ARPE-19 cells prepared by the above method were treated with each test compound at a concentration of 50 µM once, and then cultured for 24 hours. The cells were lysed by adding a cell lysis buffer containing a protease inhibitor (50 mM Tris-chloride, pH 8.0, 150 mM sodium chloride, 1% NP-40, 1% sodium deoxycholate, 1% sodium dodecyl sulfate, and 2 mM ethylenediamine tetraacetic acid) to each 96-well. The prepared cell lysate was transferred to a white 96-well plate, and the fluorescence intensity was measured using a fluorescence microplate reader (VICTOR^{™} X3, Perkin Elmer, USA) (excitation 485 nm, emission 535 nm). The results are shown in the table below.

### <1-2> Experimental results

**[Table 3]**

| Example | Capacity (%) to remove A2E-BDP at 50 µM |
|---|---|
| 1 | 20.8 |
| 2 | 28.2 |
| 3 | 0.6 |
| 4 | 24.7 |
| 5 | 26.9 |
| 6 | 24.0 |
| 7 | 16.6 |
| 8 | -15.3 |
| 9 | 26.2 |
| 10 | 7.7 |
| 11 | 65.0 |
| 12 | 21.7 |
| 13 | 31.0 |
| 14 | 35.2 |
| 15 | 48.1 |
| 16 | 49.9 |
| 17 | 61.7 |
| 18 | 65.1 |
| 19 | 37.4 |
| 20 | 17.2 |
| 21 | 64.9 |
| 22 | 47.4 |
| 23 | 45.5 |
| 24 | 38.5 |
| 25 | 45.9 |
| 26 | 66.7 |

Looking at the above Table 3, it can be confirmed that when the cells were treated with the example compound of the present invention after accumulating the A2E-fluorescent labeling substance (A2E-BDP) in ARPE-19 cells, the A2E-BDP signal by fluorescence measurement was significantly reduced. Therefore, it can be seen that the compound of the present invention removes A2E-BDP. In particular, the compounds of example 11, 17, 18, 21, and 26 showed a high removal ability of 60% or more.

The evaluation of the subsequent LC3-II production ability centered on additional compounds was performed as follows. ARPE-19 was dispensed into a 6-well plate at a concentration of 1×10⁵ cells/well and cultured for 24 hours. Thereafter, the cells were treated with the example compounds according to the present invention, listed in Table 4 below, at concentrations of 1, 5, 10, 25, and 50 µM for 12 hours. Thereafter, a cell lysis buffer containing a protease inhibitor was added to prepare a cell lysate. LC3-II levels were measured by Western immunoblotting using anti-LC3-II antibodies (Cell Signaling Technology, MA, USA). As an internal control, antibodies to β-actin (Cell Signaling Technology) were used to measure the levels by Western immunoblotting in the same manner as above. The results are shown in Table 4.

**[Table 4]**

| Example | LC3-II production capacity (fold) at 10 µM | Example | LC3-II production capacity (fold) at 10 µM |
|---|---|---|---|
| 28 | 4_{.}6 | 49 | 1.1 |
| 29 | 5.8 | 50 | >25 |
| 30 | 2.3 | 51 | >15 |
| 31 | 0.8 | 52 | >13 |
| 32 | 0.9 | 53 | 0.9 |
| 33 | 1.5 | 54 | 2.8 |
| 34 | 2.5 | 55 | 1.8 |
| 35 | 1.1 | 56 | 1.5 |
| 36 | 2.3 | 57 | 1.1 |
| 37 | 2.2 | 58 | 3.2 |
| 38 | 1.2 | 59 | 3.1 |
| 39 | 0.7 | 60 | 3.1 |
| 40 | 1.6 | 61 | 0.9 |
| 41 | 2.3 | 62 | 2.4 |
| 42 | 2.3 | 63 | 1.9 |
| 43 | 19.7 | 64 | 2.6 |
| 44 | 3.8 | 65 | 1.8 |
| 45 | 2.5 | 66 | >18 |
| 46 | 1.5 | 67 | 22.7 |
| 47 | 2.7 | 68 | 7.7 |
| 48 | >15 | 69 | 5 |

### <Experimental Example 2> Removal effect of A2E-fluorescent labeling substance by concentration

### <2-1> Experimental method

ARPE-19 cell line was dispensed into a 24-well plate at a concentration of 4×10⁴ cells/well and cultured for 24 hours. In order to induce A2E-BDP accumulation in ARPE-19 cell line, the cells were treated with A2E at a concentration of 10 µM for 24 hours. The A2E-BDP-containing ARPE-19 cells were treated with compound 17 (10, 25, 50 µM) for 24 hours. The level of A2E-BDP in each cell was measured as a fluorescence image at 510 nm using a Nikon Eclipse Ti-U fluorescence microscope (Tokyo, Japan). In addition, the change in cell number according to compound treatment was observed using a general phase contrast microscope, and the results are shown in Figure 1. Meanwhile, lutein was used as a control substance.

### <2-2> Experimental results

As shown in Figure 1, there was no significant change in the number of cells observed under a phase contrast microscope after treatment with example 17 compound of the present invention. Therefore, it was confirmed that example 17 compound had no cytotoxicity against ARPE-19 cells at the concentration used in the experiment.

Meanwhile, when A2E and A2E-BDP were accumulated in ARPE-19 cells and observed under a fluorescence microscope (Nikon NIS-Elements, AR, Japan), A2E-BDP was easily identified, and in the experimental group treated with example 17 compound of the present invention at concentrations of 10, 25, and 50 µM together with A2E, a decrease in fluorescence intensity was observed in a concentration-dependent manner. This means that the quantitative decrease in intracellular A2E-BDP occurred due to example 17 compound treatment. In the case of the lutein treatment group used as a control group, a decrease in fluorescence intensity was observed, but it was confirmed that example 17 compound of the present invention had a better A2E-BDP removal ability than lutein.

### <Experimental Example 3> Confirmation of the effect of removing A2E in human retinal pigment epithelial cells

In Table 1 and Figure 1, in order to measure the reduction of intracellular A2E by compounds, A2E-fluorescent substance (A2E-BDP) that is easy to observe fluorescence was used. In previous research reports, A2E-BDP is known to have the same intracellular dynamics and characteristics as A2E, but since A2E is a substance that actually exists in a living body, the quantitative change in intracellular A2E was directly measured using the HPLC method as follows.

### <3-1> Experimental method

ARPE-19 cell lines were dispensed into 6-well plates at a concentration of 4×10³ cells/well and cultured for 24 hours. ARPE-19 cell lines were treated with A2E at a concentration of 50 µM for 48 hours to accumulate A2E intracellularly. To evaluate the A2E removal ability, ARPE-19 cells containing A2E were treated with compound 17 of the present invention (10, 25, 50 µM) for 24 hours. Thereafter, ARPE-19 cells were washed with phosphate-buffered saline (PBS), and cell lysis buffer containing a protease inhibitor was added to prepare a cell lysate. Chloroform and methanol (2: 1, vol/vol) were added to the sample obtained above, shaken to extract A2E, and then centrifuged at 10,000 g for 10 minutes. The organic phase was dried under vacuum and redissolved in 100 mL of acetonitrile. LC-MS (Shimadzu LC-MS 2010EV) analysis was performed using a C18 column (2 '50 mm) with acetonitrile and 0.1% formic acid (gradient, 0-100%, 0-10 min; flow rate 1.0 ml/min; monitored at 503 nm). All operations during the extraction of A2E present in the cytoplasm of ARPE-19 cells were performed under weak red light to prevent the destruction of A2E. The experimental results are shown in Figure 2.

### <3-2> Experimental results

Looking at Figure 2, it is confirmed that the amount of accumulated A2E is significantly reduced in a concentration-dependent manner when ARPE-19 cells with accumulated A2E are treated with example 17 compound of the present invention. Therefore, it can be seen that the compound of the present invention directly removes A2E.

### <Experimental Example 4> Effect of inhibiting death of retinal pigment epithelial cell induced by blue light

### <4-1> Experimental method

A2E accumulated in human retinal pigment epithelial cells is known to induce cytotoxicity by blue light irradiation. Using a human retinal pigment epithelial cell line, the effect of the compound of the present invention on inhibiting cell death by blue light irradiation was measured.

The ARPE-19 cell line was dispensed into a 6-well plate at a concentration of 2×10⁴ cells/well and cultured for 24 hours. In order to induce A2E accumulation in the ARPE-19 cell line, cells were treated with A2E three times at a concentration of 25 µM at 48-hour intervals. Twenty-four hours after the last A2E treatment, cells were treated with example 17 compound of the present invention twice at 24-hour intervals at concentrations of 10, 25, and 50 µM, respectively. After an additional 24 hours of culture, blue light (BL, 430 nm, 7500 lux) was irradiated for 30 minutes and cultured for 24 hours. Cell death was observed under a microscope, and the number of living cells was measured using Image J (National Institutes of Health, USA) to calculate the quantitative cell viability. The results are shown in Figure 3.

### <4-2> Experimental results

Looking at the results of the upper photo of Figure 3, it was confirmed that no change in cell viability was observed when A2E accumulation alone (A2E group), and when A2E and blue light irradiation were performed together (AB group), ARPE-19 cell death was induced. In the experimental group treated with example 17 compound of the present invention in the cell death model where A2E and blue light irradiation were performed together, it was confirmed that cell death was significantly inhibited.

Looking at the experimental results of the lower part of Figure 3, it was confirmed that the cell death caused by A2E and blue light irradiation was statistically significantly inhibited by the treatment of example 17 compound of the present invention in the cell viability quantified using Image J.

### <Experimental Example 5> Inhibitory Effect of Blue Light-Induced NF-κB Activation in Retinal Pigment Epithelial Cells

### <5-1> Experimental method

When cells are stimulated by external pathogenic molecules (PAMPs), NF-κB dimers (p50-p65 dimers) are separated from IκB and activated. The separated NF-κB dimers pass through the nuclear membrane and enter the nucleus, where they bind to DNA and activate gene transcription, indicating the activation of NF-κB. Therefore, the inhibitory effect of the compound of the present invention on NF-κB activation can be evaluated by measuring changes in the cytoplasmic or nuclear levels of p65. The experiment was conducted as follows.

ARPE-19 cells were seeded in 6-well plates at a concentration of 2×10⁴ cells/well and cultured for 24 hours. In order to induce A2E accumulation in the ARPE-19 cell line, cells were treated with A2E three times at a concentration of 25 µM at 48-hour intervals. 24 hours after the final A2E treatment, cells were treated with compound 17 of the present invention twice at concentrations of 10 and 25 µM at 24-hour intervals, respectively. After an additional 24-hour incubation, blue light (BL, 430 nm, 7500 lux) was irradiated for 30 minutes, and cells were cultured for 24 hours. Cytoplasmic proteins were extracted using a cytoplasmic extraction buffer (10 mM HEPES, 60 mM KCl, 1 mM EDTA, 0.03%, 1 mM DTT, pH 7.6) containing a protease inhibitor. After collecting nuclei by centrifugation, nuclear proteins were extracted using Nuclear Extract Buffer (20 mM Tris.HCl, 420 mM NaCl, 1.5 mM MgCl₂, 0.2 mM EDTA and 25 % glycerol, pH 8.0) containing protease inhibitors. The level of p65 was measured by Western immunoblotting using anti-p65 antibody (Cell Signaling Technology, MA, USA). The cytoplasmic and nuclear fractions were measured by Western immunoblotting in the same manner as above using antibodies against α-tubulin (Cell Signaling Technology) and histone H3 (Abcam, Cambridge, UK) as internal controls, respectively. The results are shown in Figure 4.

### <5-2> Experimental results

As shown in Figure 4, when ARPE-19 cells were irradiated with A2E and blue light, the movement of p65 into the nucleus was observed, indicating the activation of NF-κB. Meanwhile, it can be seen that the activation of NF-κB was inhibited by the inhibition of the movement of p65 into the nucleus by the treatment with example 17 compound of the present invention.

### <Experimental Example 6> Inhibition of blue light-induced inflammatory gene expression in retinal pigment epithelial cells

### <6-1> Experimental method

ARPE-19 cell lines were seeded in 6-well plates at a concentration of 2×10⁴ cells/well and cultured for 24 hours. The process of accumulating A2E in ARPE-19 cell lines, irradiating with blue light, and treating with example 17 compound of the present invention was performed in the same manner as in Experimental Example 5. After 24 hours of irradiating with blue light, total RNA was isolated from ARPE-19 cells using Trizol reagent (Invitrogen), and total RNA was quantified using Nanodrop (Nanodrop, Thermo Scientific). Thereafter, cDNA was synthesized using 0.5 µg of total RNA, and the cDNA was amplified using the primers described in Table 5 below as a template and a real-time genetic cycler LC480 (Roche) (PCR conditions: denaturation -95 °C, 10 seconds; primer binding -60 °C, 10 seconds; and extension -72 °C, 10 seconds).

**[Table 5]**

| Gene | Primer (forward) | Primer (reverse) |
|---|---|---|
| 18S | GAGGATGAGGTGGAACGTGT | TCTTCAGTCGCTCCAGGTCT |
| IL1β | CCACCTCCAGGGACAGGATA | AACACGCAGGACAGGTACAG |
| IL6 | CCCCAGGAGAAGATTCCAAA | TTGTTTTCTGCCAGTGCCTC |
| CXCL8 | TGAATTACGGAATAATGAGTTAGA AC | TCAACCAGCAAGAAATTACTAAT |

At this time, the PCR results for 18S were used as a control to correct the relative amount of genes measured in the ARPE-19 cell line, and the experimental results are shown in Figure 5.

### <6-2> Experimental results

Looking at Figure 5, it can be seen that the expression of mRNA of inflammationrelated genes (IL-1β, IL-6, and CXCL8) in the ARPE-19 cell line significantly increased due to A2E accumulation and blue light irradiation. However, treatment with example 17 compound of the present invention led to a statistically significant reduction in the expression of these genes (P<0.05).

### <Experimental Example 7> Effect of activating autophagy in retinal pigment epithelial cells

### <7-1> Experimental method

ARPE-19 was dispensed into a 6-well plate at a concentration of 1×10⁵ cells/well and cultured for 24 hours. Then, cells were treated with example 17 compound of the present invention at concentrations of 10, 25, and 50 µM for 12 hours. Then, a cell lysis buffer containing a protease inhibitor was added to prepare a cell lysate. The LC3-II level was measured by Western immunoblotting using an anti-LC3-II antibody (Cell Signaling Technology, MA, USA). As an internal control, an antibody against β-actin (Cell Signaling Technology) was used and measured by Western immunoblotting in the same manner as above, and is shown in Figure 6 (upper graph).

Meanwhile, intracellular autophagosome formation was observed as follows. The ARPE-19 cells were transfected with a plasmid expressing GFP-LC3 (Addgene Inc., MA, USA) using Lipofectamine^{™} 2000 reagent (Invitrogen, Carlsbad, CA, USA). After 48 hours of culture, the cells were treated with example 17 compound of the present invention at a concentration of 25 µM. After additional 12 hours of culture, the cells were fixed using 4% paraformaldehyde for 10 minutes. The cells were then washed three times with PBS every 5 minutes. Finally, the puncta formation of GFP-LC3 was observed using a confocal laser scanning microscope (Nikon, Tokyo, Japan). The nuclei were stained with 1 µg/mL of Hoechst 33342 (Thermo Scientific, Rockford, IL, USA), and the observed images are shown in Figure 6 (bottom photograph results).

### <7-2> Experimental results

Looking at the graph results of Figure 6, an increase in LC3-II, an autophagy marker, was observed by treatment with example 17 compound of the present invention in the ARPE-19 cell line. LC3-II is one of the major indicator proteins of autophagy activation. When autophagy is activated, LC3-II participates in the formation of autophagosomes and forms puncta in the cytoplasm.

Looking at the photograph results of Figure 6, intracellular LC3-II puncta were observed by treatment with example 17 compound. Therefore, it is confirmed that the compound of the present invention activates the autophagy process.

### <Experimental Example 8> Effect of improving autophagy flux in retinal pigment epithelial cells

### <8-1> Experimental method

Autophagy flux was measured by comparing the degree of change in LC3-II protein formation by treatment with example 17 compound of the present invention in the presence and absence of bafilomycin A1 (Abcam, Cambridge, United Kingdom) by Western blotting (Seranova E. et al. Autophagy: Methods and Protocols, Methods in Molecular Biology, vol. 1880, pp 389-428, 2019, https://doi.org/10.1007/978-1-4939-8873-0_26).

The autophagy flux measurement in this experiment used saturating concentrations of bafilomycin A1, known as an autophagy inhibitor. Bafilomycin A1 blocks autophagy flux by inhibiting lysosomal V-ATPase, thereby preventing its acidification, and by inhibiting the Ca2+ pump SERCA, thereby preventing autophagosome-lysosome fusion. During autophagy, LC3 is converted from LC3-I to LC3-II in the autophagosome membrane, and subsequently LC3-II is degraded. Exposure to inhibitors such as bafilomycin A1 inhibits autophagosome-lysosome fusion, resulting in LC3-II accumulation. Therefore, the effect of the example compound of the present invention on autophagy flux can be determined by examining the level of LC3-II following treatment with the example compound of the present invention in the presence or absence of saturating concentrations of bafilomycin A1.

Specifically, in this experiment, ARPE-19 cell lines were seeded in 6-well plates at a concentration of 1×10⁵ cells/well and cultured for 24 hours. The ARPE-19 cell lines were treated with example 17 compound of the present invention at concentrations of 10, 25, and 50 µM for 12 hours. A cell lysis buffer containing a protease inhibitor was added hereto to prepare a cell lysate. The LC3-II level was measured by the Western immunoblotting method using a specific anti-LC3-II antibody (Cell Signaling Technology, MA, USA). Meanwhile, after treating with example 17 compound in the same manner as above under the condition of treating with bafilomycin A1 at a concentration of 2 nM for 4 hours, the amount of LC3-II was measured by the Western immunoblotting method. The amount of β-actin (Cell Signaling Technology) was measured as an internal control. The density of the bands obtained above was quantified using the Image J program, and the autophagy flux was measured by calculating the difference in the amount of LC3-II protein in the presence and absence of bafilomycin A1, and the results are shown in Figure 7.

### <8-2> Experimental results

As shown in Figure 7, example 17 compound of the present invention showed a more significant increase in LC3-II in the presence of bafilomycin compared to when bafilomycin was not present. This confirmed that example 17 compound of the present invention is a compound that significantly increases autophagy flux in ARPE-19 cell lines.

### <Experimental Example 9> Effect of activating autophagy in A2E-accumulated retinal pigment epithelial cells

### <9-1> Experimental method

A2E is known to inhibit intracellular autophagy. Therefore, it was measured whether example 17 compound of the present invention also increases autophagy in ARPE-19 cell lines accumulating A2E.

Specifically, ARPE-19 cell lines were dispensed into 6-well plates at a concentration of 1×10⁵ cells/well and cultured for 24 hours. The cells were treated with A2E at a concentration of 25 µM for 24 hours. Thereafter, the cells were treated with example 17 compound of the present invention at concentrations of 25, 50, and 100 µM for 12 hours each. Thereafter, cell lysate preparation and measurement of LC3-II by Western immunoblotting were performed in the same manner as in Experimental Example 7, and the results are shown in Figure 8.

### <9-2> Experimental results

As shown in Figure 8, an increase in LC3-II, a marker of autophagy, was observed by treatment with example 17 compound of the present invention in the ARPE-19 cell line in which A2E was accumulated. This confirms that the compound of the present invention activates the autophagy process even in the ARPE-19 cell line in which A2E was accumulated.

### <Experimental Example 10> Effect of improving autophagy flux in A2E-accumulated retinal pigment epithelial cells

### <10-1> Experimental method

It was measured whether example 17 compound of the present invention increases autophagy flux in the ARPE-19 cell line in which A2E is accumulated. That is, the ARPE-19 cell line was dispensed into a 6-well plate at a concentration of 1×10⁵ cells/well and cultured for 24 hours. The cells were treated with A2E at a concentration of 25 µM for 24 hours. The cells were treated with example 17 compound of the present invention at concentrations of 10, 25, and 50 µM for 12 hours each. The method of treating with bafilomycin (2 nM for 4 hours), preparing cell lysate, and measuring LC3-II by Western method was the same as in Experimental Example 8.

### <10-2> Experimental results

The experimental results are shown in Figure 9. As shown in Figure 9, example 17 compound of the present invention showed a more significant increase in LC3-II in the presence of bafilomycin in the ARPE-19 cell line in which A2E was accumulated. As a result, it was found that the example 17 compound significantly increased the autophagy flux in the ARPE-19 cell line in which A2E was accumulated, and from this, it was found that example 17 compound improved the autophagy flux and thereby removed the A2E accumulated in the cell.

### <Experimental Example 11> Improvement effect of compound on autophagy inhibited by A2E and blue light

### <11-1> Experimental method

A2E is known to inhibit the autophagy process more significantly after being activated by blue light. Therefore, it was confirmed whether the compound of the present invention can restore autophagy inhibited by A2E and blue light.

During the autophagosome formation process, p62 specifically binds to a substance to be degraded and simultaneously binds to LC3 protein to form an autophagosome. When autophagy is inhibited, aggregates composed of p62 and ubiquitinated proteins are formed and p62 accumulates in the cell. Therefore, by measuring the change in the level of intracellular p62, the improvement effect of the compound of the present invention on autophagy inhibited by A2E and blue light can be evaluated, and the experiment was conducted as follows.

Specifically, ARPE-19 cells were seeded into 6-well plates at a concentration of 2×10⁴ cells/well and cultured for 24 hours. To induce A2E accumulation in the ARPE-19 cell line, the cells were treated with A2E three times at a concentration of 25 µM at 48-hour intervals. 24 hours after the final A2E treatment, the cells were treated with example 17 compound twice at 24-hour intervals each. After an additional 24-hour incubation, blue light (BL, 430 nm, 7500 lux) was irradiated for 30 minutes and cells were cultured for 24 hours. Cell lysis buffer containing a protease inhibitor was added to each cell to prepare a cell lysate. The amount of intracellular p62 was measured by Western immunoblotting using an anti-p62 antibody (Santa Cruz, TX, USA). The amount of intracellular β-actin was measured in the same manner using anti-β-actin antibody (Santa Cruz) and used as a control. The results are shown in Figure 10.

### <11-2> Experimental results

The experimental results are shown in Figure 10. As shown in Figure 10, a significant increase in p62 protein was observed in the ARPE-19 cell line due to the accumulation of A2E and blue light irradiation. When the p62 protein increased by the accumulation of A2E and blue light irradiation was treated with example 17 compound of the present invention, it was confirmed that the p62 protein concentration was decreased in a concentration-dependent manner. Therefore, it can be seen that the compound of the present invention has the effect of restoring the autophagy activity in human retinal pigment epithelial cells reduced by A2E and blue light, and promotes the removal of A2E accumulated in the cells by restoring the autophagy activity.

### <Experimental Example 12> Improvement effect of compound on blue light-induced retinal damage animal model

The improvement effect of the compound of the present invention on blue light-induced retinal damage in experimental animals (Balb/c mice) was confirmed. The following experiment was conducted by repeatedly administering the example compound orally for 4 weeks. All tests were conducted at the Non-Clinical Center of the Korea Conformity Laboratories (KCL) Bio Headquarters (Songdo, Incheon) and were performed after obtaining approval from the Animal Experiment Ethics Committee (Approval No.: IA20-04287).

### <12-1> Experimental method

Specific pathogen-free (SPF) male BALB/c mice (Orient Bio, Korea) were acclimatized for 1 week. The body weight was measured the day before administration, and the groups were separated by the weight ranked using an excel program. Only animals within ±20% of the average body weight were used in the test. After separation of the group, dark room conditions were maintained for 24 hours, and dark room conditions were maintained throughout the blue light exposure period.

After anesthetizing the experimental animals with isoflurane, blue light of 10,000 lux was irradiated for a total of 14 times (1 hr/day) for 2 weeks using a blue light irradiation device. The test substance was prepared by dissolving example 17 compound of the present invention in a solution made by dissolving a HP-beta-cyclodextrin in a 5% CMC Na solution to make a 10% concentration. 10 ml/kg/day was administered as the administration amount, and 25 mg/kg/day was administered to each experimental animal. The test substance was administered by forceful oral administration using an oral administration sonde after grasping and correcting the skin of the dorsum of the test animal. It was administered once/day, for 7 days/week, for 4 weeks, in the morning on the day of administration. The initial 2 weeks overlapped with the blue light irradiation period, and administration was performed for a total of 4 weeks by adding 2 weeks after the end of blue light irradiation. On the day of administration, the test substance was administered before blue light irradiation. All animals were examined in both eyes before the start of administration and once a week. After dilating the pupils by instilling mydriatic agents, the fundus of the eyes was observed using a fundus camera (Genesis-D, Kowa Co. Ltd., Japan), and photographs were taken of each animal the week after the end of test substance administration.

After the end of the experiment, both eyes of all animals were enucleated and fixed in Davidson's solution. Hematoxylin and eosin staining (H&E) was performed, and the average values of the ONL (outer nuclear layer) and IS/OS (inner segments/outer segments layer) thickness were measured at a location 140 µm (left/right) from the retinal nerve center using the Axio Vision SE644 (ZEISS) program. The ONL and IS/OS thickness of the contralateral eye were measured in a similar manner, and the average values of both eyes were used as the final thickness. One-way ANOVA test and Student's t-test were used between each group, and it was determined that the result was statistically significant when p<0.05 or less. The SPSS 12.0 K program (SPSS, Chicago, IL, U.S.A.), a commercially widely used statistical package, was used for statistical analysis. Animals for which the test model was not formed were excluded from the statistics.

### <12-2> Experimental results

The experimental results are shown in Figures 11 and 12. As a result of observation using a fundus camera, retinal damage was confirmed in the blue light irradiated control group compared to the normal control group. The histomorphological analysis results of ocular tissue also confirmed that ONL and IS/OS thickness were reduced in the blue light irradiated control group compared to the normal control group. On the other hand, in the group administered 25 mg/kg/day of the example compound of the present invention, retinal damage was improved as shown in the fundus examination (Figure 11). In addition, the ONL and IS/OS thickness, which were significantly reduced by blue light irradiation, were statistically significantly (p<0.01) recovered by administration of the example compound of the present invention (Figure 12).

### <Experimental Example 13> Evaluation of A2E-BDP removal ability by concentration of other example compounds

In the experimental results of Table 3 above, the A2E-BDP removal ability by concentration was re-evaluated for the compounds of examples 11, 17, 18, 21, and 26, which showed high removal ability of 60% or more. The A2E-fluorescent labeling substance (A2E-BDP) removal ability was evaluated according to Experimental Example 1 (except that the test compound was treated at the indicated concentration). The results are shown in Figure 13.

As shown in Figure 13, it was confirmed that not only example 17 compound according to the present invention, but also example 11, 18, 21, and 26 compounds showed a concentration-dependent decrease in the amount of A2E. Therefore, it can be seen that the compounds of the present invention directly remove A2E.

### <Experimental Example 14> Evaluation of autophagy activation of other example compounds

The autophagy activation effect of example 11, 17, 18, 21, and 26 compounds, which showed high removal ability of 60% or more in the experimental results of Table 3 above, in human retinal pigment epithelial cells was evaluated. The cells were treated with the test compounds at the indicated concentrations, and the autophagy activation effect through the increase of p62 protein (p-p62 (S349)) phosphorylated at serine 349 residue was measured by Western immunoblotting using an anti-p-p62 antibody, and the results are shown in Figure 14. The increase in the intracellular P-p62 (S439) level is one of the major indicators of autophagy activation.

As shown in Figure 14, it was found that not only example 17 compound according to the present invention, but also example 11, 18, 21, and 26 compounds activated autophagy in a concentration-dependent manner.

### <Experimental Example 15> Evaluation of autophagy activation of other example compounds

The autophagy activation effect of example compounds expected to have autophagy activation ability and low cytotoxicity among the example compounds in Table 2 above in human retinal pigment epithelial cells was evaluated according to Experimental Example 7. That is, the autophagy activation effect through the increase of LC3-II was evaluated in a concentration-dependent manner, and the results are shown in Figures 15 and 16.

As shown in Figures 15 and 16, example 28, 30, 34, 41, 42, 47, 58, 59, and 60 compounds according to the present invention showed excellent autophagy activation effects dependent on the concentration of the compound.

## Claims

1. A compound represented by the following chemical formula 1 or a pharmaceutically acceptable salt thereof. 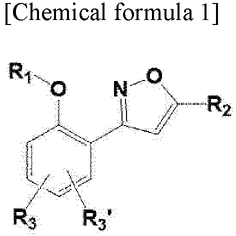 In chemical formula 1,
R₁ is hydrogen, C₁₋₆alkyl, or C₃₋₆cycloalkyl, wherein at least one hydrogen of the C₁₋₆alkyl or C₃₋₆cycloalkyl is optionally substituted with at least one substituent independently selected from the group consisting of hydroxy, C₁₋₃alkoxy and halogen,
R₂ is hydrogen, -C₁₋₆alkyl, C₃₋₆cycloalkyl, -C₁₋₆alkoxy, -C₁₋₃alkylene-ORa, -C₁₋₃alkylene-NRaRb, -C₁₋₃alkylene-heterocycloalkyl, or -C₁₋₃alkylene-heteroaryl, wherein Ra and Rb are each independently hydrogen, C₁₋₆alkyl, C₃₋₆cycloalkyl, allyl, benzyl, -C₁₋₃alkylene-NH-C₁₋₃alkyl, -C₁₋₃alkylene-OH, -C₁₋₃alkylene-COOH, -C₁₋₃alkylene-COO-C₁₋₃alkyl, pyrrolidine, -C₁₋₃alkylene-pyrrolidine, aryl, or heteroaryl, wherein at least one hydrogen of the -C₁₋₆alkyl, C₃₋₆cycloalkyl, C₁₋₆alkoxy, C₁₋₃alkylene or heterocycloalkyl is optionally substituted with at least one substituent independently selected from the group consisting of =O, -C₁₋₃alkyl, -OH, -COOH, -CO-C₁₋₃alkyl, -CONH₂, -SO₂-C₁₋₃alkyl, -NH₂, -NHCH₃, - N(CH₃)₂, -NHCOCH₃, halogen, allyl, phenyl, benzyl, -C₁₋₃alkylene-OH, -C₁₋₃alkylene-COOH, -C₁₋₃alkylene-COOC₁₋₃alkyl, methoxycarbonyl, ethoxycarbonyl, or tert-butoxycarbonyl,
R₃ and R₃' are each independently hydrogen, halogen, C₁₋₆alkyl, C₁₋₆alkoxy, C₃₋₆cycloalkyl, heterocycloalkyl, -CH₂-CR₄ (=CR₅R₆), -CN, haloC₁₋₃alkyl, -C₁₋₃alkylene-OR₄, - C(O)R₄, -C₁₋₃alkylene-C(O)R₄, -C₁₋₃alkylene-COOH, or -COOH, wherein R₄, R₅, and R₆ are each independently hydrogen, methyl, or -NH₂.

2. The compound of Claim 1 or a pharmaceutically acceptable salt thereof, wherein the compound has the structure of the following chemical formula 1a: 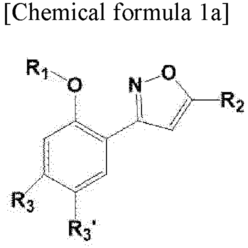 In the chemical formula 1a,
R₁ is hydrogen, C₁₋₆alkyl, or C₃₋₆cycloalkyl, wherein at least one hydrogen of the C₁₋₆alkyl or C₃₋₆cycloalkyl is optionally substituted with at least one substituent independently selected from the group consisting of hydroxy, C₁₋₃alkoxy and halogen,
R₂ is hydrogen, -C₁₋₆alkyl, C₃₋₆cycloalkyl, -C₁₋₆alkoxy, -C₁₋₃alkylene-ORa, -C₁₋₃alkylene-NRaRb, -C₁₋₃alkylene-heterocycloalkyl, or -C₁₋₃alkylene-heteroaryl, wherein Ra and Rb are each independently hydrogen, C₁₋₆alkyl, C₃₋₆cycloalkyl, allyl, benzyl, -C₁₋₃alkylene-NH-C₁₋₃alkyl, -C₁₋₃alkylene-OH, -C₁₋₃alkylene-COOH, -C₁₋₃alkylene-COO-C₁₋₃alkyl, pyrrolidine, -C₁₋₃alkylene-pyrrolidine, aryl, or heteroaryl, wherein at least one hydrogen of the -C₁₋₆alkyl, C₃₋₆cycloalkyl, C₁₋₆alkoxy, C₁₋₃alkylene or heterocycloalkyl is optionally substituted with at least one substituent independently selected from the group consisting of =O, -C₁₋₃alkyl, -OH, -COOH, -CO-C₁₋₃alkyl, -CONH₂, -SO₂-C₁₋₃alkyl, -NH₂, -NHCH₃, - N(CH₃)₂, -NHCOCH₃, halogen, allyl, phenyl, benzyl, -C₁₋₃alkylene-OH, -C₁₋₃alkylene-COOH, -C₁₋₃alkylene-COOC₁₋₃alkyl, methoxycarbonyl, ethoxycarbonyl, or tert-butoxycarbonyl,
R₃ and R₃' are each independently hydrogen, halogen, C₁₋₆alkyl, C₁₋₆alkoxy, C₃₋₆cycloalkyl, heterocycloalkyl, -CH₂-CR₄ (=CR₅R₆), -CN, haloC₁₋₃alkyl, -C₁₋₃alkylene-OR₄, - C(O)R₄, -C₁₋₃alkylene-C(O)R₄, -C₁₋₃alkylene-COOH, or -COOH, wherein R₄, R₅, and R₆ are each independently hydrogen, methyl, or -NH₂.

3. The compound of Claim 2 or a pharmaceutically acceptable salt thereof, in the Chemical Formula 1a,
R₁ is hydrogen or C₁₋₆alkyl, wherein at least one hydrogen of the C₁₋₆alkyl is optionally substituted with at least one substituent independently selected from halogen,
R₂ is -C₁₋₆alkyl, -C₁₋₃alkylene-ORa, -C₁₋₃alkylene-NRaRb, -C₁₋₃alkylene-piperazine, - C₁₋₃alkylene-piperidine, -C₁₋₃alkylene-morpholine, -C₁₋₃alkylene-4,5,6,7-tetrahydrothieno[3,2-c]pyridine, -C₁₋₃alkylene-5,6,7,8-tetrahydroimidazo[1,2-a]pyrazine, -C₁₋₃alkylene-pyrrolidine, -C₁₋₃alkylene-3,8-diazabicyclo[3.2.1]octane, or -C₁₋₃alkylene-2,5-diazabicyclo[2.2.2]octane, wherein Ra and Rb are each independently hydrogen, C₁₋₆alkyl, C₃₋₆cycloalkyl, allyl, benzyl, C₁₋₃alkylene-OH, -C₁₋₃alkylene-COOH, -C₁₋₃alkylene-COO-C₁₋₃alkyl, aryl, or heteroaryl, wherein at least one hydrogen of the -C₁₋₆alkyl, C₃₋₆cycloalkyl, C₁₋₆alkoxy, C₁₋₃alkylene or heterocycloalkyl is optionally substituted with at least one substituent independently selected from the group consisting of =O, -C₁₋₃alkyl, -OH, -COOH, -CO-C₁₋₃alkyl, -CONH₂, -SO₂-C₁₋₃alkyl, -NH₂, -NHCH₃, -N(CH₃)₂, -NHCOCH₃, halogen, allyl, phenyl, benzyl, -C₁₋₃alkylene-OH, -C₁₋₃alkylene-COOH, -C₁₋₃alkylene-COOC₁₋₃alkyl, methoxycarbonyl, ethoxycarbonyl, and tert-butoxycarbonyl,
R₃ and R₃' are each independently hydrogen, halogen, C₁₋₆alkyl, C₁₋₆alkoxy, heterocycloalkyl, -CH₂-CR₄(=CR₅R₆), -CN, haloC₁₋₃alkyl, -C₁₋₃alkylene-OR₄, -C(O)R₄, -C₁₋₃alkylene-C(O)R₄, -C₁₋₃alkylene-COOH, or -COOH, wherein R₄, R₅, and R₆ are each independently hydrogen, methyl, or -NH₂.

4. The compound of Claim 3 or a pharmaceutically acceptable salt thereof, in the Chemical Formula 1a,
R₁ is hydrogen, methyl, or -CF₃,
R₂ is -C₁₋₆alkyl, -C₁₋₃alkylene-ORa, -C₁₋₃alkylene-piperazine, -C₁₋₃alkylene-piperidine, -C₁₋₃alkylene-morpholine, -C₁₋₃alkylene-4,5,6,7-tetrahydrothieno[3,2-c]pyridine, or -C₁₋₃alkylene-5,6,7,8-tetrahydroimidazo[1,2-a]pyrazine, wherein Ra and Rb are each independently hydrogen, C₁₋₆alkyl, allyl, benzyl, -C₁₋₃alkylene-COO-C₁₋₃alkyl, phenyl, quinazoline, or pyridyl, wherein at least one hydrogen of the -C₁₋₆alkyl, C₁₋₃alkylene or heterocycloalkyl is optionally substituted with at least one substituent independently selected from the group consisting of =O, -C₁₋₃alkyl, -OH, -COOH, -CO-C₁₋₃alkyl, -CONH₂, -SO₂-C₁₋₃alkyl, halogen, allyl, phenyl, benzyl, -C₁₋₃alkylene-OH, -C₁₋₃alkylene-COOH, -C₁₋₃alkylene-COOC₁₋₃alkyl, methoxycarbonyl, ethoxycarbonyl, and tert-butoxycarbonyl,
R₃ and R₃' are each independently hydrogen, halogen, C₁₋₆alkyl, C₁₋₆alkoxy, heterocycloalkyl, -CH₂-CR₄(=CR₅R₆), -CN, -haloC₁₋₃alkyl, -C₁₋₃alkylene-OR₄, -C(O)R₄, -C₁₋₃alkylene-C(O)R₄, -C₁₋₃alkylene-COOH, or -COOH, wherein R₄, R₅, and R₆ are each independently hydrogen or methyl.

5. The compound of Claim 1 or a pharmaceutically acceptable salt thereof, wherein the compound is
(1) 3-(5-bromo-2-methoxyphenyl)-5-propylisoxazole;
(2) 4-bromo-2-(5-propylisoxazole-3-yl)phenol;
(3) 3-(2-methoxy-5-propylphenyl)-5-propylisoxazole;
(4) 3-(5-allyl-2-methoxyphenyl)-5-propylisoxazole;
(5) 4-allyl-2-(5-propylisoxazole-3-yl)phenol;
(6) 4-propyl-2-(5-propylisoxazole-3-yl)phenol;
(7) (3-(5-bromo-2-methoxyphenyl)isoxazole-5-yl)methanol;
(8) 4-bromo-2-(5-(hydroxymethyl)isoxazole-3-yl)phenol;
(9) 5-((allyloxy)methyl)-3-(5-bromo-2-methoxyphenyl)isoxazole;
(10) (3-(2-methoxy-5-propylphenyl)isoxazole-5-yl)methanol;
(11) 5-((benzyloxy)methyl)-3-(5-bromo-2-methoxyphenyl)isoxazole;
(12) ethyl 2-((3-(5-bromo-2-methoxyphenyl)isoxazole-5-yl)methoxy)acetate;
(13) 3-(5-bromo-2-methoxyphenyl)-5-(methoxymethyl)isoxazole;
(14) 2-(5-(hydroxymethyl)isoxazole-3-yl)-4-propylphenol;
(15) (3-(5-allyl-2-methoxyphenyl)isoxazole-5-yl)methanol;
(16) 4-allyl-2-(5-(hydroxymethyl)isoxazole-3-yl)phenol;
(17) 3-(5-allyl-2-methoxyphenyl)-5-((allyloxy)methyl)isoxazole;
(18) 3-(5-allyl-2-methoxyphenyl)-5-((benzyloxy)methyl)isoxazole;
(19) ethyl 2-((3-(5-allyl-2-methoxyphenyl)isoxazole-5-yl)methoxy)acetate;
(20) 3-(2-methoxy-5-propylphenyl)-5-(methoxymethyl)isoxazole;
(21) tert-butyl 4-((3-(5-bromo-2-methoxyphenyl)isoxazole-5-yl)methyl)piperazine-1-carboxylate;
(22) 3-(5-allyl-2-methoxyphenyl)-5-((4-allylpiperazine-1-yl)methyl)isoxazole;
(23) 3-(5-allyl-2-methoxyphenyl)-5-((4-benzylpiperazine-1-yl)methyl)isoxazole;
(24) ethyl 2-(4-((3-(5-allyl-2-methoxyphenyl)isoxazole-5-yl)methyl)piperazine-1-yl)acetate;
(25) tert-butyl 4-((3-(5-allyl-2-methoxyphenyl)isoxazole-5-yl)methyl)piperazine-1-carboxylate;
(26) tert-butyl 4-((3-(2-methoxy-5-propylphenyl)isoxazole-5-yl)methyl)piperazine-1-carboxylate;
(27) 4-allyl-2-(5-((allyloxy)methyl)isoxazole-3-yl)phenol;
(28) 3-(5-bromo-2-methoxyphenyl)-5-(piperazine-1-ylmethyl)isoxazole;
(29) 3-(5-allyl-methoxyphenyl)-5-(piperazine-1-ylmethyl)isoxazole;
(30) 3-(2-methoxy-5-propylphenyl)-5-(piperazine-1-ylmethyl)isoxazole;
(31) (3-(2-methoxy-5-(trifluoromethyl)phenyl)isoxazole-5-yl)methanol;
(32) 2-(3-(2-methoxy-5-(trifluoromethyl)phenyl)isoxazole-5-yl)propan-2-ol;
(33) 2-(3-(2-methoxy-5-(trifluoromethyl)phenyl)isoxazole-5-yl)ethan-1-ol;
(34) 3-(2-methoxy-5-(trifluoromethyl)phenyl)-5-(piperazine-1-ylmethyl)isoxazole;
(35) (3-(2-methoxy-4-(trifluoromethyl)phenyl)isoxazole-5-yl)methanol;
(36) 2-((3-(2-methoxy-5-(trifluoromethyl)phenyl)isoxazole-5-yl)methoxy)acetic acid;
(37) 4-((3-(2-methoxy-5-(trifluoromethyl)phenyl)isoxazole-5-yl)methyl)-1-methylpiperazine-2-on;
(38) (3-(2-methoxy-5-morpholinophenyl)isoxazole-5-yl)methanol;
(39) 2-(3-(5-allyl-2-methoxyphenyl)isoxazole-5-yl)propan-2-ol;
(40) 2-(3-(2-methoxy-5-propylphenyl)isoxazole-5-yl)propan-2-ol;
(41) 3-(5-allyl-2-methoxyphenyl)-5-((4-methylpiperazine-1-yl)methyl)isoxazole;
(42) 3-(2-methoxy-5-propylphenyl)-5-((4-methylpiperazine-1-yl)methyl)isoxazole;
(43) (3-(5-allyl-2-(trifluoromethoxy)phenyl)isooxazole-5-yl)methanol;
(44) (3-(4-allyl-2-methoxyphenyl)isoxazole-5-yl)methanol;
(45) (3-(5-isopropyl-2-methoxyphenyl)isoxazole-5-yl)methanol;
(46) tert-butyl 4-((3-(5-isopropyl-2-methoxyphenyl)isoxazole-5-yl)methyl)piperazine-1-carboxylate;
(47) 3-(5-isopropyl-2-methoxyphenyl)-5-(piperazine-1-ylmethyl)isoxazole;
(48) tert-butyl 4-((3-(4-allyl-2-methoxyphenyl)isoxazole-5-yl)methyl)piperazine-1-carboxylate;
(49) 3-(4-allyl-2-methoxyphenyl)-5-(piperazine-1-ylmethyl)isoxazole;
(50) tert-butyl 4-((3-(5-allyl-2-(trifluoromethoxy)phenyl)isoxazole-5-yl)methyl)piperazine-1-carboxylate;
(51) 3-(5-allyl-2-(trifluoromethoxy)phenyl)-5-(piperazine-1-ylmethyl)isoxazole;
(52) 3-(5-allyl-2-(trifluoromethoxy)phenyl)-5-((4-methylpiperazine-1-yl)methyl)isoxazole;
(53) (2S,6R)-4-((3-(2-methoxy-5-propylphenyl)isoxazole-5-yl)methyl)-2,6-dimethylmorpholine;
(54) 3-(2-methoxy-5-propylphenyl)-5-((4-methoxypiperidine-1-yl)methyl)isoxazole;
(55) 1-((3-(2-methoxy-5-propylphenyl)isoxazole-5-yl)methyl)piperidine-4-carboxylic acid; (56) 5-((6,7-dihydrothieno[3,2-c]pyridin-5(4H)-yl)methyl)-3-(2-methoxy-5-propylphenyl)isoxazole;
(57) 5-((5,6-dihydroimidazo[1,2-a]pyrazin-7(8H)-yl)methyl)-3-(2-methoxy-5-propylphenyl)isoxazole;
(58) 3-(4-fluoro-2-methoxy-5-propylphenyl)-5-(piperazine-1-ylmethyl)isoxazole;
(59) 3-(4-fluoro-2-methoxy-5-propylphenyl)-5-((4-methylpiperazine-1-yl)methyl)isoxazole;
(60) 5-((4-methylpiperazine-1-yl)methyl)-3-(5-propyl-2-(trifluoromethoxy)phenyl)isoxazole;
(61) 2-(4-methoxy-3-(5-((4-methylpiperazine-1-yl)methyl)isoxazole-3-yl)phenyl)ethan-1-ol;
(62) 3-(2-methoxy-5-(2-methoxyethyl)phenyl)-5-((4-methylpiperazine-1-yl)methyl)isoxazole;
(63) 1-(4-((3-(5-isopropyl-2-methoxyphenyl)isoxazole-5-yl)methyl)piperazine-1-yl)ethan-1-on;
(64) 3-(5-isopropyl-2-methoxyphenyl)-5-((4-(methylsulfonyl)piperazine-1-yl)methyl)isoxazole;
(65) 3-(5-allyl-2-methoxyphenyl)-5-((quinazolin-4-yloxy)methyl)isoxazole;
(66) tert-butyl 4-((3-(2-methoxy-5-(2-methylallyl)phenyl)isoxazole-5-yl)methyl)piperazine-1-carboxylate;
(67) 3-(2-methoxy-5-(2-methylallyl)phenyl)-5-(piperazine-1-ylmethyl)isoxazole;
(68) (3-(2-methoxy-5-(2-methylallyl)phenyl)isoxazole-5-yl)methanol; or
(69) 3-(2-methoxy-5-(3-methylbut-2-en-1-yl)phenyl)-5-((4-methylpiperazine-1-yl)methyl)isoxazole.

6. The compound of Claim 5 or a pharmaceutically acceptable salt thereof, wherein the compound is
(11) 5-((benzyloxy)methyl)-3-(5-bromo-2-methoxyphenyl)isoxazole;
(15) (3-(5-allyl-2-methoxyphenyl)isoxazole-5-yl)methanol;
(17) 3-(5-allyl-2-methoxyphenyl)-5-((allyloxy)methyl)isoxazole;
(18) 3-(5-allyl-2-methoxyphenyl)-5-((benzyloxy)methyl)isoxazole;
(21) tert-butyl 4-((3-(5-bromo-2-methoxyphenyl)isoxazole-5-yl)methyl)piperazine-1-carboxylate;
(25) tert-butyl 4-((3-(5-allyl-2-methoxyphenyl)isoxazole-5-yl)methyl)piperazine-1-carboxylate;
(26) tert-butyl 4-((3-(2-methoxy-5-propylphenyl)isoxazole-5-yl)methyl)piperazine-1-carboxylate;
(28) 3-(5-bromo-2-methoxyphenyl)-5-(piperazine-1-ylmethyl)isoxazole;
(29) 3-(5-allyl-methoxyphenyl)-5-(piperazine-1-ylmethyl)isoxazole;
(30) 3-(2-methoxy-5-propylphenyl)-5-(piperazine-1-ylmethyl)isoxazole;
(34) 3-(2-methoxy-5-(trifluoromethyl)phenyl)-5-(piperazine-1-ylmethyl)isoxazole;
(41) 3-(5-allyl-2-methoxyphenyl)-5-((4-methylpiperazine-1-yl)methyl)isoxazole;
(42) 3-(2-methoxy-5-propylphenyl)-5-((4-methylpiperazine-1-yl)methyl)isoxazole;
(43) (3-(5-allyl-2-(trifluoromethoxy)phenyl)isooxazole-5-yl)methanol;
(44) (3-(4-allyl-2-methoxyphenyl)isoxazole-5-yl)methanol;
(47) 3-(5-isopropyl-2-methoxyphenyl)-5-(piperazine-1-ylmethyl)isoxazole;
(48) tert-butyl 4-((3-(4-allyl-2-methoxyphenyl)isoxazole-5-yl)methyl)piperazine-1-carboxylate;
(50) tert-butyl 4-((3-(5-allyl-2-(trifluoromethoxy)phenyl)isoxazole-5-yl)methyl)piperazine-1-carboxylate;
(51) 3-(5-allyl-2-(trifluoromethoxy)phenyl)-5-(piperazine-1-ylmethyl)isoxazole;
(52) 3-(5-allyl-2-(trifluoromethoxy)phenyl)-5-((4-methylpiperazine-1-yl)methyl)isoxazole;
(58) 3-(4-fluoro-2-methoxy-5-propylphenyl)-5-(piperazine-1-ylmethyl)isoxazole;
(59) 3-(4-fluoro-2-methoxy-5-propylphenyl)-5-((4-methylpiperazine-1-yl)methyl)isoxazole;
(60) 5-((4-methylpiperazine-1-yl)methyl)-3-(5-propyl-2-(trifluoromethoxy)phenyl)isoxazole;
(66) tert-butyl 4-((3-(2-methoxy-5-(2-methylallyl)phenyl)isoxazole-5-yl)methyl)piperazine-1-carboxylate;
(67) 3-(2-methoxy-5-(2-methylallyl)phenyl)-5-(piperazine-1-ylmethyl)isoxazole; or
(68) (3-(2-methoxy-5-(2-methylallyl)phenyl)isoxazole-5-yl)methanol.

7. The compound of Claim 6 or a pharmaceutically acceptable salt thereof, wherein the compound is
(28) 3-(5-bromo-2-methoxyphenyl)-5-(piperazine-1-ylmethyl)isoxazole;
(30) 3-(2-methoxy-5-propylphenyl)-5-(piperazine-1-ylmethyl)isoxazole;
(34) 3-(2-methoxy-5-(trifluoromethyl)phenyl)-5-(piperazine-1-ylmethyl)isoxazole;
(41) 3-(5-allyl-2-methoxyphenyl)-5-((4-methylpiperazine-1-yl)methyl)isoxazole;
(42) 3-(2-methoxy-5-propylphenyl)-5-((4-methylpiperazine-1-yl)methyl)isoxazole;
(47) 3-(5-isopropyl-2-methoxyphenyl)-5-(piperazine-1-ylmethyl)isoxazole;
(58) 3-(4-fluoro-2-methoxy-5-propylphenyl)-5-(piperazine-1-ylmethyl)isoxazole;
(59) 3-(4-fluoro-2-methoxy-5-propylphenyl)-5-((4-methylpiperazine-1-yl)methyl)isoxazole; or
(60) 5-((4-methylpiperazine-1-yl)methyl)-3-(5-propyl-2-(trifluoromethoxy)phenyl)isoxazole.

8. A pharmaceutical composition for treating or preventing an eye disease, comprising as an active agent a compound of any one of claims 1 to 7 or a pharmaceutically acceptable salt thereof.

9. The pharmaceutical composition of claim 8, wherein the eye disease is an eye disease in which N-retinylidene-N-retinyl-ethanolamine (A2E) accumulates in eye cells.

10. The pharmaceutical composition of claim 8, wherein the eye disease dry macular degeneration.

11. A composition for protecting the retina from damage caused by blue light, comprising as an active agent a compound of any one of claims 1 to 7 or a pharmaceutically acceptable salt thereof.
